# EUROPEAN PATENT APPLICATION

(11) **EP 1 582 105 A1**
(43) Date of publication of application: **05.10.2005**
(21) Application number: 03780774.0
(22) Date of filing: 15.12.2003
(51) Int. Cl.: A23L 3/3562, A23L 1/03, A23L 1/09, A23K 1/16, A61K 7/00, A61K 7/075, A61K 7/08, A61K 7/16, A61K 7/50, A61K 9/06, A61K 9/08, A61K 47/26, C09G 1/18, C09K 15/06, C11B 5/00, C11D 10/06

(54) **METHOD OF INHIBITING WATER CONTENT VARIATION OF COMPOSITION AND USE THEREOF**

(30) Priority: 19.12.2002 JP 2002368153; 07.03.2003 JP 2003062117; 26.03.2003 JP 2003086567; 09.06.2003 JP 2003163732; 08.10.2003 JP 2003349976
(71) Applicant: KABUSHIKI KAISHA HAYASHIBARA SEIBUTSU KAGAKU KENKYUJO, Okayama 700-0907 (JP)
(72) Inventor: TAKEUCHI, Kanou c/o K. K. Hayashibara Shoji, Okayama-shi, Okayama 700-0907 (JP); KUBOTA,M. K.K Hayashibara Seibutsu Kagaku Kenkyujo, Okayama-shi, Okayama 700-0907 (JP); MIYAKE,T. K.K Hayashibara Seibutsu Kagaku Kenkyujo, Okayama-shi, Okayama 700-097 (JP)
(74) Representative: Daniels, Jeffrey Nicholas
(86) International application number: PCT/JP2003/016047
(87) International publication number: WO 2004/056216

(57) **Abstract**

The present invention has objects for providing a method for inhibiting the moisture variation in compositions, a composition whose moisture variation is inhibited, and an agent for inhibiting the moisture variation in compositions. The objects are solved by providing a method for inhibiting the moisture variation in compositions comprising incorporating into a composition a saccharide-derivative(s) of α,α-trehalose as an effective ingredient, a composition whose moisture variation is inhibited by incorporating the saccharide-derivative(s), a moisture variation inhibiting agent containing the saccharide-derivative(s) as an effective ingredient, and uses thereof.

## Description

### TECHNICAL FIELD

The present invention relates to a method for inhibiting the moisture variation in compositions and uses of the same, more particularly, a method for inhibiting the moisture variation in compositions by incorporating a saccharide-derivative of α,α-trehalose into the compositions, a moisture variation inhibiting agent (simply abbreviated as "MVIA" , hereinafter) for compositions, which comprises a saccharide-derivative of α,α-trehalose as an effective ingredient, and uses thereof.

### BACKGROUND ART

In general, compositions such as food products, cosmetics, pharmaceuticals, etc., have complicated components, as well as their inherent qualities, features, and functions such as characteristic physiological properties, tastes, flavors, color tints, and mouth feel and tastes. Depending on the ingredient compositions, texture structures, and environmental conditions, the above qualities, features, and functions of such compositions have been recognized to be gradually deteriorated just after their processings through circulation and storage until delivered to users. As such environmental conditions, oxygen, light, moisture, and temperature, as well as shock, vibration, compression, microorganisms, and other living bodies have been known as factors for deteriorating the compositions. In case that these physical, chemical or biological environmental conditions are not adequate for the compositions, any one of the occurrences thereof may subsequently induce other changes or various changes in parallel to proceed unfavorable phenomena of quality deterioration, and therefore the control of the above conditions has been recognized as a quite important thing for the person skilled in the art (cf. *"Yogashi-Seizo-no-Kiso-to-Jissai"* (*The Basis and practice in processing Western confectionery,* pp. 303-372, 1991, published by KORIN Publishing Co., Ltd., Tokyo, Japan).

The most influential things of physicochemical factors for deteriorating compositions are temperature change, dryness, and moisture absorption. Moisture imparts characteristic features to the shape, texture, flavor and taste of compositions: solves water-soluble material ingredients such as saccharides, acids, alkalis, and salts; forms a gel when it is absorbed in hydrophilic colloidal substances such as starches and proteins while effectively forming the desired texture and stabilizing these ingredients; and exists in a variety of forms such as a suspension form after formed with lipid into an emulsion. The water in compositions exists both in a state of free water which retains the features inherent to water in a normal aqueous solution; and in a state of bound water which, unlike water in a general liquid water, is hardly evaporated, incapable of dissolving substances, and free from being utilized by microorganisms; where the free water and the bound water in the compositions are present in a constant ratio depending on the types of the compositions and their surrounding circumstances. Also, it is known that a mere change in the moisture content of compositions may deteriorate their inherent properties, induce bacterial contamination, and affect their storage properties.

For example, high moisture content hydrophilic gel materials such as a solidifying dough for jelly and bavaroise, cream dough for butter cream and custard cream, and proceed fruit products such as purée and jam may release water, i.e., syneresis, as the lapse of time; affect the appearance; and deteriorate or tend to deteriorate the taste, flavor, color, and mouth feel (texture), and to induce bacterial contamination, even if there appears no particular change in circumstantial conditions.

The moisture content of compositions changes depending on their existing circumstances and it is predominantly controlled by atmospheric relative humidity of circumstances (simply designated as "moisture", hereinafter), where the compositions release to or absorb moisture from the outer atmosphere to show an equilibrium moisture content under a constant temperature condition. It has been known that the moisture variation in compositions may induce changes in physical and physicochemical properties, deteriorate proteins as constituents of the compositions, induce retrogradation of gelatinized starch, and proceed the oxidation and decomposition of lipids, followed by proceeding solidification, shrink, cracking, browning, dissolution, deliquescence, crystallization, and precipitation to deteriorate the texture, shape, taste, flavor, color, and mouth feel of the compositions; inactivate the effective ingredients of the compositions; diminish the nutritional ingredients of the compositions; and to deteriorate the compositions due to bacterial contamination. Thus, the inhibition of moisture variation in compositions is a quite important aspect in retaining their quality.

The quality deterioration of compositions accompanied by the moisture variation would be a problem inducible in a wide variety of fields of food products, cosmetics, medicated cosmetics, pharmaceuticals, daily goods, groceries, and chemical industries. Therefore, the inhibition of moisture variation in compositions must be a quite important aspect in retaining their quality and functions, without restricting to a specific field.

As a means for solving the above object, there has been employed a method to keep the equilibrium moisture contents of compositions to a constant level by minimizing the influence of outer phase on the compositions as low as possible by using packages with a lesser moisture permeability, incorporation of desiccants, sealed containers, humidistats, desiccators, etc., (cf. *"Yogashi-Seizo-no-Kiso-to-Jissai" (The Basis and practice in processing Western confectionery,* pp. 303-372, 1991, published by KORIN Publishing Co., Ltd., Tokyo, Japan). These methods, however, have the demerits of being costly, and the moisture variation of compositions, which have been treated with the methods, will be promptly initiated just after they are placed in an open system.

Based on the fact that most of the moisture variation in compositions occurs depending on free water, there has been employed a method for inhibiting the moisture content of the impositions by adding thereunto water-soluble high molecules such as gelatin and agar, or saccharides such as sucrose, sorbitol, α,α-trehalose, and maltitol; which all have a relatively high affinity to water (cf. *"Yogashi-Seizo-no-Kiso-to-Jissai" (The Basis and practice in processing Western confectionery,* pp. 303-372, 1991, published by KORIN Publishing Co., Ltd., Tokyo, Japan); Japanese Patent Kokai No. 56,342/97; and International Patent Kokai No. WO 02/088246). However, to meet the recent diversified life style of eating, it has been desired to explore an improved food material which has a safeness and an advantageous moisture variation inhibiting ability without deteriorating food taste, flavor, color, mouth feel (texture), etc.

### DISCLOSURE OF INVENTION

The first object of the present invention is to provide a method for inhibiting the moisture variation in compositions to prevent the following; the moisture variation in compositions may deteriorate proteins as constituents of the compositions, induce retrogradation of the gelatinized starch contained in the compositions, and proceed oxidation of the lipids contained in the compositions, followed by solidification, shrink, cracking, browning, dissolution, deliquescence, crystallization, and precipitation which may deteriorate the texture, shape, taste, flavor, color, and mouth feel of the compositions; inactivate the effective ingredients of the compositions; diminish the nutritional ingredients of the compositions; or may deteriorate or lower the properties and functions of the compositions due to bacterial contamination. The second object of the present invention is to provide compositions, where the moisture variation is inhibited, obtainable by the above method. The third object of the present invention is to provide MVIA for compositions and uses thereof.

To solve the above objects, the present inventors focused on the use of saccharides and studied a method for inhibiting the moisture variation in compositions for a relatively long period of time. As a result, they found that saccharide-derivatives of α,α-trehalose, with a superior moisture-retaining ability and being substantially free of moisture absorbency, have advantageous property of minimizing the moisture variation in a variety of compositions such as food products, cosmetics, medicated cosmetics, pharmaceuticals, daily goods, livestocks, feeds, groceries, and chemical industrial products; and established a method for inhibiting the moisture variation in these compositions by incorporating the saccharide-derivatives of α,α-trehalose, novel compositions where the moisture variation is inhibited by the method, and established MVIA for compositions and uses thereof. Thus, they accomplished this invention.

According to the present invention, the moisture variation in compositions is inhibited; the denaturation of proteins, retrogradation of starch, oxidation and decomposition of lipids, which are accompanied by the moisture variation in compositions , are inhibited; and the quality of compositions is kept for a relatively long period of time.

### BEST MODE FOR CARRYING OUT THE INVENTION

The term "compositions" as referred to as in the present invention means food products, cosmetics, medicated cosmetics, pharmaceuticals, daily goods, livestocks, feeds, groceries, and chemical industrial products; includes any of materials, intermediate materials, and products obtainable by processing the materials; and may include individual components in case that the compositions are composed of a plurality of components with different properties. In addition, the above-identified term may include plant bodies, for example, agricultural products and garden products such as vegetables, crops, lawn grasses, teas/green teas, fruits, and petals; and a part of plant bodies such as cut flowers, tea leaves, roots and stems, and root vegetables.

The term "the moisture variation in compositions" as referred to as in the present invention means a phenomenon where the moisture, i.e., mainly free water, in compositions moves within or out of the compositions, or the external moisture moves into the compositions. The moisture variation in compositions includes all phenomena such as moisture absorption and dryness induced depending on the ambient humidity where the compositions are placed, moisture transfer depending on the moisture level of adjacent another compositions, denaturation of compositions *per se* such as denaturation of proteins and retrogradation of gelatinized starch, and the moisture variation induced by the quality change of these compositions. All of these phenomena are included in the term of "the moisture variation in compositions" throughout the specification.

The saccharide-derivatives of α,α-trehalose, which are incorporated into compositions as agents for inhibiting the moisture variation in compositions used in the method according to the present invention, include anyone or more saccharides selected from non-reducing oligosaccharides composed of at least three glucose molecules having an α,α-trehalose structure intramolecularly. Concrete examples of such include those which have mono-, di-, tri- or tetra-glucose molecules bound to at least either of the glucose residues of α,α-trehalose molecule. For example, the following saccharide-derivatives of α,α-trehalose with a glucose polymerization degree of three to six, disclosed in Japanese Patent Kokai Nos. 143,876/95, 73,504/96 and 2000-228,980, and Japanese Patent No. 3,182,679, which were all applied for by the same applicant as the present invention, are preferably used; monoglucosyl α,α-trehalose such as α-maltosyl α-glucoside and α-isomaltosyl α-glucoside: diglucosyl α,α-trehalose such as α-maltotriosyl α-glucoside or α-maltosyl α,α-trehalose, α-maltosyl α-maltoside, α-isomaltosyl α-maltoside, and α-isomaltosyl α-isomaltoside; triglucosyl α,α-trehalose such as α-maltotetraosyl α-glucoside or α-maltotriosyl α,α-trehalose, α-maltosyl α-maltotrioside and α-panosyl α-maltoside; and tetraglucosyl α,α-trehalose such as α-maltopentaosyl α-glucoside or α-maltotetraosyl α,α-trehalose, α-maltotriosyl α-maltotrioside, and α-panosyl α-maltotrioside.

These saccharide-derivatives of α,α-trehalose can be used independently of their origins and processes, and include any of those which are produced by fermentation method, enzymatic method, and organic synthetic method. For example, those which are arbitrarily produced directly from starch or partial starch hydrolyzates by the methods disclosed in Japanese Patent Kokai Nos. 143,876/95, 73,504/96 and 2000-228, 980, and Japanese Patent No. 3,182,679, which were all applied for by the same applicant as the present invention; those which are produced in such a manner of preparing partial starch hydrolyzates with an improved content of specific oligosaccharides such as maltotetraose, maltopentaose, maltohexaose, and maltoheptaose by using maltotetraose-forming amylase disclosed in Japanese Patent Kokai No. 143,876/95, α-amylase capable of forming maltopentaose in a higher yield disclosed in Japanese Patent Kokai No. 14,962/95, or maltohexaose/maltoheptaose-forming amylase disclosed in Japanese Patent Kokai No. 236,478/95; and contacting the partial starch hydrolyzates with a non-reducing saccharide-forming enzyme disclosed in Japanese Patent Kokai No. 143,876. The saccharide-derivatives of α,α-trehalose can be arbitrarily produced by contacting solutions, which contain starch or partial starch hydrolyzates, with enzymes such as cyclodextrin glucanotransferase capable of transferring glycosyl residues. The resulting reaction mixtures thus obtained can be arbitrarily used intact or after partially or highly purified, as a solution with saccharides containing saccharide-derivatives of α,α-trehalose. Since the above-identified methods produce saccharide-derivatives of α,α-trehalose in a higher yield and at a lesser cost, they can be advantageously used on an industrial scale.

Among the above saccharide-derivatives of α,α-trehalose, particularly, saccharides having a trehalose structure at the end of their molecules, such as monoglucosyl α,α-trehalose, α-maltosyl α,α-trehalose, and α-maltotriosyl α,α-trehalose, can be advantageously used in the present invention because of their relatively strong moisture variation inhibiting action. Examples of such saccharides are those which contain, as a main ingredient, α-maltotriosyl α-glucoside or α-maltosyl α,α-trehalose along with one or more other α-maltosyl α-glucoside or α-glucosyl α,α-trehalose, α-tetraosyl α-glucoside or α-maltotriosyl α,α-trehalose, and α,α-glycosyl α-glucoside or α-glycosyl α,α-trehalose, as disclosed in Japanese Patent Kokai No. 143,876/95; particularly, those which contain α-maltosyl α,α-trehalose in an amount of at least about 5% by weight (hereinafter the term "% by weight" is abbreviated as "%", unless specified otherwise), preferably, at least about 10%, more preferably, at least about 30%.

As disclosed in Japanese Patent No. 3,182,679 and Japanese Patent Kokai No. 2000-228,980, among the above-identified saccharides, α-maltosyl α-glucoside and α-maltotetraosyl α-glucoside in the form of a crystal have been well known. However, to exert the moisture variation inhibiting effect according to the present invention, for example, the above saccharides should preferably be used in an amorphous form such as a syrupy or glass form.

In the method for inhibiting the moisture variation in compositions according to the present invention, the saccharide-derivatives of α,α-trehalose incorporated into the compositions as an effective ingredient are preferably those in an amorphous form; any of those which are in any form of a syrup, massecuite, paste, powder, solid, granule, or tablet can be arbitrarily used intact or after mixed with any of fillers, excipients, and binders and shaped into a granule, sphere, short-rod, plate, cubic, or tablet form.

In practicing the method for inhibiting the moisture variation in compositions, the saccharide-derivatives of α,α-trehalose used as effective ingredients are incorporated into the objective compositions to exert a desired effect. In any fields, such saccharide-derivatives can be used from the stage of handling the materials for the compositions and to the stage of obtaining the final products, considering the percentage of components of the compositions and their final use.

In the method for inhibiting the moisture variation in compositions according to the present invention, the saccharide-derivatives of α,α-trehalose as effective ingredients should be incorporated into the contents during any processes up to completion of the final compositions or into the final products. Examples of such incorporation method can be arbitrarily selected from mixing, kneading, dissolving, melting, spreading, suspending, emulsifying, reversed micelling, penetrating, crystallizing, sprinkling, applying, adhering, spraying, coating, injecting, soaking, solidifying, and incorporating. The amount of the MVIA of the present invention to be incorporated into compositions is not specifically restricted as long as it is an amount effective for inhibiting the moisture variation in the compositions, usually at least 0.5%, desirably, at least 5%, and most desirably, at least 10% to the total amount of each of the compositions, on a dry solid basis (d.s.b.). In general, the amount less than 0.5% is not sufficient to inhibit the moisture variation in compositions. The upper limit of the amount of the saccharide-derivatives of α,α-trehalose to be incorporated should not be limited as long as it does not hinder the functions or the objective use of the compositions. In practicing the method for inhibiting the moisture variation according to the present invention, when used for coating the surface of a composition by soaking it in a liquid such as *" tare"* (a kind of seasoning for foods) with the saccharide-derivatives of α,α-trehalose, or by applying or spraying such a liquid to the composition, the saccharide-derivatives should not be incorporated into the composition in an amount of 0.5% or more to the total amount of the composition, but sufficient in an amount of at least about 0.5%, desirably, about 5%, and most desirably, about 10%. In the case of treating by heating vegetables, fruit, fish and shell, and spawns thereof for branching, preferably used are solutions of the saccharide- derivatives of α,α-trehalose, having a concentration of at least about 0.5%, desirably, about 2%. The saccharide-derivatives of α,α-trehalose in a powder- or a high concentration solution-form can be directly sprinkled or coated over the surface of compositions or can be used to inhibit the moisture variation of compositions by wrapping them, for example, with papers having a high moisture retaining ability incorporated with a solution of any of the saccharide-derivatives. Concrete examples of such are as follows: Heat an about 50 to about 70% syrup containing about 10 to about 50% of MVIA of the present invention and about 10 to about 50% of other saccharide(s) to about 80°C; add the heated syrup to rice just after cooking or noodles after boiling and draining in an amount of about 5 to about 80% thereof, desirably, about 15 to about 40%; stir the rice or noodle lightly, heat the resultant without giving a remarkable temperature change by allowing the heated resultant to settle or further heating it for about one to about one and half hours while keeping alone or stirring in order to incorporate the above saccharides into a food product to attain the effect of the present invention.

MVIA containing the saccharide-derivatives as effective ingredients of the present invention preferably contains the effective ingredients in an amount of at least about 10%, desirably, at least about 20%, and more desirably, at least about 30% to the total amount of the agent, on a dry solid basis.

MVIA, which contains either a saccharide-derivative(s) of α,α-trehalose or a saccharide composition containing such a saccharide-derivative(s), inhibits the quality deterioration of compositions induced by the denaturation of proteins resulted from moisture variation, retrogradation of gelatinized starch, oxidation and decomposition of lipids, sticking, and texture change of the compositions; and improves the deposit property, because the above saccharide-derivative(s) or the saccharide composition inhibits the moisture variation in the compositions and keeps the water content therein at a constant level. Since the above saccharide-derivative(s) or the saccharide composition generally has a lesser sweetness, well harmonizes with other substances with acid taste, saltiness, astringency, *umami* (favorable taste), bitterness, or the like, and has a relatively high acid- and heat-tolerance, they can be advantageously used as MVIA to prevent moisture absorption, syneresis, and sticking, as well as imparting water holding capacity, savory flavor, and body; and they can be arbitrarily used as a quality-improving agent for food products in general. Since the above-identified saccharide composition has a relatively high level of moisture variation inhibiting ability, it can be arbitrarily applied or sugar-coated over the surface of baked confectionery including rice confectionery, cookies, biscuits, pies and cakes; other confectionery such as chocolates, candies, gums, gummy candies, and jellies; nuts such as coffee beans and almonds; foods such as cereals, sesames, and *Porphyra umbilicalis;* tablet-like candies; health foods; foods for special use; and pharmaceuticals in a tablet form, while retaining the moisture content in the above products at an appropriate level and promptly allowing the surface of the products to a dried condition when used to coat the products or made into a syrup for soaking the products. In particular, when nuts such as almonds and seeds such as cereals and coffee beans, and sesames are sprayed with, soaked in, or coated with a syrup containing a saccharide-derivative(s) of α,α-trehalose before, during, or just after roasting, the moisture variation of and the oxidization of lipids in the resulting roasted products will be well inhibited. Because of this, the roasted products will keep the desired flavor and taste just after their processings for a relatively long period of time. In the case of sugar-coating the above confectionery, foods, and pharmaceuticals, MVIA and α,α-trehalose are preferably used in combination as a syrup for such sugar coating, i.e., a substrate for sugar coating, which may be optionally used together with sugar alcohols such as sorbitol and maltitol; maltooligosaccharides such as glucose and maltose; binders such as gum arabic, hydrolyzates of guar gum, and pullulan; sweeteners with a relatively high sweetening power; flavors; and pigments. The content of each of the saccharide-derivatives of α,α-trehalose and α,α-trehalose, which are contained in the syrup, is not specifically restricted as long as it can be used in hard-, semi-, soft-, or fondant-sugar-coating. Usually, the former content is preferably in an amount of 5 to 30%, desirably, 10 to 20%; and the later one, 70 to 95%, d.s.b. The binder(s) is usually used in an amount of 0 to 0.5%, preferably, 0 to 0.2%. The syrup preferably has a concentration of 50 to 70% to the total sugars, d.s.b. , and it is used for sugar coating at a temperature of 45 to 70°C with a blowing air warmed to about 20 to about 45°C.

MVIA of the present invention can be arbitrarily used, for example, in a variety of seasonings and sweeteners such as amino acids, peptides, soy sauces, powdered soy sauces, *miso,* powdered *miso, "moromi"* (a refined sake), *"hishio"* (a refined soy sauce), *"furikake"* (a seasoned fish meal), mayonnaises, dressings, *"funmatsu-sushi-su"* (powdered vinegar for sushi), *"chuka-no-moto"* (an instant mix for Chinese dish), sauces, catsups, *"yakiniku-no-tare"* (a sauce for Japanese grilled meat), curry roux, instant stew mix, instant soup mix, *"dashi-no-moto"* (an instant stock mix), liquids for treating processed meet for aging and retaining its quality, pickles for ham, seasonings for processing fish, shell, and fish eggs, nucleic seasonings, mixed seasonings, *"mirin"* (a sweet sake), *"shin-mirin"* (a synthetic mirin), table sugar, and coffee sugar.

MVIA of the present invention can be arbitrarily used, for example, in rice confectionery such as *"senbei"* (a rice cracker), *"arare"* (a rice-cake cube), and *"okoshi"* (a millet- and rice cake); Japanese confectionery such as *"gyuhi"* (starch paste), *"monaka"* (a beam- jam-filled wafer), *"mochi"* (a rice paste), "*ohagi*" (a rice dumpling covered with bean jam), *"manju"* (a bun with a bean jam), *"karukan"* (a sweetened jelly of yam and rice flour), *"uiro"* (a sweet rice jelly); an such *"tsubu-an"* (a jam prepared by using different beans), *"koshi-an"* (a strained bean jam), and *"kagou-an"* (a kind of *an);* Japanese confectionery such as *"yokan"* (a sweet jelly of beans), *"mizu-yokan"* (a soft adzuki-bean jelly), *"kingyoku"* (a kind of yokan), *"kintsuba"* (a kind of baked Japanese confectionery), sweet potato, jelly, bavaroise, *pao de Castella,* gong cake , *"karintou"* (a fried dough cake), and *"amedama"* (a Japanese toffee) ; Western confectionery such as baked confectioneries including a biscuit, cookie, cracker, pie, cream puff, waffle, sponge cake, doughnut, and pastry, as well as soft candies, hard candies, fondants, and icings including a pudding, butter cream, custard cream, chocolate, chewing gum, nougat, jelly beans, caramel, and marshmallow; snack confectionery; cereals; center liquid confectionery; meringue confectionery; bread such as a *"shoku-pan"* (a Japanese-style loaf of bread), bun, *"an-pan"* (a bean jam bun), and muffin; syrups such as a syrup, *"korimitsu"* (a sugar syrup for shaved ice), and other syrups with coffee, cocoa, green tea or *"matcha"* (a ground tea); pastes such as a *"flour paste"* (a bread or confectionery filled or coated with cream etc.), peanut paste, fruit paste, spread, and other pastes with coffee, cocoa green tea or *matcha,* as well as vegetable pastes; fruit-processed foods such as a jam, marmalade, preserves, fruit preserved in syrup, *"toka"* (a conserve), fruit pieces, and fruit sauce; processed foods of vegetables such as sprouts and juices of vegetables including cut pieces of vegetables, for example, a bean sprout, soybean sprout, green shoot of buckwheat, *"kaiware-daikon"* (a Japanese radish in an early stage after budding), alfalfa sprout, and sprouting broccoli, as well as other boiled foods of sprouting plants or vegetables such as a butter-type lettuce, *kaiware-daikon*, alfalfa, bean sprout, and sprouting broccoli; germs of cereals such as a wheat, rice, buckwheat, and corn; hypocotyl of beans such as a soybean and adzuki beans, and processed products thereof; pickled products such as a *"fukujin-zuke"* (a red colored radish pickle), *"bettara-zuke"* (a kind of whole fresh radish pickles), *"senmai-zuke"* (a kind of sliced fresh radish pickles) and *"rakkyo-zuke"* (a pickled shallot); premixes for pickles and pickled products such as a *"takuan-zuke-no-moto"* (a premix for pickled radish), *"hakusai-zuke-no-moto"* (a premix for fresh white rape pickles), and *"ume-boshi"* (a dried Japanese apricot after pickled), as well as premixes thereof for pickling such as *"asazuke-no-moto"* (an instant premix with seasonings for pickling fresh vegetables); cooked rice products such as a cocked rice, rice ball, cooked rice with adzuki beans, rice gruel, rice seasoned with vinegar, seasoned steamed rice with vegetables and meat, and precooked rice; processed bean products such as a soy-milk, soybean curd, freeze-dried bean curd, *"natto"* (a fermented soybean with *Bacillus natto),* sweetened dried soybean, and black soybean; noodles such as a Japanese wheat noodle, Japanese buckwheat noodle, Chinese noodle, and pasta; *"okonomi-yaki"* (a Japanese-style pancake containing vegetables and other foodstuff), *"takoyaki"* (a Japanese-style pancake containing octopus, i.e. , octopus dumpling), *"taiyaki"* (a Japanese-style pancake baked in a mold with a shape of sea bream), crepe, croquette, jiao-zi, shao mai, spring roll, ham, and sausage; processed fish meat such as a fish ham, fish sausage, *"kamaboko"* (a processed fish meat), *"chikuwa"* or fish stick, and *"tenpura"* (a Japanese deep-fat fried fish paste); *"chinmi"* (relish) such as *"uni-no-shiokara"* (salted guts of sea urchin), *"ika-no-shiokara"* (a salted gut of squid), *"su-konbu"* (a processed tangle), *"saki-surume"* (a dried squid strip), *"fugu-no-mirin-boshi"* (a dried mirin-seasoned swellfish), fish eggs of flying fish and salmon, as well as seasoned sea lavers, seasoned small fish with optional drying; sauces for seasoning a grilled meat, grilled eel, rice dumpling, and baked rice cake; foods boiled in soy sauce produced from sea lavers, wild plants, dried squids, small fish, and shellfish; daily dishes such as boiled and seasoned beans, potato salad, and rolled tangles; fresh eggs and processed egg products such as boiled eggs, omelette, grilled eggs, *"dashimaki-tamago"* (a rolled fried egg), *"chawan-mushi"* (a pot-steamed hotchpotch), egg york, and egg white; milk products such as cheese and yogurt; fresh fish and meat, fruit, and vegetables, and their frozen, cooled, chilled, retort-pouched, dried, freeze-dried, and heat-processed products; canned or bottled vegetables; premixes such as a pudding premix, hot cake mix, and butter mix; instant foods such as instant *"shiruko"* (boiled adzuki beans with rice paste), and instant soup; solid foods; therapeutic foods; peptide foods; alcohols such as *sake,* synthetic *sake,* liqueur, Western alcohols, beer, and sparkling liquor; and beverage such as teas including green tea, coffee, cocoa, juice, carbonated drinks, milk beverage, lactic acid beverage, and lactic acid bacterial drinks, as well as their concentrates for business use, optionally processed in a portion type for use after diluting it with water or hot water prior to use. The saccharide-derivativesof a,a-trehalose,used asan effective ingredient in MVIA of the present invention, and the saccharide compositions containing thereof had been found to have a relatively high glass transition temperature. Then, it was revealed that, when incorporated into compositions, unlike starch hydrolyzates in general, the saccharide-derivatives or the saccharide compositions increase the glass transition temperatures of the compositions. As a result, it was found that MVIA can be advantageously used in improving the storage property of vitrificated compositions. As described, for example, in *"Shokuhin-to-Garasuka-Kesshoka-Gijutsu" (Foods and vitrification*/*crystallization technology",* pp. 3 to 60, 2000, published by Science Forum Inc., Tokyo, Japan, most of compositions including foods are prepared by using the property of such vitrification. Since such vitrification is closely related to the property of storage and preservation of the compositions, it is an essential factor for formulating foods. The vitrification of compositions is caused by solidification thereof without crystallizing from their solutions or melted forms, and thus the property of the resulting products generally is varied depending on the temperature and the moisture content thereof; in the case that the compositions are foods, as the increase of the temperature and the moisture content, foods change from their glass state to rubber state and become viscus, where the temperature exhibiting such change is called a glass transition temperature. When placed in a condition with a temperature higher than a glass transition temperature, any of the vitrificated compositions become soft and feasible to show quality change such as adhesion and stickiness, deterioration induced by chemical and enzymatic reactions, and reduction of viable cells. While the vitrificated compositions may be easily melted or cause crystallization when placed in a higher temperature condition than those of their glass transition temperatures. On the contrary, they are characteristically stable in quality because they are hard, crispy, and free of stickiness when placed in a lower temperature condition than those of their glass transition temperatures, and they have a greatly reduced physicochemical molecular movement, resulting in being substantially free from chemical reactions such as the Maillard reaction and oxidation reaction, as well as enzymatic reactions by amylase, protease, etc. Accordingly, it is speculated that the property of enhancing the temperature of the glass transition temperature of the saccharide-derivatives of α,α-trehalose would greatly contribute to their moisture variation inhibiting actions, and that the stability of compositions would be improved by maintaining the vitrification state of the compositions even under a relatively high temperature to inhibit the movement of water molecules and to inhibit the change of the compositions. Representative examples of compositions using vitrification include candy coatings, for example, those for Chinese sweet potatoes and candies such as hard candies; fondants; candy suckers molded on a griddle; cotton candies; cookies; chocolates; *"okoshi"* (a kind of rice cake); *"karinto"* (a fried flour paste coated with molasses); coatings for *"tenpura"* (a Japanese deep-fat fried fish paste); pasta; noodles; precooked rice; snack confectionery; dried bean curd; freeze-dried bean curd; *"yakifu"* (a baked mixture of proteins separated from wheat and strong wheat) ; dried bonito; margarine; fat spread; frozen foods such as frozen confectionery such as ice creams, as well as frozen fish paste; freeze-dried foods; spray-dried foods; spray-concentrated foods; edible films using pullulan, etc.; dried or frozen bacteria including those which are contained in yogurt, fermented foods such as pickled products); microorganisms such as viruses ; plant seeds; animal cells, tissues, organs, and bowels. In the case of using the saccharide-derivatives of α,α-trehalose as a glass transition temperature-increasing agent for compositions (hereinafter, called "vitrification agent" throughout the specification), they can be arbitrarily used alone or optionally in combination with substances, which have an advantageous improving effect on the glass transition temperature of the compositions, for example, saccharides such as α,α-trehalose, and polysaccharides. The amount of the saccharide-derivatives of α,α-trehalose added to compositions is not specifically restricted as long as it does not hinder the effect and function of the present invention and effectively increases the glass transition temperatures of the compositions. When applied to candies, the above saccharide-derivatives are added to each candy in an amount of at least about 12%, desirably, at least about 18%, most preferably, at least about 24% to the total amount of the saccharides in each candy. In general, the amount of less than about 6% is not sufficient to effectively improve the glass transition temperatures of candies.

MVIA of the present invention can be used as an agent for inhibiting the moisture variation in feeds and baits for the following animals and insects to keep the quality thereof; domestic animals, poultry, pets, bees, silk worms, fishes, crustaceans including shrimps and crabs, echinoderms such as sea urchins and sea cucumber; and larva, under-growth, and grown-up pet animals such as insects. Further, the agent can be arbitrarily used to inhibit the transpiration and to keep the freshness of the body of germed or infant plants and their roots, as well as infant body of transplanted mycelia. In addition, MVIA of the present invention can be used in the following preferences, cosmetics, medicated cosmetics, and pharmaceuticals in the form of a solid, paste, or liquid to inhibit their moisture variation and to stably keep their quality; tobaccos, tablets, troches, drops of liver oil, creams for cosmetics, medicated cosmetics, and medicals, as well as shampoos, hair rinses, foundations, lip sticks, lip creams, solid or liquid dentifrices , mouth refreshments , cachous, gargles, eye drops, detergents for eyes and noses, soaps, and cleaners.

Also MVIA of the present invention can be used in effective ingredients such as biologically active substances susceptible to lose their activities, as well as in health foods, cosmetics, medicated cosmetics, pharmaceuticals, feeds, pet foods, and baits, which contain the active ingredients, in the form of a liquid, paste, or solid, without losing the activity of the effective ingredients and stably keeping their quality for a relatively long period of time. Examples of such biologically active substances are lymphokines such as α-, β- and γ-interferons, tumor necrosis factor-α (TNF-α), tumor necrosis factor-β (TNF-β), macrophage migration inhibiting factor, colony-stimulating factor, transfer factor, and interleukin 2 (IL-2); hormones such as insulin, growth hormone, prolactin, erythropoietin, and follicle-stimulating hormone; biological preparations such as BCG vaccine, Japanese encephalitis vaccine, measles vaccine, live polio vaccine, smallpox vaccine, tetanus toxoid, Trimeresurus antitoxin, and human immunoglobulin; antibiotics such as penicillin, erythromycin, chloramphenicol, tetracycline, streptomycin, and kanamycin sulfate; vitamins such as thiamine, riboflavin, L-ascorbic acid, carotenoid, ergosterol, tocopherol, pyrroloquinoline quinone, and derivatives thereof; vitamin-containing products such as cod liver oil; enzymes such as lipase, elastase, urokinase, protease, α-amylase, isoamylase, glucanase, and lactase; extracts such as crude-drug ginseng extract, snapping turtle extract, chlorella extract, aloe extract, propolis extract, *Agaricus, Reishi* mushrooms or microorganisms of the genus *Ganoderma* including *Ganoderma lucidum, Phellinus liteus,* and *Houttuynia cordata,* as well as theirmycelia used as materials for the above extracts; herbs; processed products of plant and animal bodies; live viruses, lactic acid bacteria, and yeasts; biologically active substances such as royal jelly and purified products thereof; and other compositions containing any of these products.

Since MVIA of the present invention is stable *per se,* it can be arbitrarily used in agricultural, fishery, and livestock products and processed products thereof as an osmosis-controlling agent, excipient/gloss-imparting agent/shape-retaining agent for pharmaceutical preparations, retrogradation-preventing agent for gelatinized starch, denaturation inhibiting agent for proteins, freshness-retaining agent, fading or falling down-preventing agent for vegetables and cut flowers, agent for inhibiting the metmyoglobin formation and the sulfide blackening in fish meat such as fillets and shrimps removed their heads. MVIA of the present invention can be also used as an excipient or stabilizer for effective ingredients for chemical industrial products including pesticides. MVIA of the present invention can be advantageously used as a sweetener, taste-improver, or quality-improver for compositions such as foods. Since MVIA has a strong moisture-retaining ability, it can be advantageously used in place of glycerol in food products, cosmetics, medicated cosmetics, pharmaceuticals, daily goods, feeds, pet foods, baits, groceries, and chemical industrial products.

By using MVIA of the present invention alone or in combination with other conventionally well known growth-promoting agents and/or surfactants, it can be directly sprayed over plant leaves, stems, roots, flowers, and seeds of agricultural fruiters and garden plants including lawn grasses, tea trees, rice plants, wheat plants, corn plants, vegetables, and flowers; or sprayed into soils around the above plants, whereby the moisture variation in the above plants is effectively inhibited to impart them freezing tolerance, drying tolerance, and salt tolerance. Accordingly, MVIA can be used to protect plants from damages of dryness, frost, and salts; promote plant growth; activate and protect plant cells; and/or to increase the yield of the plant bodies *per se,* and the yields of fruit, seeds, and cereals and the sugar content in fruit. In applying to the above plants, the surfactants usable in combination with MVIA of the present invention are not specifically restricted as long as they do not affect the plants; polyoxyethylene alkyl ether and dialkyl sulfonate succinate alkylammonium salt are preferably used.

MVIA of the present invention can be the saccharide-derivatives of α,α-trehalose as effective ingredients of the agent as long as they exert the desired effect on compositions, and they optionally contain the following saccharides other than the above derivatives; glucose, isomaltose, maltose, oligosaccharides, and dextrins, which are prepared from starch during the process of producing the above derivatives. MVIA can be those which are obtained by hydrogenating a saccharide mixture of the saccharide-derivatives of α,α-trehalose and other reducing saccharides to convert the coexisting reducing saccharides into sugar alcohols. To improve the moisture variation inhibiting action, MVIA can be optionally advantageously used in combination with water-soluble polysaccharides such as gum arabic, guar gum, carrageenan, pectin, hemicellulose, or pullulan.

Comparing with reducing partial starch hydrolyzates, the saccharide-derivatives of α,α-trehalose as effective ingredients of MVIA of the present invention are stable and low in reducibility. Then the saccharide-derivatives do not impart color or browning to other materials, particularly, amino acids and substances containing the same such as oligopeptides, polypeptides, and proteins, even when mixed or processed therewith; and do not cause undesirable taste and smell and scarcely spoil the mixed materials. Unlike reducing partial starch hydrolyzates, the saccharide-derivatives of α,α-trehalose have a lesser reducibly and lower viscosity with a smooth viscosity free of stickiness; have no gelatinized starch smell inherent to dextrin while retaining dextrin-like properties; have lower moisture absorbency, readily dryness, and quick solubility; and have a high-quality, and mild sweetness with a lower sweetening power. Therefore, the saccharide-derivatives of α,α-trehalose *per se* can be used as MVIA for a variety of compositions and also used as a saccharide substitutable for dextrin. If necessary, depending on the purpose of increasing the spreadability and bulking, the saccharide-derivatives of α,α-trehalose can be arbitrarily used together with one or more substances of reducing saccharides other than the above identified saccharides, non-reducing saccharides, sugar alcohols, highly sweetened sweeteners, water-soluble polymers, organic acids, inorganic acids, salts, emulsifiers, antioxidants, and chelating substances; optionally, they can be further used in combination with adequate amounts of one or more ingredients of conventional colors, flavors, preservatives, acids, taste-imparting materials, sweeteners, stabilizers, fillers, alcohols, and water-soluble high molecules. Concrete examples of such are powdered starch syrup, glucose, maltose, sucrose, paratinose, α,α-trehalose, neotrehalose, isotrehalose, isomerized sugar, honey, maple sugar, glycosyl sucrose, isomaltooligosaccharides, galactooligosaccharides, fructooligosaccharides, gentiooligosaccharides, nigerooligosaccharides, kojioligosaccharides, galactosylglucoside, lactosucrose, reducing or non-reducing saccharides such as cyclic tetrasaccharides (or "cyclotetrasaccharide") and/or saccharide-derivatives thereof which are disclosed in International Patent Publication Nos. WO 02/24832, WO 02/10361 and WO 02/072594 applied for by the same applicant as the present invention, and sugar alcohols such as erythritol, xylitol, sorbitol, maltitol, lactitol, and panitol. MVIA can be arbitrarily used as a crystallization controlling agent for saccharides relatively susceptible to crystallizing, such as sucrose and α,α-trehalose, by adding thereunto to inhibit the crystallization and the growth of crystals of such saccharides, and to impart the desired mouth feel, color and gloss, depending on the final use of the saccharides.

Compared with saccharides and sugar alcohols to be used with the saccharide-derivatives of α,α-trehalose as effective ingredients for MVIA of the present invention, the saccharide-derivatives may have a larger molecular weight. In such a case, the saccharide-derivatives can be used by mixing with other appropriate saccharides to impart to have the desired property, within the limitation that the saccharide-derivatives will not be affected. In general, non-reducing saccharides, for example, α,α-trehalose or maltitol, are preferably used, and the amounts thereof are not specifically restricted as long as they do not affect the function of MVIA of the present invention, and usually in an amount of, on a dry solid basis, less than about 0.5 part by weight, desirably, less than about one part by weight, more desirably, less than about 0.3 part by weight to one part by weight of the saccharide-derivatives of α,α-trehalose. When used in compositions such as fondants and meringues which contain α,α-trehalose as a main ingredient and require crystallized α,α-trehalose and an appropriate moisture content, the saccharide-derivatives of α,α-trehalose can be preferably used in a lesser amount than that of α,α-trehalose.

In addition to the above saccharides and sugar alcohols, MVIA of the present invention can be further used after admixing with one or more of highly sweetened sweeteners such as dihydrochalcone, stevioside, α-glycosyl stevioside, rebaudioside, glycyrrhizin, L-aspartyl-L-phenylalanine methyl ester, acesulfam K, sucralose, and saccharin, as well as other sweeteners such as glycine, alanine, and salts thereof. If necessary, fillers such as dextrin, starch, and lactose can be used by mixing. Further, MVIA of the present invention can be used in combination with one or more of organic acids such as lactic acid, citric acid, sodium citrate, and salts thereof; polyphenols such as saponin, isoflavon, flavonoid, catechin from tea, grape fruit seed extract, and saccharide-transferred products thereof; alcohols such as ethanol; and water-soluble high molecules such as levan, sodium alginate, agar, gelatin, casein, methyl cellulose, carboxymethyl cellulose, poly(vinyl alcohol), poly(vinylpyrrolidone), and polydextrose. In the case of using the saccharide-derivatives of α,α-trehalose in combination with the above-identified organic acids and salts thereof and/or alcohols, they can be used as agents for inhibiting the growth of microorganisms.

Particular problems arising in the field of food products are the quality deterioration and the reduction of preference of food products , which are induced by the oxidation or the decomposition of lipids due to their constitutive main ingredients and processes, the retrogradation of gelatinized starch, the denaturation of proteins, and the texture change after moisture absorption or release. While, since the saccharide- derivatives of α,α-trehalose have the above mentioned various functions, they not only inhibit the moisture variation in these food products but have the following advantageous features; they can be used as an agent for inhibiting the oxidation or decomposition of lipids in lipid-containing foods such as confectionery prepared with oils, butter creams, caramels, fried noodles, or coffee whiteners; used as a moisture-absorption inhibiting agent, agent for inhibiting stickiness to teeth, shape-retaining agent, and coating to form a thin layer with a satisfactory gloss or sugar coating in candy-type foods such as hard candies, soft candies, candies for Chinese sweet potatoes, and cotton confectionery; used as a shape-retaining agent to effectively inhibit changing in shape, the loss of crispy mouth feel when one bites the surface of the following baked foods , and the occurrence of stickiness induced by the moisture from the foods when *"takoyaki"* (a Japanese-style pancake containing octopus, i.e., octopus dumpling), *"taiyaki"* (a Japanese-style pancake baked in a mold with a shape of sea bream), *"okonomi-yaki"(a* Japanese-style pancake containing vegetables and other foodstuffs), and crepes are allowed to stand or wrapped; used as a moisture-retaining agent, glaze, gloss-imparting agent, or adhesiveness enhancer in sea urchins, fishes , crustacean including shrimps and crabs, echinoderms such as fish eggs such as of sea urchins, flying fishes, and red caviars, seasoned-dried fishes, dried fish larvae, boiled-dried fishes, scaled sardines soaked in vinegar, *"shiokara"* (salted guts of fishes), shrimps, fishes such as yellowtail amberjacks, yellowtails, Japanese sea bassms, sea breams, Pacific cods, large scale black fish, jacks, sardines, mackerels, and pacific herrings, as well as marine foods and processed products thereof such as fillets; used as an agent for inhibiting metmyoglobin formation or denaturation when processed vegetables and fruits and proteinaceous foods including eggs and meat such as beef and pork are subjected to heat treatment including branching and any of the treatments of drying, freezing, freeze-drying, frozen/cold/chilled storage, or ambient temperature storage; used as a color-change preventive to prevent browning or fading in vegetable juice, coffee, tea beverage including tea, green tea and *"matcha"* (a ground green tea), syrups containing concentrates of the aforesaid beverage, pigments, i.e., chlorophyll and flavonoids, containing foods such as pastes of vegetables, fruit, coffee, tea, green tea, *and matcha;* used as a flavor-retaining agent in pastes or massecuites of spices such as Japanese horseradish, mustard, garlic, nutmeg, and herbs; and used as a shape-retaining agent to prevent the deformation of foods by boiling in boiled foods and retort pouched foods, i.e., foods treated withhigh-pressure-heat-treatment, such as mackerels boiled with "*miso*", Japanese hotchpotch including materials thereof, and *"nabemono"* (a winter cuisine served in a pot). MVIA of the present invention can be arbitrarily used in amylaceous foods such as pastas, noodles, instant noodles, cocked rice products, boiled sweet potato paste, *"dango"* (a boiled starch paste), *"ohagi"* (a kind of rice paste), *"an"* (a bean-jam), *"mochi"* (a rice paste), and *"manju"* (a bun with a bean-jam) as a retrogradation-preventing agent including those which contain amylase preparations such as α-amylase and β-amylase, crystallization preventive for *an* with sucrose, and agent for preventing adhesiveness between the products each and/or between the products and wrapping materials such as parchment papers, aluminum foils , and leaves such as of *Quercus dentata.* In order to impart an effect as a binder to MVIA of the present invention, it can be arbitrarily used in combination with water-soluble arabic gum, guar gum, carrageenan, hemicellulose, or pullulan.

Since food products generally contain lipids, starch and proteins, MVIA of the present invention has a function of retaining their inherent flavor, taste, pleasant smell, color, and mouth feel whenever applied to any of the above food products. Thus, the agent can be used as a flavor- and taste-retaining agent. When used in confectionery and bread, such as *"monaka", "manju", "an-pan"* (a bread with adzuki-beans paste), and cookies with sandwiched jams, which are prepared using *"an"* with a relatively high moisture content and constructed with at least two layers with different moisture contents, MVIA effectively inhibit the moisture variation in such layers and the dryness of the *an* and *jams* contained therebetween, as well as the stickiness of the contents of *monaka* and the dough of *manju* and biscuits. As a result, the texture of these food products just after baking will be retained for a relatively long period of time.

By replacing a part or the whole of conventionally used dextrins, etc., with the saccharide-derivatives of α,α-trehalose for use as substrates for pulverization, they can be mixed with foods, cosmetics, medicated cosmetics, pharmaceuticals, and their materials or intermediates, such as vegetable juice, processed vegetable juice, fruit juice, coffee, extracts of tea, green tea, *and matcha,* seasonings, milk, whole egg, egg york, egg white, oil and fat, amino acids, vitamins, minerals, flavors, pigments, health supplement foods, and functional substances used as pharmaceuticals; and then the resulting mixtures are dried with drying methods such as spray drying, freeze drying, and heat drying to facilitate to obtain high quality powdery compositions such as powdered vegetable juice, coffee, tea, green tea, and *matcha;* powdered seasonings such as Japanese horseradish, mustard, and garlic; powders such as powdered herbs, powdered seasonings, powdered milk, powdered eggs , powdered oil and fat, powdered vitamins, powered minerals , powdered DHA, powdered essential oils including pepper mint oil; powdered flavors; and powdered pigments. The above powdery products can be arbitrarily processed into products in the form of a solid such as a granule or tablet, and optionally in the form of a solution, syrup, paste or massecuite. Since these compositions are well inhibited in moisture absorbency and/or moisture releasability by the action of the saccharide-derivatives of α,α-trehalose, they keep their satisfactory flavor, taste and functions just after their processings even after a relatively long term storage, and have an imparted property of being readily dissolved in cold or hot water as compared with conventional compositions prepared with dextrin as a powdered base. When pulverized, essential oils and flavors, for example, oils such as herbs, fruit oils of citrus fruits such as lemon oil and grape fruit juice oil, as well as isothiocyanate, derivatives thereof, fatty acids including DHA and EPA, and lipids composed of fatty acids, which are all insoluble or substantially insoluble in water, can be arbitrarily admixed with conventionally known emulsifiers such as gums and sucrose fatty acid ester, emulsified the resulting mixtures, and drying the emulsified mixtures with conventional methods such as vacuum drying and spray drying. Since the powdery products thus obtained contain the saccharide-derivatives of α,α-trehalose, to the products have been imparted the quality of emulsified ingredients will be retained for a relatively long period of time, and the unsatisfactory taste and flavor inherent to the added emulsifiers will be inhibited.

Since the water change inhibiting agent of the present invention imparts a smooth mouth feel to foods, low calorie foods substitutable for lipids with a lesser calorie can be produced by replacing a part or the whole of fatty-acid-containing foods such as frozen desserts, creams, dressings, coffee whiteners, cakes, and biscuits, while retaining the smooth mouth feel inherent to lipids. In preparing food products such as the above-mentioned lipid-containing foods, bread, ice creams, and mayonnaises, more desired products which have a satisfactory taste, flavor and mild mouth feel can be prepared by using MVIA of the present invention by using a lesser amount of such emulsifiers or even prepared without them. In such a case, the amount of MVIA of the present invention is not specifically restricted and any amount sufficient to produce products with a similar mouth feel to that of those which are prepared by using a lesser amount of such emulsifiers or even prepared without them; usually, a preferable amount of MVIA is 3 to 30% to the total weight of the final food product. Combination use of MVIA with α,α-trehalose will provide food products with an improved mouth feel by substituting α,α-trehalose for the total sugars in an amount of 10 to 90%, desirably, 50 to 80% of the sugars.

In addition to the above features, MVIA of the present invention has an anti-inflammatory action, an improved effect on protecting cells from external stresses such as heat, ultraviolet ray, and drying, and a cell activating action. Then, even when incorporated alone into pharmaceuticals, medicated cosmetics, and cosmetics, the agent augments the cellular metabolism of the skin damaged or induced inflammation by ultraviolet ray and other factors, inhibits the aging and inflammation of the skin, and promotes the recovery of the affected skin to the normal conditions. Further, MVIA alleviates both the stimulation by using the ingredients contained in these external dermal preparations or the absorption-promoting agents in combination, and the physical stimulation, induced when the external dermal preparations are administered to the skin or the mucus membrane by the methods such as applying and dropping. Because of these, the pharmaceuticals, medicated cosmetics, and cosmetics containing MVIA can be advantageously applied to affected parts with inflammation due to suntan or other disorders, where pain is induced by such application, or they can applied to mucus membrane parts such as of the nose, eye, and throat. The cosmetics and medicated cosmetics, containing MVIA of the present invention, exert an improved moisture retaining effect on the skin and the hair to inhibit the skin disorders and the hair damages induced by dryness and detergents and to improve the smoothness of the skin and hair after coating their surfaces. Also, the cosmetics and medicated cosmetics impart a smooth feeling and quality to the skin and hair, strengthen the skin barrier function, protect the skin surface and the surface of hair cuticles, and prevent them from static electricity, and therefore they can be also advantageously used as protective agents for the hair, hair root, and skin; agents for improving the smoothness of the skin and hair; gloss-imparting agents; conditioners; preventives for static electricity; and bases for hair colors. The saccharide-derivatives of α,α-trehalose or the saccharide compositions containing the same well harmonize with substances used in cosmetics and medicated cosmetics, for example, the later described substances with emulsifying action; solvents such as ethanol, 1,3-butylene glycol, propylene glycol, glycerine concentrate, dipropylene glycol, and 1,2-pentanediol; polyols such as sorbitol and maltitol; and synthetic high molecules such as polyethylene glycol usually having a molecular weight of 400 to 6,000 and carboxyvinylpolymer; and they do not become cloudy or form precipitates. Then they are feasible to make the following creams into those having homogeneous, emulsified particles with a more homogeneous, fine particle size compared with conventional creams for cosmetic, quasi-drug, or pharmaceutical use which contain only glycerin as an emulsifier and in which substances having an emulsifying action are incorporated. When used in cleansing foams, shampoos and rinses, the saccharide-derivatives of α,α-trehalose or the saccharide compositions containing the same impart a solidity and elasticity to their foams, increase the formation of foams, improve the retention of foams, and inhibit the physical stimulation to the skin by the foams. Then, with these external dermatological agents, one can comfortably wash his or her skin without giving stress to the skin, while clearly removing the stains in pores to the deep part with enriched foams and simultaneously imparting a user a fresh feeling on his or her skin and moisture to the skin. In addition, the saccharide-derivatives of α,α-trehalose of the present invention or the saccharide compositions containing the same have actions of inhibiting the oxidation and/or deterioration of lipids; stabilizing lipid membranes of liposomes and cells and inhibiting the oxidation thereof; inhibiting the oxidation and decomposition of ingredients susceptible to oxidation, browning, and color changing, which are used in cosmetics, medicated cosmetics, and pharmaceuticals, for example, substances capable of emulsifying ability such as amino acid surf actants, coloring materials such as pigments, flavors, ascorbic acid, ascorbic acid-2-glucoside, tannin liquid, honey, beeswax, propolis, and amino acids; and inhibiting the occurrence of agreeable smell. Thus, they can be advantageously used as materials free of imparting stickiness even when incorporated into cosmetics, medicated cosmetics, pharmaceuticals, and detergents for kitchen and clothes.

Based on the above, the saccharide-derivatives of α,α-trehalose can be advantageously used in a variety of compositions such as food products, cosmetics, medicated cosmetics, pharmaceuticals, feeds, pet foods, baits, agents for vitrification, daily goods, groceries, and chemical industrial products, as a sweetener, taste-improving agent, quality-improving agent, luster-imparting agent, gloss-imparting agent, shape-retaining agent, inhibiting agent for oxidation and decomposition of lipids, denaturation inhibiting agent, color change preventive, color fading preventive, freshness-retaining agent, agent for retaining taste and flavor, agent for inhibiting metmyoglobin formation, moisture-retaining agent or moisturizer, texture-improving agent, retrogradation-preventing agent, agent for protecting cells, cell activator, and growth-promoting agent for plants.

The following experiments explain MVIA of the present invention in detail:

### Experiment 1

### Influence of saccharides for candy on the glass transition temperature and the mold-releasing ability of candy

Candy is a representative example of vitrified foods obtainable by solidifying a saccharide(s) in a solution or melting form without crystallizing the saccharide(s). The vitrification phase generally changes depending on the temperature and the moisture content of saccharide compositions and turns into a phase called "rubber phase" from vitrification phase and becomes viscous as the increase of temperature or moisture content. It is called glass transition temperature at which the phase change is occurred. It is said that the glass transition temperature is an essential regulatory factor for keeping the quality of vitrified foods. Increment of the level of the glass transition temperature of foods results in improvement of their storage stability. As evident from the later described Experiments 2 to 4, compared with the one prepared without using MVIA of the present invention, the candy prepared with the agent is more inhibited in moisture variation, substantially free from moisture absorption, and capable of stably retaining its vitrification phase even when allowed to stand at ambient temperature. The present inventors speculated that the vitrification and the moisture variation inhibiting action according to the present invention were related each other and then conducted the following experiment to examine the influence of the saccharide-derivatives of α,α-trehalose on the glass transition temperature of saccharides for candy: Seven types of candies (candy Nos. 1 to 7), containing any of the saccharides as shown in the compositions 1 to 7 in Table 1, were prepared in a usual manner by using as saccharides sucrose or granulated sugar; "MALTRUP™", a starch syrup commercialized by Hayashibara Shoji, Co., Ltd., Okayama, Japan; and MVIA, prepared by the later described method in Example 1, a 72.8%, d.s.b., saccharide solution containing, as saccharide-derivatives of α,α-trehalose, 4.1%, d.s.b., α-glucosyl α,α-trehalose, 52.5%, d.s.b., α-maltosyl α,α-trehalose, 1.1%, d.s.b., α-maltotriosyl α,α-trehalose, and 0.4%, d.s.b., α-glycosyl α,α-trehalose. To prepare these candies, water was added to each saccharide into an about 70% syrup and boiled to concentrate after placed in a pan.

**Table 1**

| Candy No. (Composition) | Ratio of saccharides (dry solid basis) | | | | | Moisture content (%) |
|---|---|---|---|---|---|---|
| | Sucrose | Starchy syrup | Hydrous Crystallline Tre* | HMCS** | Syrupy MVIA*** | |
| 1 | 6 | 4 | 0 | 0 | 0 | 3.2 |
| 2 | 6 | 0 | 4 | 0 | 0 | 3.7 |
| 3 | 6 | 0 | 0 | 4 | 0 | 3.8 |
| 4 | 6 | 3 | 0 | 0 | 3 | 3.3 |
| 5 | 6 | 2 | 0 | 0 | 2 | 2.8 |
| 6 | 6 | 1 | 0 | 0 | 1 | 2.7 |
| 7 | 6 | 0 | 0 | 0 | 0 | 3.8 |

| | | | | | | |
|---|---|---|---|---|---|---|
| * Tre: α,α-Trehalose | | | | | | |
| ** HMCS: High maltotetraose content syrup | | | | | | |
| *** MVIA: Moisture variation inhibiting agent | | | | | | |

The seven types of candies for testing were respectively crushed and about 5 mg of any of the resultant mixtures was placed in an aluminum container for powdery sample equipped with "DSC 200C" , a thermal analyzer commercialized by Seiko Instruments Inc., Tokyo, Japan, followed by setting the container to the thermal analyzer and heating each sample at an increasing temperature rate of 10°C/min from 30°C to 150°C. Thereafter, the samples were promptly removed from the containers, placed on a glass plate for instantly cooling the contents to vitrify the samples as candies again. The containers with the resulting vitrified candies were respectively set to "DSC 6200R" , a thermal analyzer commercialized by Seiko Instruments Inc., Tokyo, Japan, cooled to -50° C, and measured for specific heat change at an increasing temperature rate of 10°C/min from -50°C to 150° C, followed by recording the endothermically shifted temperature, as a glass transition temperature, for each sample. The results are in Table 2.

**Table 2**

| Candy No. (Composition) | Glass-transition temperature (°C) |
|---|---|
| 1 | 13.2 |
| 2 | 37.6 |
| 3 | 24.6 |
| 4 | 12.9 |
| 5 | 25.0 |
| 6 | 36.2 |
| 7 | 38.3 |

As evident from the results in Table 2, the candy of composition 4, containing about 6%, d.s.b., of the saccharide-derivatives of α,α-trehalose and containing sucrose, starch syrup, and MVIA in a syrup form in a weight ratio of 6:3:1, d.s.b., gave a glass transition temperature of 12.9°C, substantially the same value as 13.2°C of the candy of composition 1 containing sucrose and starch syrup in a weight ratio of 6:4, d.s.b. While, the candies of compositions 5, 6 or 7, containing about 12, 18 and 24%, d.s.b., of the saccharide-derivatives of α,α-trehalose and having a ratio of at least two for MVIA in a syrup form, gave glass transition temperatures of 25.0° C, 36.2° C and 38.3° C, respectively, that were higher than that of the candy of composition 1 by 11.8° C, 23.0° C, and 25.1° C, respectively. The candy of composition 6, containing sucrose, starch syrup, and the syrupy MVIA in a ratio of 6:1:3, d.s.b.; the one of composition 7, containing sucrose and the syrupy MVIA in a ratio of 6:4, d.s.b.; and the one of composition 2, containing sucrose and α,α-trehalose in a ratio of 6:4, d.s.b., gave similar glass transition temperatures of 36.2°C, 38.3°C and 37.6°C, respectively. The candy of composition 7 with MVIA gave a glass transition temperature of 13.7° C higher than that of the one of composition 3 with the syrup enriched in maltotetraose having substantially the same glucose polymerization degree as that of MVIA. Based on these results, it was revealed that MVIA containing the saccharide-derivatives of α,α-trehalose has a high glass transition temperature that is substantially the same level as that of α,α-trehalose, one of the saccharides with the highest glass transition temperature among a variety of saccharides (cf. *"Shokuhin-to- Garasuka-Kesshoka-Gijutsu" (Foods and technology of vitrification and crystallization thereof),* pp. 3 to 60 (2000). It was revealed that, unlike starch syrup, the saccharide-derivatives of α,α-trehalose or saccharide compositions containing the same can increase the glass transition temperature of vitrified candy compositions to be admixed therewith, when added to the compositions, and that MVIA of the present invention can be advantageously used as a vitrifying agent for improving the storage stability of vitrified compositions including candies.

### Test on mold-releasing ability of candy

Among the seven-types of candies, those with the saccharides of compositions 1, 2, 3, 5 and 7 were experimented on feasibility of releasing from deposits in preparing the candies as follows: To these saccharides of compositions 1, 3, 5 and 7 was added water to make a syrup with a concentration of 70%, and the resulting syrups were respectively placed in a pan, concentrated up to give a temperature of 155° C, transferred to four deposits for each concentrate, and allowed to stand at ambient temperature for one hour. For the saccharide of composition 2, it was concentrated into a syrup similarly as in the above other saccharides, except for heating to concentrate the syrup up to give a temperature of 145°C to adjust the moisture level to the same level as the composition 7. Thereafter, the number of falling-down candies from the four deposits for each composition were counted by successively turning over the deposits, lifting up either side of each of the four deposits to a height one centimeter over the surface of a table and then releasing the lifted-up sides to allow the deposits to fall down on the surface of the table, lifting up either side of each of the four deposits to a height three centimeters over the surface of the table and then releasing the lifted-up sides to allow the deposits to fall down similarly as above, and lifting up either side of each of the four deposits to a height five centimeters over the surface of a table and then releasing the lifted-up sides to allow the deposits to fall down; followed by totalizing the counted numbers of candies. The results are in Table 3. In this experiment, a deposit with 12 rectangular holes, which were respectively positioned at an equal interval between a pair of holes on a plate having 34 mm in width and 440 mm in length, for housing candies, 24 mm in length and 18 mm in width each, made of a casting coated with polytetrafluorethylene.

**Table 3**

| Candy No. (Composition) | Handling of deposits and sum of candies detached from four deposits (pieces) | | | | Sum of candies remained to four deposits (pieces) |
|---|---|---|---|---|---|
| | Wipe out | Fall from the height of 1 cm | Fall from the height of 3 cm | Fall from the height of 5 cm | |
| 1 | 0 | 9 | 14 | 12 | 13 |
| 2 | 28 | 12 | 8 | 0 | 0 |
| 3 | 3 | 20 | 9 | 8 | 8 |
| 5 | 9 | 16 | 11 | 4 | 8 |
| 7 | 26 | 13 | 9 | 0 | 0 |

As evident from the results in Table 3, the candy of composition 1 incorporated with sucrose and starch syrup was inferior in mold-releasability, and no released candy was observed only when the deposits were turned over. The candy of composition 3 incorporated with sucrose and high maltotetraose content starch syrup showed a superior mold-releasability to the one of composition 1, however, 16 candies were stilled remained in the four deposits in number even when the deposits were fallen down from the height of three centimeters above the surface of the table. While the candies of compositions 5 and 7, which contained, on a dry solid basis, about 12% and about 24% of the saccharide-derivatives of α,α-trehalose, respectively, along with sucrose, were superior in mold-releasability to the candy of composition 1. Among which, the candy of composition 7 with sucrose and MVIA in a weight ratio of 6:4 gave a similar mold-releasability as of the candy of composition 2, i.e., all the candies were released from the four deposits when the deposits were fallen down from the height of three centimeters above the surface of the table. Based on these results, it was speculated that, compared with the case of using starch syrup, the use of, as a candy base, α,α-trehalose or MVIA containing the saccharide-derivatives of α,α-trehalose increased the glass transition temperature of candy, resulting in stabilizing the vitrification phase and reducing the adhesiveness to the internal surface of the deposits. Among the candies tested, those with α,α-trehalose or the saccharide-derivatives of α,α-trehalose had a lesser stickiness to the teeth and an improved anti-fragility.

### Experiment 2

### Influence of saccharides on the moisture-absorption inhibiting action in hard candy containing sucrose

A hard candy prepared with only sucrose loses its transparency when the saccharide is crystallized therein. In general, for the purpose of inhibiting such occurrence, hard candies incorporated with saccharides other than sucrose have been prepared, however, such incorporation may often impart a strong hygroscopicity to the candies. Accordingly, the following experiment was conducted for confirming the influence of saccharides on the moisture inhibiting action in hard candy prepared with sucrose. Sucrose and any one of the following saccharides were mixed in a weight ratio of 6:4, based on a dry solid, and admixed with water into an about 70% aqueous solution; a reagent grade anhydrous crystalline glucose with a purity of at least 99.5%, commercialized by Sigma Chemical company, St. , Louis, USA; a reagent grade crystalline maltose with a purity of at least 99.0%, commercialized by Hayashibara Biochemical Laboratories, Inc., Okayama, Japan; a reagent grade hydrous crystalline α,α-trehalose with a purity of at least 99.0%, commercialized by Hayashibara Biochemical Laboratories, Inc. , Okayama, Japan; a reagent grade isomaltose with a purity of at least 97.0%, commercialized by Hayashibara Biochemical Laboratories, Inc., Okayama, Japan; a reagent grade hydrous crystalline paratinose with a purity of at least 97.0%, commercialized by Hayashibara Biochemical Laboratories, Inc., Okayama, Japan; a reagent grade anhydrous crystalline maltitol with a purity of at least 99.0%, commercialized by Hayashibara Biochemical Laboratories, Inc., Okayama, Japan; a maltotriose with a purity of at least 97.0%, commercialized by Hayashibara Biochemical Laboratories, Inc., Okayama, Japan; a hydrous crystalline panose with a purity of at least 97.0%, commercialized by Hayashibara Biochemical Laboratories, Inc., Okayama, Japan; an erlose with a purity of at least 97.0%, commercialized by Hayashibara Biochemical Laboratories, Inc. , Okayama, Japan; a reagent grade hydrous crystalline raffinose with a purity of at least 99.0%, commercialized by Sigma Chemical company, St., Louis, USA; and a powdery α-glucosyl α,α-trehalose with a purity of at least 98.1%, prepared by the later described method in Example 5. The resulting aqueous solutions were respectively placed in a pan, heated to concentrate up to give a temperature of 150°C, and cooled to ambient temperature to obtain hard candies, having two centimeters in length, one centimeter in width, and a half centimeter in thickness each. After 3-day storage at 25°C under a relative humidity of 70%, these candies were macroscopically observed and evaluated by comparing the degree of their moisture absorption inhibiting actions, based on their change in appearance. The criterion for evaluating the change in appearance was as follows: The symbol (-) means that no moisture absorption inhibiting action was found after a candy changed into a syrupy product with no original shape; the symbol (+), a weak moisture absorption inhibiting action was found after initiation of loosening the original shape of a candy; and the symbol (++), a positive moisture adoption inhibiting action was found and had substantially no change in shape but with moisture absorption on the surface of a candy. The results are in Table 4.

**Table 4**

| Saccharides | After preserving for 3 days |
|---|---|
| Sucrose + Glucose | - |
| Sucrose + Maltose | - |
| Sucrose + α,α-Trehalose | + |
| Sucrose + Isomaltose | - |
| Sucrose + Palatinose | - |
| Sucrose + Maltitol | - |
| Sucrose + Maltotriose | - |
| Sucrose + Panose | - |
| Sucrose + Erlose | - |
| Sucrose + Raffinose | - |
| Sucrose + α-Glucosyl α,α-trehalose | ++ |
| Sucrose + α-Maltosyl α,α-trehalose | ++ |

As evident from the results in Table 4, the candy prepared with sucrose and α,α-trehalose or α-maltosyl α-trehalose retained its original shape even after 3-day storage. While those prepared with sucrose and a saccharide other than the α,α-trehalose or the α-maltosyl α,α-trehalose apparently changed into syrupy products and did not keep their original shapes, except that the one with sucrose and α,α-trehalose could only show a slight moisture absorption inhibiting action. These results confirmed that α-glycosyl α,α-trehalose and α-maltosyl α,α-trehalose well inhibited the absorption in candies compared with glucose, maltose, α,α-trehalose, isomaltose, paratinose, maltitol, maltotriose, panose, erlose, and raffinose.

### Experiment 3

### Influence of the ratio of MVIA, containing saccharide-derivatives of α,α-trehalose, on the moisture absorption inhibiting action in hard candy

An experiment for examining the influence of the ratio of the moisture absorption inhibiting agent, containing saccharide-derivatives of α,α-trehalose, on the moisture absorption inhibiting action in hard candy was conducted as follows by using hard candies, prepared similarly as in Experiment 1 in such a manner of mixing sucrose and another saccharide in a weight ratio of 6:4, d.s.b. Candies for testing were prepared by using saccharides in Table 5 in such a manner of mixing 180 parts by weight of sucrose and a prescribed amount (part by weight) of any one of MVIA in a syrup form, prepared by the later described method in Example 1, having, on a dry solid basis, a concentration of 72.8% and containing as saccharide-derivatives of α,α-trehalose 4.1% of α-glucosyl α,α-trehalose, 52.5% of α-maltosyl α,α-trehalose, 1.1% of α-maltotriosyl α,α-trehalose, and 0.4% of α-glycosyl α,α-trehalose; and "MALTRUP®", a high maltose syrup, having, on a dry solid basis, a concentration of 75% and containing 1.5% of glucose, 51.2% of maltose, maltotriose 21.7%, and 25.6% of other saccharides, commercialized by Hayashibara Shoji, Co., Ltd., Okayama, Japan; admixing with water to obtain aqueous saccharide mixture solutions; and heating to concentrate the resulting solutions up to give a temperature of 155°C. The candies thus obtained were stored at 25°C under a relative humidity of 70% for seven days and comparatively evaluated the moisture absorption inhibiting action of the testing saccharides, based on the change in appearance when observed macroscopically. The results are in Table 6. The criterion for macroscopic observation of the change in appearance was as follows: The symbol (-) means that no moisture absorption inhibiting action was found after a candy changed into a syrupy product with no original shape; the symbol (+), a weak moisture absorption inhibiting action was found after initiation of loosening the original shape of a candy; the symbol (++), a positive moisture absorption inhibiting action was found and had substantially no change in shape but with moisture absorption on the surface of a candy; and the symbol (+++), a positive moisture absorption inhibiting action was found and had no change in shape.

**Table 5**

| Candy No. (Composition) | Ratio of components (parts by weight) | | | | MVIA content (%-DS) | Content of saccharide-derivatives of Tre*** (%-DS) |
|---|---|---|---|---|---|---|
| | Sucrose | MVIA* | HMCS** | Water | | |
| 1 | 180 | 0 | 160 | 89 | 0 | 3.2 |
| 2 | 180 | 43 | 120 | 86 | 0 | 3.7 |
| 3 | 180 | 86 | 80 | 83 | 0 | 3.8 |
| 4 | 180 | 129 | 40 | 80 | 3 | 3.3 |
| 5 | 180 | 171 | 0 | 78 | 2 | 2.8 |

| | | | | | | |
|---|---|---|---|---|---|---|
| * MVIA: Moisture variation inhibiting agent, comprising saccharide-derivatives of α,α-trehalose | | | | | | |
| ** HMCS: High maltose content syrup | | | | | | |
| *** Tre: α,α-Trehalose DS: dry solid | | | | | | |

**Table 6**

| Candy No. (Composition ) | Time elapsed from the start of preservation (days) | | | | |
|---|---|---|---|---|---|
| | 1 | 2 | 3 | 6 | 7 |
| 1 | + | + | - | - | - |
| 2 | +++ | + | - | - | - |
| 3 | +++ | ++ | - | - | - |
| 4 | +++ | ++ | + | - | - |
| 5 | +++ | ++ | ++ | - | - |

As evident from the results in Table 6, the moisture absorption inhibiting agent, containing the saccharide-derivatives of α,α-trehalose as effective ingredients, strongly inhibited candies from absorbing moisture in proportion to the amount used. Although the ratios of the high maltose content syrup in the compositions 1 to 5 were different, the moisture absorption inhibiting effect was observed in the candies of compositions 2 to 5, containing at least 6%, d.s.b., of the saccharide-derivatives of α,α-trehalose, and the effect was remarkable for the candy of composition 3 having at least 12%, d.s.b., of the saccharide-derivatives of α,α-trehalose, and was more remarkable for the ones of compositions 4 and 5 having at least 18%, d.s.b., of the saccharide-derivatives of α,α-trehalose.

### Experiment 4

### Influence of the coexistence of the saccharide-derivatives of α,α-trehalose and α,α-trehalose or maltose on the moisture absorption inhibiting action in hard candy

An experiment for confirming the influence of the coexistence of the saccharide-derivatives of α,α-trehalose and α,α-trehalose or maltose on the moisture absorption inhibiting action in hard candy was conduced as follows: Aqueous saccharide mixture solutions were prepared similarly as in Experiment 1 by mixing sucrose and another saccharide in a weight ratio of 6:4, d.s.b., in such a manner of mixing 300 parts by weight of sucrose with 286 parts by weight of MVIA in a syrup form, prepared by the later described method in Example 1, having, on a dry solid basis, a concentration of 72.8% and containing, as saccharide-derivatives of α,α-trehalose, 4.1% of α-glucosyl α,α-trehalose, 52.5% of α-maltosyl α,α-trehalose, 1.1% of α-maltotriosyl α,α-trehalose, and 0.4% of α-glycosyl α,α-trehalose to obtain composition 1; or with 222 parts by weight of "TREHA®", a hydrous crystalline α,α-trehalose commercialized by Hayashibara Shoji Co., Ltd., Okayama, Japan to obtain composition 2; and the resulting compositions were respectively admixed with water. While to 300 parts by weight of sucrose was added either a mixture of 143 parts by weight of MVIA in a syrup form, prepared by the method in Example 1 and 105 parts by weight of "SUNMALT®" commercialized by Hayashibara Shoji Co., Ltd., Okayama, Japan; or a mixture of 111 parts by weight of the above hydrous crystalline α,α-trehalose and 105 parts by weight of the above maltose to obtain composition 3 or 4, respectively, followed by mixing the compositions with water into aqueous saccharide mixture solutions. All of these aqueous saccharide mixture solutions were heated to concentrate up to give a temperature of 145°C or 155°C into hard candies. The candies thus obtained were stored at 25°C under a relative humidity of 70% for three days and comparatively evaluated for moisture absorption inhibiting effect, based on the change in moisture level and appearance when observed macroscopically. The water content of the candies was measured in a usual manner by the method using diatomaceous earth. The criterion for evaluating the change in appearance was similarly conducted as in Experiment 2. The results are tabulated in Table 8.

**Table 7**

| Candy No. (Composition) | Ratio of components (part by weight) | | | | | Content of saccharide-derivatives of Tre (%-DS) |
|---|---|---|---|---|---|---|
| | Sucrose | MVIA* | Hydrous crystalline Tre** | Hydrous crystalline Mal*** | Water | |
| 1 | 300 | 286 | 0 | 0 | 129 | 24 |
| 2 | 300 | 0 | 222 | 0 | 193 | 0 |
| 3 | 300 | 143 | 0 | 105 | 168 | 12 |
| 4 | 300 | 0 | 111 | 105 | 199 | 0 |

| | | | | | | |
|---|---|---|---|---|---|---|
| * MVIA: Moisture variation inhibiting agent, comprising saccharide-derivatives of α,α-trehalose | | | | | | |
| ** Tre: α,α-trehalose | | | | | | |
| *** Mal: Maltose DS: dry solid | | | | | | |

**Table 8**

| Candy No. (Composition) | Temperature for boiling down | Initial Value | Value after 1 day | Value after 2 days | Value after 3 days |
|---|---|---|---|---|---|
| 1 | 145°C | 4.2% | 5.6% (+++)* | 6.2% (++) | 6.6% (++) |
| | 155°C | 3.8% | 4.8% (+++) | 5.2% (++) | 6.1% (++) |
| 2 | 145°C | 4.6% | 8.4% (+) | 10.0% (-) | 11.4% (-) |
| | 155°C | 4.1% | 7.4% (+) | 8.9% (-) | 10.1% (-) |
| 3 | 145°C | 5.2% | 8.3% (+) | 9.7% (-) | 11.1% (-) |
| | 155°C | 4.0% | 7.9% (+) | 9.5% (-) | 10.9% (-) |
| 4 | 145°C | 5.6% | 9.1% (-) | 10.8% (-) | 12.3% (-) |
| | 155°C | 4.9% | 8.9% (-) | 10.8% (-) | 12.5% (-) |

| | | | | | |
|---|---|---|---|---|---|
| * : Symbols in parentheses mean the evaluation by macroscopic examination | | | | | |

As evident from the results in Table 8, the candies containing the saccharide-derivatives of α,α-trehalose as effective ingredients had a lower water content and a lesser change in appearance and moisture absorption level than those with no such saccharide-derivatives, even though there was observed a relatively large difference between the ambient humidity level and the water content of the candies with the saccharide- derivatives. Thus, the moisture variation of the candies, containing the saccharide-derivatives of α,α-trehalose, was well inhibited. The candy of composition 1 prepared from MVIA, containing sucrose and the saccharide-derivatives of α,α-trehalose, as effective ingredients, i.e., the saccharide-derivatives of α,α-trehalose in an amount of 24%, d.s.b. , showed a stronger moisture absorption inhibiting effect than the candy of composition 2 prepared from sucrose and hydrous crystalline α,α-trehalose, independently of the heating temperature of 145°C or 155°C for concentration. The candy of composition 3, prepared from sucrose, maltose, and MVIA containing the saccharide-derivative of α,α-trehalose as effective ingredients in an amount of 12%, d.s.b., showed a stronger moisture absorption inhibiting effect than the candy of composition 4 prepared from sucrose, α,α-trehalose, and maltose. Since the candy of composition 3, however, only contained the moisture absorption inhibiting agent in a half amount of that of the candy of composition 1, the moisture absorption inhibiting effect of the candy of composition 3 was lower than that of the one of composition 1. Based on these, it was revealed that the moisture absorption inhibiting agent, containing the saccharide- derivatives of α,α-trehalose, more effectively inhibits the moisture variation in candies than α,α-trehalose and maltose. Differing the conditions of the presence or the absence of maltose, the candy with 24%, d.s.b., of the saccharide-derivatives of α,α-trehalosehad a higher moisture inhibiting effect than the one with 12%, d.s.b., of the saccharide- derivatives of α,α-trehalose. The data from Experiments 1 to 4 indicates that the action, which the saccharide-derivatives of α,α-trehalose increase the glass transition temperatures of compositions and then stabilize the vitrification condition of compositions and lower the motion of water molecules, correlates with the mechanism of the moisture variation inhibiting action by MVIA of the present invention.

### Experiment 5

### Influence of saccharide-derivatives of α,α-trehalose on the moisture releasing inhibiting action in jelly

An experiment for confirming the influence of saccharide-derivatives of α,α-trehalose on the moisture releasing inhibiting action in jelly was conducted as follows: 49.7 parts by weight of deionized water were admixed with 0.3 part by weight of agar and 50 parts by weight of MVIA, prepared by the later described method in Example 2, containing, on a dry solid basis, 4.1% of α-glucosyl α,α-trehalose, 52.5% of α-maltosyl α,α-trehalose, 1.1% of α-maltotriosyl α,α-trehalose, and 0.4% of α-glycosyl α,α-trehalose as saccharide-derivatives of α,α-trehalose; and the resultingmixture was heated to 100° C; incubated at the temperature for two minutes; poured into 60-ml jelly cups, having two centimeters in depth, to equally fill up; and cooled at 4°C for 16 hours to gel the contents to obtain a jelly. As a control, a jelly was prepared in a similar composition as in the above except for substituting sucrose for MVIA. The jellies thus obtained were scooped out with a cork bowler, with one centimeter in diameter, to obtain cylindrical specimens, having one centimeter in diameter and two centimeters in length. The specimens were instantly placed in a weighing container, allowed to stand therein at 25° C under a relative humidity of 35%, and measured for weight change of lowering weight due to moisture release by using an electrobalance from the time just after their processings through the end of 24-hour standing. The results are in Table 9 and expressed with relative values when the weight of each jelly just after processing was regarded as 100%.

**Table 9**

| Saccharide | Time elapsed after the production (hours) | | | | | | |
|---|---|---|---|---|---|---|---|
| | 0 | 0.5 | 1.0 | 2.0 | 4.0 | 5.0 | 24.0 |
| Sucrose | 100.0 | 97.4 | 94.9 | 91.4 | 86.7 | 85.3 | 74.9 |
| MVIA* | 100.0 | 97.4 | 95.3 | 92.5 | 89.1 | 87.9 | 79.7 |

| | | | | | | | |
|---|---|---|---|---|---|---|---|
| *: Moisture variation inhibiting agent comprising saccharide-derivatives of α,α-trehalose | | | | | | | |

As evident from the results in Table 9, every jelly was observed to release moisture in a time dependent manner. Comparing with the control jelly with sucrose, the degree of moisture release of the jelly, prepared with the saccharide-derivatives of α,α-trehalose as effective ingredients, was suppressed to a lower level, meaning that the saccharide-derivatives exerted a strong moisture release inhibiting effect. The fact revealed that the saccharide-derivatives of α,α-trehalose have a stronger moisture variation inhibiting effect than sucrose. The difference between the two was particularly remarkable from after four-hour standing under a prescribed relatively low moisture condition.

### Experiment 9

### Influence of saccharide-derivatives of α,α-trehalose on the moisture release inhibiting action in the surface of frozen dough for bread

There exists the problem that a frozen dough for bread releases and loses moisture from its surface during freeze-storage, and then the surface could not be baked homogeneously after thawing, resulting in giving an unfavorable patchy pattern, so-called, a pear-like surface. In this regard, an experiment for confirming the influence of the saccharide- derivatives of α,α-trehalose on the moisture releasing inhibition on the surface of a frozen-bread was conducted as follows: One thousand parts by weight of "LYS D'OR®", a flour for French bread commercialized by Nisshin Flour Milling Inc., Tokyo, Japan; 50 parts by weigh of a yeast for freezing commercialized by Sankyo Foods, Co., Ltd., Tokyo, Japan; 20 parts by weight of salt; one part by weight of "AMYLA A™" , a yeast food commercialized by Awajiya Inc. , Tokyo, Japan; and 650 parts by weight of water were kneaded, divided into aliquots by 50 parts by weight each, and shaped to give a roll form. To the surface of each of the shaped rolls was applied one part by weight of an aqueous saccharide solution containing 10%, 20% or 40%, d.s.b., of sucrose, hydrous crystalline α,α-trehalose, or MVIA in a powder form prepared in Example 2, containing, as saccharide-derivatives of α,α-trehalose, 4.1%, d.s.b., of α-glucosyl α,α-trehalose, 52.5%, d.s.b., of α-maltosyl α,α-trehalose, 1.1%, d.s.b. , of α-maltotriosyl α,α-trehalose, and 0.4%, d.s.b., of α-glycosyl α,α-trehalose. The resulting rolls were placed on a tray and instantly cooled to -40°C to obtain shaped frozen dough for French bread. As a control, a shaped-frozen dough for French bread was prepared by instantly cooling to -40°C a dough for bread with no application of any aqueous saccharide solution. These shaped frozen dough for French bread were kept at -20°C for 14 days, thawed at 20°C under a relative humidity of 75% over 90 min, fermented at 28°C under a relative humidity of 75% for 70 min, and baked at 190°C for 20 min under steaming conditions to obtain French bread. The degrees of *"a pear-like surface"* induced on the surface of the French bread for testing were compared with that of the control to evaluate the inhibiting effect on the occurrence of *"a pear-like surface"* based on a macroscopic observation. The criterion for evaluating the change in appearance was as follows: The symbol (-) means that no difference was found compared with the control; the symbol (+), an improvement in the occurrence of *"a pear-like surface"* was found but a slight improvement; and the symbol (++), an improvement was apparently found even though the occurrence of *"a pear-like surface"* was found; and the symbol (+++), a strong improvement was found and the occurrence of *"a pear-like surface"* was not substantially found. The results are in Table 10.

**Table 10**

| Saccharide | Concentration of saccharide (%) | | | |
|---|---|---|---|---|
| | 0 | 10 | 20 | 40 |
| Sucrose | - | + | + | + |
| MVIA* | - | ++ | ++ | +++ |
| Content of saccharide-derivatives of Tre** (%-DS) | 0 | 0.1 | 0.2 | 0.4 |
| Hydrous crystalline Tre | - | + | + ~++ | + |
| Content of Tre (%-DS) | 0 | 0.2 | 0.4 | 0.8 |

| | | | | |
|---|---|---|---|---|
| * MVIA: Moisture variation inhibiting agent comprising saccharide-derivatives of α,α-trehalose | | | | |
| ** Tre: α,α-trehalose DS: dry solid | | | | |

As evident from the results in Table 10, the frozen-dough for bread containing the saccharide-derivatives of α,α-trehalose as effective ingredients, to which the saccharide-derivatives of α,α-trehalose had been applied in an amount of 0.1%, d.s.b., exhibited a clear inhibiting effect on the occurrence of *"a pear-like surface" ,* and those applied with the saccharide-derivatives of α,α-trehalose in an amount 0.4%, d.s.b., didnot substantially induce *"a pear-like surface"* and showed a strong inhibiting effect. While in the case of using hydrous crystalline α,α-trehalose, there was found an improved effect when used in an amount of 0.4%, d.s.b., however, the level was lower than those prepared with MVIA containing the saccharide-derivatives of α,α-trehalose as effective ingredients, and those with hydrous crystalline α,α-trehalose in an amount of less than 0.4%, d.s.b., only showed a weak inhibiting effect as low as those prepared with sucrose. These data indicates that MVIA containing the saccharide-derivatives of α,α-trehalose have a distinctively stronger moisture variation inhibiting effect than sucrose and hydrous crystalline α,α-trehalose, and thus it well inhibits the moisture release from the surface of frozen dough for bread, strongly inhibits the occurrence of *"a pear-like surface"* even when such frozen dough are baked after thawing, and facilitates baking up the dough into a high quality bread. Since MVIA containing the saccharide-derivatives of α,α-trehalose used in this experiment is easily applied on the surface of dough for bread, it locally locates just on its applied part to give a final concentration of far exceeding 6%, d.s.b. , in the applied part, resulting in effectively exerting the moisture variation inhibiting effect on the surface tissue of dough for bread.

Based on the above results in experiments, it was revealed that incorporation of MVIA, containing the saccharide-derivatives of α,α-trehalose as effective ingredients, into compositions inhibits the moisture variation in the compositions and then advantageously inhibits their quality deterioration.

Following examples concretely explain compositions incorporated with MIVA of the present invention. However, the present invention is not restricted by them.

### Moisture variation inhibiting agent (MVIA)

MVIA can be used for inhibiting the moisture variation of various compositions, in addition, the denaturation of proteins, the retrogradation of gelatinized starch, and/or the oxidation of lipids in various compositions. Also, MIVA can be used for inhibiting the deterioration of the quality, syneresis, and drying during the preservation under a frozen-, refrigerated-, and chilled-conditions or by the treatment of drying and freeze-drying. Therefore, MVIA can be used for keeping relish such as taste, flavor, color and texture, and functions of the compositions by incorporating it into various compositions such as foods, beverages, cosmetics, medicated cosmetics, pharmaceuticals, feeds, pet-foods, commodities, sundries or chemicals.

### Example 1

A corn starch was prepared into an about 20% of starch suspension, admixed with calcium carbonate to give a final concentration of 0.1%, and adjusted to pH 6.5. The resulting solution was admixed with 0.2%/g-starch on a dry solid basis of "TERMAMYL 60L", a α-amylase commercialized by NovoZyme A/S, Bagsværd, Denmark, and followed by the enzyme reaction at 95° C for 15 minutes. After autoclaving at 120° C for 10 minutes, the resulting reaction mixture was cooled to 50° C, adjusted to pH 5.8, admixed with 5 units/g-starch of maltotetraose-forming amylase disclosed in Japanese Patent Publication No. 240, 784/88, commercialized by Hayashibara Biochemical Laboratories Inc., Okayama, Japan, and 500 units/g-starch of isoamylase commercialized by Hayashibara Biochemical Laboratories Inc., Okayama, Japan, and followed by the enzymatic reaction for 48 hours. The reaction mixture was further admixed with 30 units/g-starch of "α-AMYLASE 2A", α-amylase commercialized by Ueda Chemical Industries Co., Ltd., Hyogo, Japan, and followed by the enzyme reaction at 65° C for four hours. After autoclaving at 120° C for 10 minutes, the reaction mixture was cooled to 45° C, admixed with 2 units/g-starch of non-reducing saccharide-forming enzyme originated from *Arthrobacter* sp. Q36 (FREM BP-4316), disclosed in Japanese Patent Publication No. 143,876/95, and followed by the enzymatic reaction for 48 hours. The reaction mixture was kept at 95° C for 10 minute, cooled and filtered to obtain a filtrate. According to the conventional manner, the resulting filtrate was decolored with activated charcoal, desalted and purified with ion exchangers in H- and OH- forms, and concentrated into 72.8%-syrup in a yield of about 90% to the material starch on a dry solid basis. The product was slightly sweet and pH 5.3. It showed the DE of 13.7 and contained 52.5% of α-maltosyl α,α-trehalose (alias α-maltotriosyl α-glucoside), 4.1% of α-glucosyl α,α-trehalose (alias α-maltosyl α-glucoside), 1.1% of α-maltotriosyl α,α-trehalose (alias α-maltotetraosyl α-glucoside), and 0.4% of other α-glycosyl α,α-trehalose, on a dry solid basis, as saccharide-derivatives of α,α-trehalose, and further contained 0.4% of other α-glycosyl. α,α-trehalose, 2.1% of monosaccharides such as glucose, 8.9% of disaccharides such as maltose, 6.7% of trisaccharides other than α-glycosyl α,α-trehalose, 17.6% of tetrasaccharides other than α-maltosyl α,α-trehalose and 6.6% of pentasaccharides or larger other than α-glycosyl α,α-trehalose. The product is used as MVIA for compositions. It has a sweetness corresponding to about 30% of sucrose. It has an advantage to process because of hardly causing of coloration such as browning (Maillard reaction) by heating. Therefore, it can be used for foods, beverages, cosmetics, medicated cosmetics, feeds, or pet foods. In addition, it can be used as an agent for raising glass transition temperature of compositions, base for powderization, additive for preparations, agent for inhibiting sticking of starch containing foods, gross imparting agent, shape keeping agent, body imparting agent, agent for inhibiting the oxidation and/or degradation of lipid, denaturation-inhibiting agent, color-deterioration preventing agent, freshness-keeping agent, flavor-keeping agent, cell-protecting agent, cell-activating agent or plant-growth promoting agent.

### Property on absorbing and releasing moisture of MVIA

Property on absorbing and releasing moisture of MVIA was examined by the following experiment. Reagent grades of magnesium chloride hexahydrate, potassium carbonate dihydrate, magnesium nitrate hexahydrate, ammonium nitrate, sodium chloride, potassium chloride, barium chloride dihydrate, or potassium sulfate were dissolved in purified water to make into a saturated solution, respectively. These saturated solutions were placed a in sealed chamber and stand in an incubator controlled to 25° C to give a relative humidity of 33.0%, 42.7%, 52.8%, 60.0%, 75.2%, 87.2%, 90.1%, or 97.3% in the chamber. Weight of each container without cap containing about 0.5 g of the product was measured and the container was placed in each chamber without contacting the preset salt solution, and kept at 25°C. After keeping the samples in each chamber for 0.3, 1, 3, 5, 7, 10, and 14 days, weight of each container was measured to calculate the rate of change of weight (%) in a manner of comparing each value at the initial weight as 100%. The results are in Table 11.

**Table 11**

| Relative humidity (%) | Preservation time (days) | | | | | | | |
|---|---|---|---|---|---|---|---|---|
| | Initial | 0.3 | 1 | 3 | 5 | 7 | 10 | 14 |
| 33.0 | 0.0 | -7.1 | -10.0 | -13.0 | -14.3 | -15.1 | -15.8 | -16.6 |
| 42.7 | 0.0 | -6.2 | -9.7 | -13.0 | -14.3 | -15.1 | -15.9 | -16.6 |
| 52.8 | 0.0 | -6.5 | -9.8 | -13.0 | -13.0 | -14.2 | -14.9 | -15.6 |
| 60.0 | 0.0 | -6.7 | -10.1 | -13.3 | -14.5 | -15.2 | -15.9 | -16.4 |
| 75.2 | 0.0 | -3.4 | -6.6 | -9.4 | -10.0 | -10.4 | -10.6 | -10.6 |
| 84.2 | 0.0 | -1.6 | -2.5 | -3.2 | -3.4 | -3.4 | -3.3 | -3.2 |
| 90.1 | 0.0 | 0.3 | 1.7 | 4.3 | 5.8 | 6.5 | 6.7 | 6.4 |
| 97.3 | 0.0 | 3.3 | 9.2 | 21.0 | 27.9 | 32.5 | 37.4 | 41.1 |

As is evident from the results in Table 11, the product absorbed moisture under the condition of a relative humidity of 97.3%, and the weight of the product was increased about 41.1% after 14 days in comparison with the initial weight. While, although the product slightly adsorbed moisture under the condition of a relative humidity of 90.1%, the weight of the product did not increase at 3 days or later. The increased weight after 14 days was just about 6.4% in comparison with the initial weight. It was revealed that the product is a syrup hardly absorbing moisture under the condition of a relatively high humidity. The product released moisture under the condition of relative humidity of 84.2% or lower. Although the weight of the product was gradually decreased until 3 days, the product stably kept its moisture content after 3 days. The decreasing rate of weight depended on the humidity of circumstance and was decreased with lower humidity of circumstance. Concretely, the weight of the product, preserved under the condition of a relative humidity of 33% for 14 days, was decreased about 16.6% in comparison with the initial weight.

### Example 2

MVIA in a syrupy form, obtained by the method in Example 1, was spray-dried by the conventional method to convert into MVIA in an amorphous powdery form. The product shows a low hygroscopicity and a satisfactory solubility in water, and can be advantageously used as a MVIA for compositions. In addition, it can be preferably used as a base for powderizing juices or oils. Further, the product can be arbitrarily used as an agent for increasing glass transition temperature of compositions, additive for preparations, stickiness-inhibiting agent for foods comprising starch, gloss-imparting agent, shape-keeping agent, body-imparting agent, agent of inhibiting the oxidation and/or degradation of lipids, denaturation-inhibiting agent, color-deterioration preventing agent, freshness-keeping agent, flavor-keeping agent, cell-protecting agent, cell-activating agent or plant-growth promoting agent.

### Example 3

The saccharified solution, desalted and decolored with ion exchangers in H- and OG-forms in Example 1, was subjected to a column chromatography using "DOWEX 50W-X4 (Mg²⁺-form)", a strongly- acidic cation-exchanger resin commercialized by Dow Chemical Company, Michigan, USA. The resin was packed into four jacketed stainless steel columns having a diameter of 5.4 cm, which were then cascaded in series to give a total gel bed depth of 20 m. Under the conditions of keeping the inner column temperature at 55° C, the saccharide solution was fed to the columns in a volume of 5%(v/v) and fractionated by feeding to the columns hot water heated to 55°C at an SV (space velocity) of 0.13 to remove high glucose and maltose content fractions, and then collected high content fractions of saccharide-derivatives of α,α-trehalose. The resulting saccharide solution was further purified and concentrated, and then spray-dried to prepare an amorphous powder comprising saccharide-derivatives of α,α-trehalose in a high content. The product contained 70.2% of α-maltosyl α,α-trehalose, 6.1% of α-glucosyl α,α-trehalose, 2.1% of α-maltotriosyl α,α-trehalose, and 4.1% of other α-glycosyl α,α-trehalose, on a dry solid basis, as saccharide-derivatives of α,α-trehalose. Since the product shows a low hygroscopicity and a satisfactory solubility in water, it can be advantageously used as MVIA for compositions. In addition, it can be preferably used as a base for powderization.

### Example 4

One part by weight of a potato starch dissolved in six parts by weight of water was admixed with "NEOSPITASE", a α-amylase product commercialized by Nagase & Co., Ltd., Osaka, Japan, to give a final concentration of 0.01%/starch, and adjusted to pH 6.0. The resulting starch suspension was kept at 85 to 95°C to gelatinize and liquefy starch simultaneously and heated immediately at 120°C for five minutes to keep the DE lower than 1.0. Then, the solution was rapidly cooled to 55°C, adjusted to pH 7.0, admixed with 150 units/g-starch on a dry solid basis of "PULULLANASE", pullulanase (EC 3.2.1.41) commercialized by Hayashibara Biochemical Laboratories Inc., Okayama, Japan, and eight units/g-starch on a dry solid basis of maltotetraose-forming amylase, disclosed in Japanese Patent Publication No. 240, 784/88, commercialized by Hayashibara Biochemical Laboratories Inc., Okayama, Japan, and followed by the enzymatic reaction at 50°C and pH 7.0 for 36 hours. After autoclaving at 120°C for 10 minutes, the reaction mixture was cooled to 53°C, admixed with 2 units/g-starch of non-reducing saccharide-forming enzyme originated from *Arthrobacter* sp. S34 (FREM BP-6450), disclosed in Japanese Patent Publication No. 228,980/2000, and followed the enzymatic reaction for 64 hours. The reaction mixture was kept at 95° C for 10 minutes, cooled and filtered to obtain a filtrate. According to the conventional manner, the resulting filtrate was decolored with activated charcoal, desalted and purified with ion exchangers in H- and OH- forms, and then concentrated. The concentrate was spray-dried to obtain an amorphous powder containing saccharide-derivatives of α,α-trehalose in a yield of about 90% to the material starch, on a dry solid basis. The product showed the DE of 11.4 and contained 62.5% of α-maltosyl α,α-trehalose, 2.1% of α-glucosyl α,α-trehalose, 0.8% of α-maltotriosyl α,α-trehalose, and 0.5% of other α-glycosyl α,α-trehaloses, on a dry solid basis. Since the product shows a low hygroscopicity and a satisfactory solubility in water, it can be used as MVIA for compositions. In addition, it can be used as a base for powderization.

### Example 5

A reagent grade maltotetraose (purity 97.0% or higher), commercialized by Hayashibara Biochemical Laboratories Inc., Okayama, Japan, was prepared into 20% solution and admixed with two units/g-saccharide of non-reducing saccharide-forming enzyme, disclosed in Japanese Patent Publicatoin No. 143,876/95, and followed by the enzymatic reaction at 46°C for 48 hours to obtain a saccharide solution containing 79.8% of α-maltosyl α,α-trehalose on a dry solid basis. After adjusting the pH to 6.0, the saccharide solution was admixed with 10 units/g-saccharide of α-amylase, commercialized by Nagase & Co. , Ltd. , Osaka, Japan, and followed by the enzymatic reaction at 50° C for 48 hours for the purpose of hydrolyzing maltotetraose. After autoclaving at 120°C for 10 minutes, the reaction mixture was cooled and filtrated. The resulting filtrate was subjected to a column chromatography using "XT-1016 (Na⁺-form, degree of crosslinking 4%)", a strongly-acidic cation-exchanger resin commercialized by Rohm and Hass Japan K.K., Fukushima, Japan, to collect fractions highly containing α-maltosyl α,α-trehalose. The saccharide solution was purified, concentrated, and spray-dried to obtain an amorphous powder highly containing α-maltosyl α,α-trehalose as MVIA. The product, containing 98.1% of α-maltosyl α,α-trehalose, has a low reducing power less than the detection limit of Somogyi-Nelson method. The product shows a low hygroscopicity and a satisfactory solubility in water. Since the product has no reducibility, it can be advantageously used as MVIA for healthy foods, cosmetics, medicated cosmetics, pharmaceuticals, feeds, pet-foods, products of chemical industries, etc. , comprising effective ingredients such as compounds having amino groups and amino acids, which are inactivated by Maillard reaction.

The product was dissolved in water again, treated with activated charcoal to remove pyrogens, and spray-dried to make into an amorphous powder of high α-maltosyl α,α-trehalose content product. Since the product shows a low hygroscopicity and a satisfactory solubility in water, it can be advantageously used as MVIA. In addition, since it is free from pyrogens, it can be preferably used as MVIA for pharmaceuticals.

### Example 6

MVIA in a syrupy form, obtained by the method in Example 1, was dissolved in water to give a concentration of about 60% and admixed with about 8.5% of Raney nickel. The mixture was autoclaved at 128° C with stirring and hydrogenated under the condition of a hydrogen pressure of 80 kg/cm² to convert reducing sugars coexisting with saccharide-derivatives of α,α-trehalose, such as glucose and maltose, into corresponding sugar alcohols. After removing Raney nickel, the resulting solution was decolored, desalted, and concentrated to 75% to obtain MVIA in a syrupy form. The product, containing about 53% of α-maltosyl α,α-trehalose and about 5% of other saccharide-derivatives of α,α-trehalose on a dry solid basis, is a colorless, transparent and sticky liquid. The product can be advantageously used as MVIA for compositions. Since the product shows no reducibility, it can be advantageously used for healthy foods, cosmetics, medicated cosmetics, pharmaceuticals, feeds, pet-foods, products of chemical industries, etc.,comprisingeffective ingredients inactivated by Maillard reaction. In addition, it can be arbitrarily used as an agent for raising glass transition temperature of compositions, base for powderization, additive for preparation, agent for preventing sticking of foods comprising starch, gross-imparting agent, shape-keeping agent, body imparting agent, agent for inhibiting oxidation and/or degradation of lipid, denaturation-inhibiting agent, color-deterioration preventing agent, freshness-keeping agent, flavor-keeping agent, cell-protecting agent, cell-activating agent or plant-growth promoting agent.

### Example 7

MVIA in an amorphous powdery form, obtained by the method in Example 2, was dissolved in water to give a concentration of about 60% was admixed with about 9% of Raney nickel. The mixture was autoclaved at 130° C with stirring and hydrogenated under the condition of a hydrogen pressure of 75 kg/cm² to convert reducing sugars coexisting with saccharide-derivatives of α,α-trehalose, such as glucose and maltose, into corresponding sugar alcohols. After removing Raney nickel, the resulting solution was decolored, desalted, and concentrated to obtain MVIA in a syrupy form. Further, the syrup was spray-dried in a usual manner to obtain MVIA in an amorphous powdery form. The product, containing about 70% of α-maltosyl α,α-trehalose and about 12% of other saccharide-derivatives of α,α-trehalose on a dry solid basis, can be advantageously used as MVIA for compositions. The product in a powdery form shows a low hygroscopicity and a satisfactory solubility in water. Since the product shows no reducibility, it is suitable for cosmetics, medicated cosmetics, pharmaceuticals, healthy foods, etc., comprising effective ingredients inactivated by Maillard reaction. In addition, it can be used as an agent for raising glass transition temperature of compositions, base for powderization, additive for preparation, agent for preventing sticking of foods comprising starch, gross-imparting agent, shape-keeping agent, body imparting agent, agent for inhibiting oxidation and/or degradation of lipid, denaturation-inhibiting agent, color-deterioration preventing agent, freshness-keeping agent, flavor-keeping agent, cell-protecting agent, cell-activating agent or plant-growth promoting agent.

### Example 8

About 6% starch suspension of potato starch was gelatinized by heating, adjusted to pH4.5 at 50°C, admixed with 2, 500units /g-starch of isoamylase commercialized by Hayashibara Biochemical Laboratories, Inc., Okayama, Japan, and followed by the enzyme reaction for 20 hours. The resulting reaction mixture was adjusted to pH6.0 and autoclaved at 120°C for 10 min. After cooled to 45°C, the reaction mixture was admixed with 150units/g-starch of "TERMAMYL 60L", a α-amylase commercialized by NovoZyme A/S, Bagsværd, Denmark, and followed by the enzyme reaction for 24 hours. The reaction mixture was autoclaved at 120°C for 20min. After cooled to 45°C, the reaction mixture was admixed with 2 units/g-starch of non-reducing saccharide-forming enzyme originated from *Arthrobacter* sp. Q36 (FREM BP-4316), disclosed in Japanese Patent Publication No. 143,876/95, and followed by the enzymatic reaction for 64 hours. The reaction mixture was kept at 95°C for 10 minute, cooled and filtered to obtain a filtrate. According to the conventional manner, the resulting filtrate was decolored with activated charcoal, desalted and purified with ion exchangers in H- and OH- forms, and then concentrated into a 65%-syrup in a yield of about 89% to the material starch on a dry solid basis. The product contains 3.2% of α-glucosyl α,α-trehalose, 6.5% of α-maltosyl α,α-trehalose, 28.5% of α-maltotriosyl α,α-trehalose, and 11.9% of α-glycosyl α,α-trehalose having a glucose polymerization degree of 6 or higer, on a dry solid basis. The product can be used as MVIA for compositions. In addition, it can be used as an agent for raising glass transition temperature of compositions, base for powderization, additive for preparation, agent for preventing sticking of foods comprising starch, gross imparting agent, shape keeping agent, body imparting agent, agent for inhibiting oxidation and/or degradation of lipid, denaturation-inhibiting agent, color-deterioration preventing agent, freshness-keeping agent, flavor-keeping agent, cell-protecting agent, cell-activating agent or plant-growth promoting agent.

### Example 9

MVIA in a syrupy form, obtained by the method in Example 8, was hydrogenated according to the method in Example 6 to convert coexisting reducing saccharides such as glucose and maltose into corresponding sugar alcohol. The resultant was purified and concentrated in a usual manner to obtain MVIA in a syrupy form. The product, containing about 6% of α-maltosyl α,α-trehalose and about 44% of other saccharide-derivatives of α,α-trehalose, on a dry solid basis, is a colorless, transparent and sticky liquid. The product can be advantageously used as MVIA for compositions. Since the product shows no reducibility, it is suitable for cosmetics, medicated cosmetics, pharmaceuticals, healthy foods, etc. , comprising effective ingredients inactivated by Maillard reaction. In addition, it can be preferably used as a base for powderization, additive for preparation, agent for preventing sticking of foods comprising starch, gross-imparting agent, shape-keeping agent, body imparting agent, agent for inhibiting oxidation and/or degradation of lipids, denaturation-inhibiting agent, color-deterioration preventing agent, freshness-keeping agent, flavor-keeping agent, cell-protecting agent, cell-activating agent or plant-growth promoting agent.

### Example 10

Thirty-three percent of starch suspension was admixed with calcium carbonate to give a final concentration of 0.1%, and adjusted to pH 6.0. The resulting solution was admixed with 0.2%/g-starch on a dry solid basis of "TERMAMYL 60L" , a α-amylase commercialized by NovoZyme A/S, Bagsværd, Denmark, and followed by the enzymatic reaction at 95° C for 15 minutes. After autoclaving at 120° C for 10 minutes, the reaction mixture was cooled to 50° C, admixed with 500 units/g-starch of isoamylase, commercialized by Hayashibara Biochemical Laboratories Inc., Okayama, Japan and 1.8 units/g-starch of maltohexaose-maltoheptaose producing amylase, and followed by the enzymatic reaction for 40 hours. The reaction mixture was autoclaved at 120° C for 10 minutes, cooled to 53° C, adjusted to pH 5.7, and admixed with 2 units/g-starch of non-reducing saccharide-forming enzyme originated from *Arthrobacter* sp. S34 (FREM BP-6450), disclosed in Japanese Patent Publication No. 228,980/00, and followed the enzymatic reaction for 64 hours. The reaction mixture was kept at 95°C for 10 minutes, cooled and filtered to obtain a filtrate. According to the conventional manner, the resulting filtrate was decolored with activated charcoal, desalted and purified with ion exchangers in H- and OH- forms, concentrated, and spray-dried to obtain an amorphous powder as a MVIA in a yield of about 87% to the material starch on a dry solid basis. The product contains 8.2% of α-glucosyl α,α-trehalose, 6.5% of α-maltosyl α, α-trehalose, 5.6% of α-maltotriosyl α,α-trehalose, and 21.9% of α-maltotetraosyl α,α-trehalose, 9.3% of α-maltopentaosyl α,α-trehalose, and 14.1% of α-glycosyl α,α-trehalose having a glucose polymerization degree of 8 or higher. Since the product shows a low hygroscopicity and a satisfactory solubility in water, it can be advantageously used as MVIA. Optionally, it can be further purified according to a usual manner for the purpose of increasing the content of saccharide-derivatives of α,α-trehalose. In addition, it can be used as an agent for increasing glass transition temperature of a composition, base for powderization, additive for preparation, agent for preventing sticking of foods comprising starch, gross-imparting agent, shape-keeping agent, body-imparting agent, agent for inhibiting oxidation and/or degradation of lipids, denaturation-inhibiting agent, color-deterioration preventing agent, freshness-keeping agent, flavor-keeping agent, cell-protecting agent, cell-activating agent or plant-growth promoting agent.

### Example 11

MVIA in a powdery form, obtained by the method in Example 10, was hydrogenated according to the method described in Example 7 to convert reducing saccharides such as glucose and maltose into corresponding sugar alcohols. The resulting solution was purified and spray-dried in a usual manner to obtain an amorphous powder as a MVIA. The product contains about 6% of α-maltosyl α,α-trehalose and about 59% of other saccharide-derivatives of α,α-trehalose, on a dry solid basis. Since the product shows a low hygroscopicity and a satisfactory solubility in water, it can be advantageously used as MVIA for compositions. Optionally, it can be further purified in a usual manner for the purpose of increasing the content of saccharide-derivatives of α,α-trehalose. Since the product shows no reducibility, it is suitable for cosmetics, medicated cosmetics, pharmaceuticals, healthy foods, etc., comprising effective ingredients inactivated by Maillard reaction. In addition, it can be arbitrarily used as an agent for increasing glass transition temperature of a composition, base for powderization, additive for preparation, agent for preventing sticking of foods comprising starch, gross-imparting agent, shape-keeping agent, body-imparting agent, agent for inhibiting oxidation and/or degradation of lipids, denaturation-inhibiting agent, color-deterioration preventing agent, freshness-keeping agent, flavor-keeping agent, cell-protecting agent, cell-activating agent or plant-growth promoting agent.

### Example 12

Sixty parts by weight of MVIA in an amorphous powdery form, prepared in Example 2, was admixed with 40 parts by weight of "MABIT®" , an anhydrous crystalline maltitol commercialized by Hayashibara Shoji Inc., Okayama, Japan, to obtain a powdery mixture. The product can be advantageously used as MVIA for compositions.

### Example 13

Seventy parts by weight of MVIA in an amorphous powdery form, obtained by the method in Example 2, was admixed with two parts by weight of ascorbic acid 2-glucoside commercialized by Hayashibara Biochemical Laboratories, Inc. , Okayama, Japan, and two parts by weight of "αG-RUTIN", an enzyme-treated rutin commercialized by Hayashibara Biochemical Laboratories, Inc., Okayama, Japan, to obtain a powdery mixture. The product can be advantageously used as MVIA for compositions.

### Example 14

One part by weight of MVIA in a syrupy form, prepared in Example 1, 0.8 part by weight of arabic gum as a water-soluble polysaccharide and 0.05 part by weight of water-soluble hemicellulose were mixed together, dissolved in appropriate amount of water, and spray-dried in usual manner to obtain a powdery mixture. The product can be advantageously used as MVIA for compositions. The product can be advantageously used for preventing the conjugation of food products comprising starch such as pastes, boiled noodles, retort pouched noodles, instant noodles and pilafs in a manner of added to the food materials at an appropriate timing during process of food products and/or applied on food products (ex. soaking in the solution of it) at any timing during process of re-cooking.

### Example 15

One part by weight of MVIA in an amorphous powdery form, prepared in Example 2, was admixed with 1.5 parts by weight of isomerized sugar to obtain a powdery mixture. The product can be advantageously used as MVIA for compositions. Since the product has an improved aftertaste of fructose even in an acidic condition of relatively low pH, it exhibits a similar taste to sucrose. Therefore, it can be advantageously used as a sweetener or seasoning.

### Example 16

One part by weight of MVIA in an amorphous powdery form, obtained by the method in Example 4, was admixed with nine parts by weight of sucrose to obtain a powdery mixture. The product can be advantageously used as MVIA for compositions. Since the product has an improved aftertaste of sucrose, it can be used as a sweetener or seasoning.

### Example 17

Sixty parts by weight of MVIA in an amorphous powdery form, obtained by the method in Example 7, was admixed with 50 parts of "TREHA®" , α,α-trehalose commercialized by Hayashibara Shoji, Inc., Okayama, Japan, to obtain a powdery mixture. The product can be advantageously used as MVIA for compositions.

### Compositions incorporated with MVIA

### Example 18

### Table Sugar

Fifty parts by weight of MVIA in a powdery form, prepared in Example 2, 46 parts by weight of anhydrous crystalline maltitol, three parts by weight of "αG-HESPERIDIN", a glycosyl hesperidin product commercialized by Toyo Sugar Refining Co, Ltd., Tokyo, Japan, one part by weight of sucralose commercialized by San-Ei Gen F.F.I. Inc., Osaka, Japan, were dissolved in 200 parts by weight of water, and spray-dried in a usual manner to obtain a powdery sweetener. Since the product shows a low hydroscopicity due to MVIA, it is a powdery sweetener free from casing with a satisfactory fluidity. It can be preferably used as a sweetener for various foods, beverages, medicated cosmetics or pharmaceuticals, including table sugar for coffee or black tea because an aftertaste of sucralose is improved by saccharide-derivatives of α,α-trehalose, which are effective ingredient in MVIA, and glycosyl hesperidin.

### Example 19

### Seasoned extract of dried bonito flake

Dried bonito flake was prepared in a usual manner of boiling a fresh bonito except for using a solution contains 18% of MVIA in a powdery form, prepared in Example 2. The product was not extremely dried and inhibited in the oxidization or degradation of lipids even after preserved for six months. Since MVIA contained in the product inhibits the denaturation of protein and fishy smell due to the formation of aldehydes or lipid peroxides by oxidizing or degrading lipids, the production stably keeps a good relish such as taste, flavor, color and texture derived from dried bonito flake for a long period.

The product preserved at a room temperature for six months was chipped by a machine. One hundred parts of the resultant was admixed with 500 parts by weight of water, boiled for five minutes, and cooled to obtain an extract of dried bonito flake. The extract had satisfactory taste and flavor nearly equal to that obtained from freshly prepared dried bonito.

Subsequently, nine parts by weight of the extract concentrated to ten-folds was admixed with one part by weight MVIA in a syrupy form, obtained by the method in Example 1, dissolved by stirring and spray-dried in a usual manner to obtain a powdery soup stock. The product is a powdery soup stock having satisfactory taste and flavor of dried bonito flake. Since the product is so stable as to be free from casing and keep fluidity equal to that of a freshly prepared product, it can be used alone or in combination with other extracts for producing a soup stock or seasoning in the form of powder, liquid, solid or paste.

### Example 20

### Processed sea urchin product

MVIA in a syrupy form, obtained by the method in Example 1, was diluted 10-folds with water and then sodium lactate was dissolved in the solution to give a content of 0.1% to make into a solution for soaking. A fresh ovary of sea urchin, placed in a basket, was soaked in the solution, kept at 5°C for 10 hours, and dehydrated by taking up the basket to obtain a product. The moisture variation of the product and the oxidation and decomposition of lipids in the product were inhibited. When the product was tasted after preserved in a cooled-, chilled- or frozen-condition for six months, it was not denaturated, drips was not generated after defrosting, and a granule form of sea urchin was not broken due to preservation in any above condition. When the product was cooked in a usual manner, it had a satisfactory taste, flavor, color and texture because the oxidation and degradation of lipids and the denaturation of proteins were inhibited.

### Example 21

### Processed sea urchin product

One part by weight of MVIA in a syrupy form, obtained by the method in Example 1, and 0.5 part by weight of "TREHA®" , α,α-trehalose commercialized by Hayashibara Shoji, Inc., Okayama, Japan, were dissolved in 8.5 parts by weight of water. Calcium carbonate was added to the resulting solution to give a concentration of 0.2% and heated to 80°C . According to Japanese Patent No. 20, 217 / 90, the processed sea urchin product, prepared in Example 20, was soaked with a stainless steel basket in the solution for five seconds and taken up to obtain a product. When the product was tasted after preserving in a cooled-, chilled- or frozen- condition for two months, it was not denaturated, drips were not generated after defrosting, and a granule form of sea urchin was not broken due to preservation in any above condition. When the product was cooked in a usual manner, it had a satisfactory taste, flavor, color and texture because the oxidation and degradation of lipids and the denaturation of proteins were inhibited.

### Example 22

### Lean Tuna

Four parts by weight of MVIA in a powdery form, obtained by the method in Example 2, 0.8 part by weight of sodium chloride and 0.01 part by weight of "LEUCOCYANIDIN" , an grape seed extract commercialized by Indiana Inc., Italy, were admixed with water to give 100 parts by weight of total amount, dissolved with stirring and cooled to 5° C. Lean tuna cut in short and narrow pieces was soaked in the resulting solution, kept at 5°C for 16 hours, taken up and frozen down to -30°C quickly to obtain a product. The moisture variation of the product and the oxidation or degradation of lipids in the product are effectively inhibited. Also, the denaturation of product by preserving in a frozen condition, and the formation of drips from the product by thawing, are inhibited. In addition, since the met hem pigment formation is inhibited, the product keeps vivid color of lean tuna and freshness. Therefore the product is preferable as a material for various food products. After preserving in a frozen condition for two months, the product was thawed, made into *"sashimi"* (sliced raw fish), and tasted. Since the oxidation and decomposition of lipids and the denaturation of proteins were inhibited, the product showed vivid color of lean tuna and low unpleasant taste and smell. It has a satisfactory taste, flavor, color and texture, equivalent to those of fresh lean tuna.

### Example 23

### Dried blowfish

One hundred parts by weight of raw blowfish fillet rolled to about 8mm in thickness was soaked in a solution containing 10% of MVIA in a syrupy form, obtained by the method in Example 1, for 30 minutes, and dried overnight to obtain a product. The product is a dried blowfish retaining freshness because the moisture variation of the product and the oxidation and degradation of lipids in the product are inhibited. The product has a satisfactory taste, flavor, color and texture with no smell such as volatile aldehydes, trimethylamine or ethyl mercaptan even when broiled.

### Example 24

### Dried blowfish

One hundred parts by weight of raw blowfish fillet rolled to about 8mm in thickness was soaked in a solution containing 7% of MVIA in a syrupy form, obtained by the method in Example 1, and 3%, on a dry solid basis, of "TREHA®", α,α-trehalose commercialized by Hayashibara Shoji, Inc., Okayama, Japan, for 30 minutes, and dried overnight to obtain a product. The product is a dried blowfish retaining freshness because the moisture variation of the product and the oxidation and degradation of lipids in the product are inhibited. The product has a satisfactory taste, flavor, color and texture with no smell such as volatile aldehydes, trimethylamine or ethyl mercaptan even when broiled.

### Example 25

### Boiled and dried anchovy

Two parts by weight of MVIA in a powdery form, obtained by the method in Example 2, was dissolved in 100 parts by weight of boiled water. Then, 10 parts by weight of raw anchovy, placed in a basket, was soaked in the resulting solution and boiled. The boiled anchovy was taken up with the basket, and dried in a usual manner to obtain a product. Since the moisture variation of the product and the oxidation and degradation of lipids in the product are effectively inhibited, it has a satisfactory color and flavor and can be used for preparing soup stock.

The product keeps its gloss of surface, blue color, and good flavor even after preserving at an ambient temperature for six months and can be used for preparing soup stock.

### Example 26

### Dried anchovy

Two parts byweight of MVIA in a powdery form, prepared in Example, one part by weight of "TREHA®", α,α-trehalose commercialized by Hayashibara Shoji Inc. , Okayama, Japan, and 0.2 part by weight of sodium lactate were dissolved in 100 parts by weight of boiled water. Then, 10 parts by weight of small raw anchovy was soaked in the resulting solution, boiled, and dried in a usual manner to obtain a dried anchovy. Since the moisture variation of the product and the oxidation and degradation of lipids in the product are effectively inhibited, it has a satisfactory color and flavor without unpleasant or fishy smell. In addition, it can be obtained in a high yield. Also, since the drying and hygroscopicity of the product are inhibited, the product keeps a satisfactory flavor for a long period even after preserving at an ambient temperature or under a cooled-, chilled- or frozen- condition.

### Example 27

### Shelled clam

Four parts by weight of MVIA in a syrupy form, obtained by the method in Example 1, was dissolved in 100 parts by weight of boiled water. Then, 10 parts by weight of raw clam, placed in a basket, was soaked in the resulting solution and boiled. The boiled clam was taken up with the basket and dried in a usual manner to obtain a boiled shelled clam. Since the moisture variation of the product and the oxidation and degradation of lipids in the product are inhibited, it has a satisfactory color, gloss and flavor. In addition, it can be obtained in a high yield. The product can be advantageously used for *"tukudani"* (clam boiled in sweetened soy sauce), seafood curry, or *"gomoku-gohan"* (boiled rice mixed with various vegetables or fish). Also, since the oxidation and decomposition of lipids and the denaturation of proteins in the product are inhibited, the product keeps a satisfactory flavor for a long period even after preserving at an ambient temperature or under a cooled-, chilled- or frozen-condition.

### Example 28

### Boiled octopus

Ten parts by weight of raw octpus kneaded with salt in a usual manner was soaked in 100 parts by weight of water containing three parts by weight of MVIA in a powdery form, obtained by the method in Example 2 , to obtain a boiled octopus. Since the moisture variation of the product and the oxidation or degradation of lipids in the product are inhibited, it has a satisfactory color, gloss and flavor. In addition, it can be obtained in a high yield. The product can be used as a material for *"sushi" ,* a vinegared food and "o*den"* (Japanese hotchpotch) after cutting into an appropriately sized piece. Also, since the oxidation and the degradation of lipids and the denaturation of proteins in the product are inhibited, the product keeps a satisfactory flavor for a long period even after preserving under a cooled-, chilled- or frozen-condition.

### Example 29

### Pickled herring

Fillets of fresh herring were seasoned with salt by the conventional method by soaking into a saline solution. After seasoning with salt at an ambient temperature for one hour, the fillets were further soaked into a seasoning solution, prepared by dissolving five parts by weight of MVIA in a syrupy form, prepared by the method in Example 1, and one part by weight of a seaweed broth into 100 parts by weight of vinegar, at an ambient temperature for five hours to make into a pickled herring. Since the moisture variation of the product is inhibited, the deterioration of the quality of meat is also inhibited even after preserving for two months. Also, since the oxidation and decomposition of lipids in the product are inhibited, the pickled herring has a satisfactory color, gloss, and relish. The product can be advantageously used as a material of *"sushi" ,* and prepared foods such as a vinegared food, by cutting it into fillets with a suitable size.

### Example 30

### Amberjack boiled with soy and sugar

One hundred parts by weight of fillet of fresh amberjack was placed in a pan, and then 10 parts by weight of MVIA in a powdery form, prepared by the method in Example 2, 10 parts by weight of soy sauce, five parts by weight of *"mirin"* (sweet cooking rice wine), and 10 parts by weight of water were further added and successively boiled according to the conventional method to make into amberjack boiled with soy and sugar. The product has a satisfactory color, gloss, and relish without change of shape. The moisture variation of the product is inhibited even after placing on a dish and the oxidation and decomposition of lipids in the product are also inhibited.

### Example 31

### Minced and steamed fish meat

To 2,000 parts by weight of fresh meat of Alaska pollock, pre-washed with water, 105 parts by weight of MVIA in a powdery form, obtained by the method in Example 7, and three parts by weight of sodium lactate were added and the resulting mixture was minced. The resulting minced fish meat was frozen at -20°C. After freezing at -20°C for 90 days, the frozen minced fish meat was thawed. Separately, 40 parts by weight of sodium glutamate, 100 parts by weight of potato starch, three parts by weight of sodium polyphosphate, 50 parts by weight of sodium chloride, and five parts by weight of sorbitol were dissolved in 150 parts by weight of ice-cold water. One hundred parts by weight of the resulting solution was admixed with the above minced fish meat. The mixture was further minced and divided into about 120 grams each, and then each divided minced fish meat was shaped on a board. The resulting minced fishmeat was steamed for 30 minutes to give an internal temperature of about 80°C. Successively, the product was cooled in an ambient temperature and stand at 4°C for 24 hours to make into a minced and steamed fish meat. Since saccharide-derivatives of α,α-trehalose have a moisture variation inhibiting activity and stabilize proteins in the product against freezing, frozen and minced fish meat of Alaska pollock keeps its freshness even after preserving in a frozen condition. The product prepared from the minced fish meat has a satisfactory relish, texture, and gloss. Since MVIA inhibits the oxidation and decomposition of lipids in the product, the modification and denaturation of proteins and amino acids by peroxides such as aldehydes, which are formed by decomposition of lipids, are inhibited. Therefore, the met hem pigment formation of the product is also inhibited and the product has a satisfactory stability during the preservation. The product shows no syneresis even after the steps of preserving under a frozen condition and thawing. The product keeps preferable relish such as taste, flavor, color, texture, etc. just after preparation.

### Example 32

### Minced and steamed fish meat

To 2,000 parts by weight of fresh meat of Alaska pollock, pre-washed with water, 105 parts by weight of MVIA in a syrupy form, obtained by the method in Example 8, 35 parts by weight of "TREHA®", α,α-trehalose commercialized by Hayashibara Shoji Inc. , Okayama, Japan, and two parts by weight of sodium lactate, and one part by weight of sodium carbonate were added and the resulting mixture was minced. The resulting minced fish meat was frozen at -20° C. After freezing at -20° C for 90 days, the frozen minced fish meat was thawed. Separately, 40 parts by weight of sodium glutamate, 100 parts by weight of potato starch, three parts by weight of sodium polyphosphate, 50 parts by weight of sodium chloride, and five parts by weight of sorbitol were dissolved in 150 parts by weight of ice water. One hundred parts by weight of the resulting solution was admixed with the above minced fish meat. The mixture was further minced and divided into about 120 grams each, and then each dividedminced fish meat was shaped on aboard. The resulting minced fish meat was steamed for 30 minutes to give an internal temperature of about 80°C. Successively, the product was cooled in an ambient temperature and stand at 4°C for 24 hours to make into a minced and steamed fish meat. Since saccharide-derivatives of α,α-trehalose have a moisture variation inhibiting activity and stabilize proteins in the product against freezing, frozen and minced fish meat of Alaska pollock keeps its freshness even after preserving in a frozen condition. The product prepared from the minced fish meat has a satisfactory relish, texture, and gloss. Since MVIA inhibits the oxidation and decomposition of lipids in the product, the modification and denaturation of proteins and amino acids by peroxides such as aldehydes, which are formed by decomposition of lipids, are inhibited. Therefore, the met hem pigment formation of the product is also inhibited and the product has a satisfactory stability during the preservation. The product shows no syneresis even after the steps of preserving under a frozen condition and thawing. The product keeps preferable relish such as taste, flavor, color, texture, etc. just after preparation.

### Example 33

### Seasoned baked laver

Five hundred parts by weight of MVIA in a powdery form, obtained by the method in Example 2, 400 parts by weight of soy sauce, 100 parts by weight of *"mirin"* (sweet cooking rice wine), 30 parts by weight of sodium chloride, and 10 parts by weight of sodium glutamate were admixed with 200 part by weight and dissolved by heating to 80°C with stirring, and then cooled to make into a solution for seasoning. One part by weight of the solution was applied to one side of baked laver produced from the conventional laver in a sheet form and the resultant was dried. The moisture variation of the product is inhibited by coating with a thin film of MVIA. The product is a baked laver with a good flavor, having a no hygroscopicity, stickiness and change of shape.

After preserving the product in a sealed container for one year, the product was kept in a humidity of 60% at 25°C for 60 minutes to observe the change of shape and then tasted. Although the conventional seasoned baked laver adsorbs the moisture and its seasoned side curved, the product does not show such phenomenon. The seasoned side of the product shows no stickiness. The product has a good flavor and dried condition just after preparation.

### Example 34

### Soy sauce powder

One point five parts by weight of MVIA in a syrupy form, prepared by the method of Example 1, was dissolved into three parts by weight of soy-sauce, and spray-dried by the conventional method to make into a powdery soy sauce. Since the moisture variation of the product is inhibited even after preserving for a long period, the product shows no hygroscopicity and retains taste and flavor of soy sauce and can be advantageously used as a seasoning for instant noodle, instant soup, etc.

### Example 35

### Milk powder

To 100 parts by weight of fresh milk, 1.5 parts by weight of MVIA in a syrupy form, prepared by the method of Example 1, was dissolved and then heated to about 50° C, and concentrated under a reduced pressure to give a milk dry solid of 30%. The concentrate was spray-dried by the conventional method to produce a milk powder. Since the moisture variation of the product is inhibited even after preserving for a long period, the product shows no hygroscopicity and no color deterioration, and retains preferable taste and flavor of milk. The product can be advantageously used as a material for various foods and beverages, and as a milk powder for coffee.

### Example 61

### Powdery vegetable juice

Sliced kale, broccoli, parsley, celery, and carrot were mixed and the mixture was blanched at 95° C for 20 minutes. After adding MVIA in a powdery form, prepared by the method of Example 2, and L-ascorbic acid to the blanched vegetables to give contents of 3% and 0.2%, respectively, the mixture was homogenized to make into a vegetable juice. After concentrating the vegetable juice to give a volume of 1/5, one part by weight of MVIA in a powdery form, prepared by the method of Example 3, was further admixed with four parts by weight of the concentrated vegetable juice. After adjusting the pH to 4.2 by adding citric acid, the mixture was spray-dried according to the conventional method to make into a powdery vegetable juice. The product showed no color deterioration and browning, and no hygroscopicity, and retains a satisfactory powdery form even after preserving in a sealed container at an ambient temperature for 90 days.

### Example 37

### Tablet containing vegetable juice

Suitable amounts of powdery vitamin B₁ and B₂ were admixed with the powdery vegetable juice, prepared by Example 36, and the resulting mixture was made into tablets using a tablet machine to produce tablets containing vegetable juice. The moisture variation of the product is inhibited and the product shows no color deterioration and no hygroscopicity, and is a tablet easy to take.

### Example 38

### Powdery green tea

MVIA in a powdery form, obtained by the method of Example 2, and L-ascorbic acid were admixed with green tea extracted from green tea leaf according to the conventional method to give the contents of 0.5% and 0.2%, respectively, to make into green tea. After concentrating the green tea to give a volume of 1/20, the concentrate was spray-dried by the conventional method to make into a powdery green tea. The product showed no color deterioration and browning, and no hygroscopicity, and retains a satisfactory fluidity and powdery form even after preserving in a sealed container at an ambient temperature for 120 days. The product can be advantageously used as a material for various foods and beverages.

### Example 39

### Powdery fat

Twenty-five parts by weight of olive oil and 75 parts by weight of MVIA in a powder form, prepared by the method of Example 2, were mixed using a mixer and the mixture was rolled with a pressing granulator to make into a plate. The resulting plate was pulverized by a conventional method to make into a powdery olive oil. The moisture variation of the product is inhibited and the product shows no hygroscopicity and retains the flavor of extra virgin oil well. Further, since the oxidation and decomposition of lipids in the product are inhibited, the product keeps the flavor for a long period.

### Example 40

### Powdery DHA (docosahexaenoic acid)

Forty parts by weight of sucrose fatty acid ester as an emulsifier, 40 parts by weight of MVIA in a powdery form, obtained by the method of Example 4, were dissolved in 120 parts by weight of water by stirring. Then, 20 parts by weight of DHA was admixed with the mixture and emulsified DHA by the conventional method to produce an emulsified preparation. The resulting preparation was spray-dried by the conventional method to make into a powdery DHA. Since the moisture variation of the product and the oxidation and decomposition of DHA are inhibited, the product shows no hygroscopicity and retains DHA stably for a long period. DHA-enriched foods and beverages can be produced by incorporating 0.001 to 1.0 part by weight of the product or the above emulsified preparation into one part by weight of conventional foods and beverages. Since the unpleasant taste and smell of DHA and emulsifier are inhibited, the product is easy to take.

### Example 41

### Powdery peppermint oil

Seventy parts by weight of arabic gum, 20 parts by weight of MVIA in a powdery form, obtained by the method of Example 14, and three parts by weight of α,α-trehalose were admixed with 150 grams of water and dissolved. The resulting mixture was heated to 85°C and kept for 15 minutes for sterilizing. After cooling the solution to about 40° C, 10 grams of peppermint oil was admixed with the solution and the mixture was emulsified using a homogenizer. The resulting emulsion was spray-dried by conventional method to make into powdery peppermint oil. Since the moisture variation of the product is inhibited, the product shows no hygroscopicity. Also, since saccharide-derivatives of α,α-trehalose inhibits the oxidation and decomposition of peppermint oil, the formation of bad smell by the deterioration is inhibited. Further, since saccharide-derivatives of α,α-trehalose masks the bad smell even when the smell is formed, the peppermint flavor of the product retains stably for a long period. The powdery peppermint oil can be advantageously used as a flavor for foods and beverages, cosmetics, medicated cosmetics and pharmaceuticals.

### Example 42

### Powdery ginseng extract

One part by weight of a ginseng extract was concentrated to give a volume of 1/5. Four parts by weight of MVIA in a syrupy form, obtained by the method in Example 1 and two parts by weight of "TREHA®" , hydrous crystalline α,α-trehalose commercialized by Hayashibara Shoji Inc., Okayama, Japan, were admixed with 10 parts by weight of the above concentrated ginseng extract and dissolved by stirring. The resulting mixture was dried *in vacuo* by the conventional method to make into a powdery ginseng extract. Since the moisture variation of the product is inhibited, the product shows no hygroscopicity and can be preserved for a long period.

### Example 43

### Polished rice

MVIA in a syrupy form, prepared by the method in Example 1, was diluted with water to give an aqueous solution with a concentration of 25%. Four parts byweight of the resulting solution was sprayed equally on 100 parts by weight of unpolished rice (old rice) with stirring. After standing the resulting unpolished rice for overnight, the unpolished rice was polished by the conventional method using a polishing machine to make into polished rice. Since the product comprises about 0.2 % of α-maltosyl α,α-trehalose, the moisture variation of the product is inhibited well. Also, since saccharide-derivatives of α,α-trehalose inhibits the oxidation and decomposition of lipids in the product, the product is polished rice having a high quality and satisfactory preserving stability. The product can be advantageously used as a material for cooked rice, rice ball, rice gruel, etc. with a good flavor. The product can be used intact as a pre-washed rice and after processing into gelatinized rice. Rice bran, obtained by the process of the above polishing, also comprises saccharide-derivatives of α,α-trehalose, which are effective ingredients of MVIA in the present invention. Therefore, the oxidation and decomposition of lipids in the rice bran is inhibited and the rice bran has a satisfactory preserving stability. The rice bran can be advantageously used for producing rice bran pickles and as a material of rice bran oil. Further, the rice bran can be advantageously used intact or in the defatted form as a material of mixed feeds.

### Example 44

### Pre-washed rice

One part by weight of MVIA in a powdery form, obtained by the method in Example 7, was admixed with 100 parts by weight of unpolished rice just after husked and preserved in a storage for six months. Successively, the unpolished rice was polished with a rice polishing machine to make into polished rice. The resulting polished rice was placed on a wire mesh belt conveyor and washed by spraying water in a high pressure for a very short time with mixing and transferring. Then, one part by weight of an aqueous solution, comprising 20% of MVIA obtained by the method in Example 7, and 1% of calcium lactate, was further sprayed on the above washed rice. The resulting rice was dried, measured, and packed to make into pre-washed rice. Since the product comprises about 0.3% of saccharide-derivatives of α,α-trehalose, the moisture variation of the product is inhibited well. Therefore, the product is a pre-washed rice with a high quality and satisfactory preserving stability. The product can be used as a material for preparing cooked rice, rice ball, rice gruel, *"sushi",* and gelatinized rice. Further, since the product is enriched with calcium, it can be preferably used for keeping or promoting health.

### Example 45

### Cooked rice

To 370 parts by weight of water, 20 parts by weight of MVIA in a syrupy form, prepared by the method in Example 1, was dissolved. Then, 300 parts by weight of polished rice, which are pre-washed and drained, was soaked into the above solution and boiled using a household rice cooker to obtain cooked rice. The moisture variation of the product and the denaturation of lipids and proteins in the product are inhibited by MVIA. Also, the retrogradation of gelatinized starch in the product is prevented by MVIA. Therefore, the cooked rice keeps preferable relish of just after boiling for a relatively long period. Since saccharide-derivatives of α,α-trehalose, which are effective ingredients of MVIA in the present invention, also inhibits the denaturation by refrigeration and freezing, the product keeps its viscosity and relish of just after preparation for a long period. Therefore, the product can be advantageously used as a cooked rice, which is distributed under a cooled-, chilled-, refrigerated-, or frozen condition. Also, the product can be advantageously used as a material or intermediate for producing various processed foods such as pilaf, cooked rice seasoned with vinegar, rice ball, rice with "*azuki*"-beans, rice gruel, and frozen-, refrigerated-, and chilled products thereof.

### Example 46

### Cooked rice

To 370 parts by weight of water, 15 parts by weight of MVIA in a syrupy form, prepared by the method in Example 1, five parts by weight of "TREHA®", α,α-trehalose commercialized by Hayashibara Shoji Inc. , Okayama, Japan, and one part by weight of vegetable protein was dissolved. Then, 300 parts by weight of polished rice, which are pre-washed and drained, was soaked into the above solution and boiled using a household rice cooker to obtain cooked rice. The moisture variation of the product and the denaturation of lipids and proteins in the product are inhibited by MVIA. Also, the retrogradation of gelatinized starch in the product is prevented and adequate viscosity is provided to the product by MVIA. Therefore, the cooked rice keeps preferable relish of just after boiling for a relatively long period. Since α,α-trehalose and saccharide-derivatives of α,α-trehalose, which are effective ingredients of MVIA in the present invention, also inhibits the denaturation by refrigeration and freezing, the product keeps its viscosity and relish of just after preparation for a long period. Therefore, the product can be advantageously used as a cooked rice, which is distributed under a cooled-, chilled-, refrigerated-, or frozen condition. Also, the product can be advantageously used as a material or intermediate for producing various processed foods such as pilaf, cooked rice seasoned with vinegar, rice ball, rice with "*azuki*"-beans, rice gruel, and frozen-, refrigerated-, and chilled products thereof.

### Example 47

### Rice grilled with salad oil

Six parts by weight of salad oil was poured into a frying pan and heated. Then, 300 parts by weight of cooked rice, prepared in Example 45, was added to the frying pan and grilled. To the cooked rice, apre-mixed powder, prepared by mixing three parts by weight of MVIA in a powdery form, obtained in Example 12, two parts by weight of salt, 0.3 part by weight of pepper, 0.4 part by weight of synthetic seasoning, 0.5 part by weight of dried green onion, and 0.5 part by weight of sucrose, was added and further grilled with salad oil for six minutes. Since the moisture variation of the product is inhibited, the product is rice grilled with salad oil with a satisfactory stability for freezing and refrigerating. The grilled rice shows no adherability even after the steps of freezing at -20°C for three months, heating, and cooled. The product keeps relish including texture, taste, flavor, etc., of just after preparation.

### Example 48

### Noodle

Ninety-nine parts by weight of all-purpose flour, three parts by weight of MVIA in a powdery form, obtained in Example 12, and five parts by weight of salt were admixed with 40 parts by weight of water and the resulting mixture was kneaded by the conventional method to make into dough for noodles. Then, the dough was cut into noodles with a thickness of 2.7 mm using a #11 cutter. The resulting noodles were boiled for 12 minutes and then cooled. The boiled noodles were soaked into an aqueous solution containing 0.5% of MVIA prepared by the method in Example 2 and 0.2% of pullulan and make into Japanese wheat noodles by removing the solution.

After preserving the boiled noodles at 6°C for one day, the noodles were dipped in a sauce and tasted. The Japanese wheat noodles showed no retrogradation of gelatinized starch and had a good taste and raveling property. The noodles retained viscosity and elasticity just after preparation.

### Example 49

### Instant noodle

To 30 parts by weight of 0.5% brine, 98.5 parts by weight of strong wheat flour and 1.5 parts by weight of MVIA in a powdery form, prepared by the method in Example 4, were mixed and the resulting mixture was kneaded by the conventional method to make into noodles with a thickness of 0.9 mm. After steaming for one minute and fifteen seconds in a steamer, the noodles were fried at 145°C for one minute and 20 seconds in a salad oil to make into instant noodles

After preserving the product in a sealed container at an ambient temperature for one year, the product was reconstituted in boiled water for three minutes and then tasted. The reconstituted noodles showed a good taste and raveling property, and retained viscosity and elasticity just after preparation.

### Example 50

### "Mochi" (rice cake)

Six hundred fifty parts by weight of water was admixed with 1,000 parts by weight of glutinous rice powder, and the mixture was steamed for 40 minutes. The steamed mixture was kneaded in a mixer and the resulting dough was cooled to 50°C. Then, a mixture prepared by mixing 180 parts by weight of sucrose, four parts byweight of "MOCHI-SOFT" , a quality-improving agent for *"mochi",* comprising α-amylase, commercialized by Sankyo Foods Co., Ltd., Tokyo, Japan, and 300 parts by weight of MVIA in a powdery form, prepared in Example 2, was gradually admixed with several times with the above dough and further kneaded for four minutes. The dough was divided into pieces, and the resulting small dough was shaped and cooled to make into *"mochi".*

Since the moisture variation of the product is inhibited, the retrogradation of starch in the product is prevented. The *"mochi"* has a delicious taste and keeps softness and extending property, which are inherent properties of *"mochi",* for a long period.

### Example 51

### "Ohagi" (kind of sweet glutinous rice cake)

Eight parts by weight of "SUNMALT®", maltose product commercialized by Hayashibara Shoji Inc. , Okayama, Japan, and two parts by weight of MVIA in a syrupy form, obtained by the method of Example 1 were dissolved in hot water to make into a saccharide solution with a concentration of 65%, on a dry solid basis. After steaming glutinous rice with a steamer according to the conventional method and cooling to 80° C, the steamed glutinous rice was soaked into the above saccharide solution and kept at temperature in the range of 70-80° C for about one hour in a keep-warm container. Then, *"ohagi"* was prepared using the steamed glutinous rice together with *"koshi-an"* (strained bean jam) prepared by the method of Example 56 mentioned below. The variation of moisture content of the product is inhibitedwell and the retrogradation of gelatinized starch of the product is prevented. The product shows no syneresis even after refrigerating or thawing after freezing and keeps the palatability and the softness just after the preparation.

### Example 52

### "Ohagi" (kind of sweet glutinous rice cake)

Seven parts by weight of "SUNMALT®", maltose product commercialized by Hayashibara Shoji Inc., Okayama, Japan, two parts by weight of MVIA in a syrupy form, obtained by the method of Example 1, and one part by weight of "TREHA®", α,α-trehalose commercialized by Hayashibara Shoji Inc, Okayama, Japan were dissolved in hot water to make into a saccharide solution with a concentration of 70%, on a dry solid basis. After steaming 1, 000 parts by weight of glutinous rice which is presoaked into water with a steamer according to the conventional method and cooling to 80°C, the steamed glutinous rice was admixed with 500 parts by weight of a solution which is prepared by dissolving three parts by weight of a commercial α-amylase product for food into 500 parts by weight of the above saccharide solution. The resulting mixture was stirred to homogeneity and kept at temperature in the range of 70-80°C for about one hour in a keep-warm container. Then, *"ohagi"* was prepared using the steamed glutinous rice together with *"koshi-an"* (strained bean jam) prepared by the method of Example 56 mentioned below. The variation of moisture content of the product is inhibited well and the retrogradation of gelatinized starch of the product is prevented. The product shows no syneresis even after refrigerating or thawing after freezing and keeps the palatability and the softness just after the preparation.

### Example 53

### "Mizu-manju" (kind of bean jam bun)

Twenty parts by weight of *"kudzu"* powder, 80 parts by weight of "INA-GEL-TSUYUKUSA" *("mizu-manju-no-moto"),* 50 parts by weight of sucrose, and 50 parts by weight of "SUNMALT®", maltose product commercialized by Hayashibara Shoji Inc., Okayama, Japan, were mixed by stirring and then the resulting mixture was gradually dissolved in 250 parts by weight of water with stirring. Further, 200 parts by weight of a powdery MVIA, obtained by the method of Example 2 was admixed with the above solution. The resulting mixture was boiled down to give a concentration of 57% over an open fire for about 25 minutes and then cooled. The cooled dough was shaped to make into *"Mizu-manju".* The variation of moisture content and the formation of crack of the dough of the product are inhibited well. The product keeps translucent color even after refrigerating or thawing after freezing and keeps the palatability and the relish such as taste, flavor, color and texture just after the preparation.

### Example 54

### "Koshi-an" (strained bean jam)

One hundred parts by weight of raw *"koshi-an"* prepared from broad bean by the conventional method, 60 parts by weight of sucrose, and 14.5 parts by weight of MVIA in a syrupy form, prepared by the method of Example 1 were admixed with 70 parts by weight of water and the resulting mixture was stirred gently with heating to boil down to give a Brix of 56 to make into a *"koshi-an".* Since the moisture variation of the product is inhibited in comparison with "*an*" (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of *"azuki"* bean and sucrose. The product shows a pleasant aftertaste and flavor and no browning. The product can be preferably used as a *"an"* material for buns, *"manju"* (Japanese bean jam cake), *" dango"* (Japanese rice flour cake), *"monaka"* (Japanese bean jam cake), *"hyoka"* (ice milk), etc. Particularly, in the case of using the product for buns, *"manju", "dango", "monaka",* etc., since the moisture variation of "an" is inhibited, the softening of these dough and *"monaka-dane"* is inhibited.

### Example 55

### "Tsubu-an" (kind of bean jam)

One hundred parts by weight of raw *"tsubu-an"* prepared from *"azuki"* bean by the conventional method, 50 parts by weight of sucrose, 29 parts by weight of MVIA in a syrupy form, prepared by the method of Example 6, a small amount of salad oil for preventing mush by boiling, and a small amount of sodium chloride as hidden taste were admixed with 50 parts by weight of water and the resulting mixture was stirred gently with heating to boil down to give a Brix of 56 to make into a *"tsubu-an".* Since the moisture variation of the product is inhibited in comparison with *"an"* (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of *"azuki"* bean and sucrose. The product shows a pleasant aftertaste and flavor and no browning. The product can be preferably used as a "an" material for buns, *"manju"* (Japanese bean jam cake), *"dango"* (Japanese rice flour cake), *"monaka"* (Japanese bean jam cake), *"hyoka"* (ice milk), etc. Particularly, in the case of using the product for buns, *"manju", "dango", "monaka",* etc., since the moisture variation of "an" is inhibited, the softening of these dough and *"monaka-dane"* is inhibited.

### Example 56

### "Koshi-an" (strained bean jam)

One hundred parts by weight of raw *"tsubu-an"* prepared from *"azuki"* bean by the conventional method, 50 parts by weight of sucrose, 10 parts by weight of "TREHA®", α,α-trehalose commercialized by Hayashibara Shoji Inc, Okayama, Japan, 14.5 parts by weight of MVIA in a syrupy form, prepared by the method of Example 6, 0.015 part by weight of magnesium chloride, and a small amount of sodium chloride as hidden taste were admixed with 60 parts by weight of water and the resulting mixture was stirred gently with heating to boil down to give a Brix of 48 to make into a *"koshi-an".* Since the moisture variation of the product is inhibited in comparison with *"an"* (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of *"azuki"* bean and sucrose. The product shows a pleasant aftertaste and flavor and no browning. The product can be preferably used as a "an" material for buns, *"manju"* (Japanese bean jam cake), *"dango"* (Japanese rice flour cake), *"monaka"* (Japanese bean jam cake), *"hyoka"* (ice milk), etc. Particularly, in the case of using the product for buns, *"manju", "dango", "monaka",* etc., since the moisture variation of "an" is inhibited, the softening of these dough and *"monaka-dane"* is inhibited. In the case of dredging the product on the surface of *"ohagi"* (kind of sweet glutinous rice cake), the moisture variation to *"an"* and the sticky property are inhibited. Chinese "*an*" can be arbitrarily prepared by admixing a suitable amount of sesame oil with the product during the preparation.

### Example 57

### "Koshi-an" (strained bean jam)

One hundred parts by weight of raw *"koshi-an"* prepared from pea by the conventional method, 50 parts by weight of sucrose, 29 parts by weight of MVIA in a syrupy form, prepared by the method of Example 1, and 0.01 part by weight of magnesium chloride were admixed with 50 parts by weight of water and the resulting mixture was stirred gently with heating to boil down to give a Brix of 60 to make into a *"koshi-an" .* Since the moisture variation of the product is inhibited in comparison with "*an*" (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of *"azuki"* bean and sucrose. The product shows a pleasant aftertaste and flavor and no browning. The product can be preferably used as a *"an"* material for buns, *"manju"* (Japanese bean jam cake), *" dango"* (Japanese rice flour cake), *"monaka"* (Japanese bean jam cake), *"hyoka"* (ice milk), etc. Particularly, in the case of using the product for buns, *"manju", "dango", "monaka",* etc., since the moisture variation of "an" is inhibited, the softening of these dough and *"monaka-dane"* is inhibited.

### Example 58

### "Koshi-an" (strained bean jam)

One hundred parts by weight of raw *"koshi-an"* prepared from *"shiro-azuki"* bean by the conventional method, 50 parts by weight of sucrose, 10 parts by weight of "TREHA®", α,α-trehalose commercialized by Hayashibara Shoji Inc, Okayama, Japan, 14.5 parts by weight of MVIA in a syrupy form, prepared by the method of Example 1, and one part by weight of sodium lactate were admixed with 70 parts by weight of water and the resulting mixture was stirred gently with heating to boil down to give a Brix of 56 to make into a *"koshi-an" .* Since the moisture variation of the product is inhibited in comparison with "*an*" (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of *"azuki"* bean and sucrose. The product shows a pleasant aftertaste and flavor and no browning. The product can be preferably used as a *"an"* material for buns, *"manju"* (Japanese bean jam cake), *"dango"* (Japanese rice flour cake), *"monaka"* (Japanese bean jam cake), *"hyoka"* (ice milk), etc. Particularly, in the case of using the product for buns, *"manju" , "dango" , "monaka",* etc., since the moisture variation of "an" is inhibited, the softening of these dough and *"monaka-dane"* is inhibited.

### Example 59

### "Tsubu-an" (kind of bean jam)

One hundred parts by weight of raw *"tsubu-an"* prepared from kidney bean by the conventional method, 50 parts by weight of sucrose, 29 parts by weight of MVIA in a syrupy form, prepared by the method of Example 6, and one part by weight of phytic acid were admixed with 50 parts by weight of water and the resulting mixture was stirred gently with heating to boil down to give a Brix of 56 to make into a *" tsubu-an" .* Since the moisture variation of the product is inhibited in comparison with "*an*" (bean jam) prepared with conventional enzyme-saccharified syrup or hydrogenated syrup, the product shows no crystallization of sucrose and low viscosity and exhibits no sticky texture. Also, the product shows a satisfactory dissolving ability and has a good flavor of kidney bean and sucrose. The product shows a pleasant aftertaste and flavor and no browning. The product can be preferably used as a "*an*" material for buns, *"manju"* (Japanese bean jam cake), *"dango"* (Japanese rice flour cake), *"monaka"* (Japanese bean jam cake), *"hyoka"* (ice milk), etc. Particularly, in the case of using the product for buns, *"manju", "dango", "monaka",* etc., since the moisture variation of *"an"* is inhibited, the softening of these dough and *"monaka-dane"* is inhibited.

### Example 60

### "Monaka" (Japanese bean jam cake)

To 200 parts by weight of water in a handy pan, two parts by weight of agar powder and 200 parts by weight of sucrose were added and dissolved by heating to make into an agar solution. Separately, to 360 parts by weight of water in a handy pan, 600 parts by weight of sucrose was added and dissolved by heating. Then, to the resulting sucrose solution, 1,000 parts by weight of raw "*an*" (bean jam) was added and the resulting mixture was kneaded. After hardening the mixture, 402 parts by weight of the above agar solution and 140 parts by weight of MVIA in a syrupy form, prepared by the method of Example 1 was admixed with the mixture, kneaded and boiled down to make into *"an"* with a Brix of 65. After cooling the *"an"* with covering with a kitchen cloth, a half of *"monaka-dane"* (shell of *"monaka")* was filled with the *"an"* and then another half of *"monaka-dane"* was fitted to sandwich the *"an"* to make into *"monaka".* Since the moisture variation is inhibited, *"an"* in the product is not dried by losing the moisture and the product keeps preferable flavor of just after preparation without softening *"monaka-dane".*

### Example 61

### Castella

Two hundred ninety parts by weight of whole egg, 190 parts by weight of sucrose, and 45 parts by weight of "SUNMALT®" , a maltose product commercialized by Hayashibara Shoji Inc., Okayama, Japan, were mixed in an electric mixer and whipped with a high speed and then 20 parts by weight of pre-warmed honey and 40 parts by weight of MVIA in a syrupy form, prepared by the method of Example 1 were admixed with the whipped cream and stirred lightly. To the resulting mixture, 120 parts by weight of sifted flour was added, fed in a flame, and baked in an oven for 60 minutes with removing bubble by the conventional method to make into castella. Since the moisture variation is inhibited, the product keeps the flavor just after baking for a long period and the frequency of removing bubbles during baking can be reduced. Further, the castella has a fine texture and gloss of surface without browning.

### Example 62

### Castella

Two hundred ninety parts by weight of whole egg, 160 parts by weight of sucrose, 45 parts by weight of "SUNMALT®", a maltose product commercialized by Hayashibara Shoji Inc. , Okayama, Japan, and 30 parts by weight of "TREHA®", α,α-trehalose commercialized by Hayashibara Shoji Inc. , Okayama, Japan, were mixed in an electric mixer and whipped with a high speed and then 20 parts by weight of pre-warmed honey and 40 parts by weight of MVIA in a syrupy form, prepared by the method of Example 1 were admixed with the whipped cream and stirred lightly. To the resulting mixture, 120 parts by weight of sifted flour was added, fed in a flame, and baked in an oven for 60 minutes with removing bubble by the conventional method to make into castella. Since the moisture variation is inhibited, the product keeps the flavor just after baking for a long period and the frequency of removing bubbles during baking can be reduced. Further, the castella has a fine texture and gloss of surface without browning.

### Example 63

### Dough for breads for frozen use

According to the method described in Experiment 6, 100 parts by weight of flour for French bread, five parts by weight of yeast, two parts by weight of sodium chloride, and 0.1 part by weight of yeast food were admixed with 65 parts by weight of water and kneaded. Then, the resulting dough was segmented to small groups and shaped in a roll. To 99 parts by weight of dough, one part by weight of a solution, prepared by dissolving MVIA in a powdery form, which is prepared by the method of Example 2, to give a concentration of 40% was applied. The resulting dough was put on a tray and frozen at -40° C to make into shaped frozen dough for French bread. After preserving the dough at -20°C for one month, the dough was thawed under the humidity of 75% for 90 minutes. After fermenting the resulting dough at 28°C under the humidity of 75% for 70 minutes, the dough was baked at 190° C for 20 minutes in the presence of steam to make into French bread. Since the moisture variation of the dough is inhibited under the frozen condition, drying and deterioration of yeast and dough are inhibited and gluten and texture are stabilized even after baking. The bread obtained after baking has a smooth surface, and adequate gloss, and shows no unattractive surface. Also, the bread has a satisfactory bulk and natural color, and shows no excessively dried surface. The bread has a satisfactory taste, flavor, and texture as well as breads baked using dough without freezing.

### Example 64

### Rice flour bread

Four hundred parts by weight of *"KOMENO-KO* (for bread)", a rice flour comprising gluten commercialized by Saito Seifun Inc., Niigata, Japan, eight parts by weight of sodium chloride, 25 parts by weight of MVIA in a powdery form, obtained by the method of Example 2, 20 parts by weight of "SUNMALT®" , a maltose product commercialized by Hayashibara Shoji Inc., Okayama, Japan, 12 parts by weight of sucrose, 12 parts by weight of a skim milk, 10 parts by weight of raw yeast, eight parts by weight of pullulan, and 315 parts by weight of water were mixed by stirring with a vertical-type mixer and then 20 parts by weight of butter was admixed with the mixture and further kneaded to make into dough. After fermented the dough at 25°C for 50 minutes, the dough was divided into a suitable size and further fermented at 25°C under the humidity of 75% for 20 minutes. After the fermentation, the dough was baked for 40 minutes in an oven which the upper and lower temperatures were controlled to 180°C and 180°C, respectively, to prepare rice flour bread. The rice flour bread has a adequate gloss, flavor which is specific to rice flour, glutinous texture, and a good taste. Although the rice flour bread contains a relatively larger amount of moisture than wheat flour bread, the moisture variation of the bread is inhibited and the hardening by drying and the deterioration of surface and internal texture. Therefore, the bread keeps soft texture for a long period. Further, since saccharide-derivatives of α,α-trehalose, which are effective ingredients of MVIA of the present invention, inhibit the generation of specific bad smell of yeast and have an activity of keeping savory flavor generated by baking, the bread keeps a preferable flavor just after baking for a long period.

### Example 65

### "Takoyaki" (Octopus balls)

Two hundred forty parts by weight of flour for *"takoyaki",* 30 parts by weight of MVIA in a syrupy form, obtained by the method of Example 1, five parts byweight of "TREHA®", α,α-trehalose commercialized by Hayashibara Shoji Inc., Okayama, Japan, and two parts by weight of sodium chloride were mixed and then 600 parts by weight of a soup stock, 100 parts by weight of whole egg, 40 parts by weight of olive oil, 14 parts by weight of 1% xanthan gum solution, 20 parts by weight of *"agedama"* (fried batter), and 10 parts by weight of *"beni-shoga"* (sliced, colored, and seasoned ginger) were admixed with the mixture to make into dough for *" takoyaki"*. The resulting dough was put in a plate for *"takoyaki"* and a suitable amount of boiled octopus was added to the dough, then the resulting mixture was baked to make into *"takoyaki".* After preserving the *"takoyaki"* under refrigerating or freezing conditions for two months, the *"takoyaki"* was heated and tasted. Both cases of refrigerating or freezing, the tastes of *"takoyaki"* such as preferable taste, strong flavor, preferable texture, etc. just after baking was kept for a long period because the moisture variation of the product was inhibited. Also, the product keeps its texture even after rapping the baked *"takoyaki"* and preserving under low or chilled conditions. The product keeps its shape, surface, and texture even in the case of attaching the dew condensation water.

### Example 66

### Fruits gummi

Thirty parts by weight of sucrose was admixed with 7.5 parts by weight of water and dissolved by heating. Then, 50 parts by weight of MVIA in a syrupy form, obtained by the method in Example 6, was admixed with the solution and boiled down to give a concentration of 87%. After cooling the mixture to 75° C, 17.5 parts by weight of a gelatin solution, which is prepared by mixing seven parts by weight of gelatin (20 bloom) and 10.5 parts by weight of water to swell gelatin and dissolving by heating to 65°C, and three parts by weight of a citric acid solution, which is prepared by mixing 1.5 parts by weight of citric acid and 1.5 parts by weight of water and dissolving the mixture, were further added to the mixture. Further, five parts by weight of commercial fruits juice and 0.1 parts by weight of flavor were admixed with the above mixture, and stirred and put in a starch mold and then leaved overnight to make into fruits gummi with a concentration of 80%. The tastes of the product such as preferable taste, strong flavor, preferable texture, etc., were kept for a long period because the moisture variation of the product was inhibited.

### Example 67

### Caramel

One hundred fifteen parts by weight of sucrose, 140 parts by weight of condensed milk, and 170 parts by weight of MVIA in a powdery form, obtained by the method in Example 7, was mixed by stirring with heating at 35° C. Then, 42 parts by weight of hydrogenated oil, 30 parts by weight of butter, and three parts by weight of emulsifier were admixed with the above mixture. After stirring and emulsifying the resulting mixture, the mixture was boiled down to give a temperature of 122°C. After stopping the heating, one part by weight of sodium chloride and small amount of flavor were admixed with the resulting mixture. The product was spread and cooled on a cooling plate, and then extended to give a thickness of 8 mm and cut with a cutter to make into caramel. The tastes of the product such as preferable taste, strong flavor, preferable texture, etc., were kept for a long period and the product shows no moisture adsorbent because the moisture variation of the product was inhibited.

### Example 68

### Jelly

Ten parts by weight of MVIA in a syrupy form, obtained by the method of Example 1, 2.5 parts by weight of gelatin, 35 parts by weight of a commercial orange juice, five parts by weight of lemon juice, 47.5 parts by weight of water, and 0.05 part by weight of sucralose were mixed and the resulting mixture was kept at 80°C for 25 minutes, poured into a cup, cooled to the ambient temperature, and refrigerated to make into jelly. The moisture variation of the product is inhibited. The product shows no syneresis and good flavor and has a resistance to refrigerating and freezing.

### Example 69

### Fondant

One hundred fifty-four parts by weight of "TREHA®", hydrous crystalline α,α-trehalose commercialized by Hayashibara Shoji Inc., Okayama, Japan, and 35 parts by weight of MVIA in a powdery form, obtained by the method in Example 2, were admixed with 30 parts by weight of water and the resulting mixture was boiled down to give a concentration of 77%. After cooling to 70°C the mixture, it was stirred to make into fondant. As a control, fondant was prepared by using 35 parts by weight of "MALTRUP®", a maltose high content syrup commercialized by Hayashibara Shoji Inc., Okayama, Japan, instead of MVIA. When both products were leaved at an ambient temperature for overnight, fondant prepared by using MIVA of the present invention showed no syneresis and kept a firm block shape with a viscosity and the size of α,α-trehalose crystal was moderately controlled. On the contrary, the control fondant showed remarkable and rough crystallization of α,α-trehalose and transparency, in addition, remarkable syneresis by leaving for overnight. Since MVIA of the present invention inhibits crystallization of α,α-trehalose or the growth of crystal, crystal on the product is small and smooth and the product has a good dissolving property and gloss in comparison with those of the control fondant.

### Example 70

### Hard candy with high moisture content

Sixty parts by weight of sucrose, 55 parts by weight of MVIA in a syrupy form, obtained by the method in Example 1, 3.5 parts by weight of soy sauce, 0.2 part by weight of an amino acid, and 85 parts by weight of water were mixed in a pan and the resulting mixture was boiled down to give a temperature of 145° C to make into hard candy with a moisture content of 4.2%. The moisture variation of the hard candy is inhibited and the hard candy with a high moisture content shows no moisture-absorbing property and sticky surface and has a good taste with a soy sauce flavor. Since the product has a relatively high glass-transition temperature in comparison with that produced by using general syrup as a base, the hardness of candy can be kept at a relatively high temperature. Therefore, the hard candy is easily detached from deposit and has a satisfactory working ability for the production.

### Example 71

### Candy with a satisfactory fragility

Ninety-five parts by weight of "TREHA®", hydrous crystalline α,α-trehalose commercialized by Hayashibara Shoji Inc., Okayama, Japan, and five parts by weight of MVIA in a powdery form, obtained by the method in Example 2, were admixed with 45 parts by weight of water and completely dissolved by heating. Then, the resulting solution was boiled down to give a moisture content of about 15% to make into a supersaturated solution. Suitable amounts of flavor and coloring were admixed with the supersaturated solution and the resulting mixture was poured into a mold with a diameter of about one cm and a depth of about one cm. The mold was leaved at an ambient temperature for overnight to crystallize α,α-trehalose to make into a candy. The moisture variation of the candy is inhibited. The candy shows no moisture-absorbing property and no sticky property. Also, the candy has a good taste and dissolving property and a satisfactory fragility and texture. Since saccharide-derivatives of α,α-trehalose controls the size of α,α-trehalose crystal, fine crystal is formed on the surface of the candy and the candy has a fragility and a moist taste.

### Example 72

### Cotton candy

Sixty-five parts by weight of MVIA in a powdery form, obtained by the method in Example 2, 20 parts by weight of "TREHA®", hydrous crystalline α,α-trehalose commercialized by Hayashibara Shoji Inc., Okayama, Japan, 15 parts by weight of sucrose and suitable amount of flavor were mixed in a pan and boiled down to give a temperature of 145°C to make into candy. The resulting candy was cooled and crushed to make into crushed candy. Cotton candy was prepared by using the crashed candy as a material. The moisture variation of the cotton candy is inhibited. The cotton candy shows no moisture-absorbing property and no sticky surface. The cotton candy has a satisfactory shape-keeping ability that the cotton candy shows no adhesion even in the case of piling up each other and no reduction of bulk even in the case of preserving for three days.

### Example 73

### Chinese sugar coated fried sweet potato

Coarsely cut sweet potatoes were fried for eight minutes in edible oil of 150°C. Separately, sixty parts by weight of sucrose, and 40 parts by weight, on a dry solid basis, of MVIA in a syrupy form, obtained by the method of Example 1, were admixed with water, dissolved by heating, and boiled down to make into candy. The candy was coated on the above fried sweet potatoes to make into Chinese sugar coated sweet potatoes. Since the product comprises MVIA, the sticky property is quickly disappeared with keeping the adequate surface humidity. The product has a crispy taste and satisfactory gloss. The product shows no moisture-absorbing property and no crystallization of sucrose of candy coated on the surface of sweet potatoes and no leak of sugar from the surface and keeps a satisfactory gloss and good flavor even in the case of preserving at -20° C for one year, thawing, and leaving in an ambient temperature for 20 hours.

### Example 74

### Chinese sugar coated fried sweet potato

Coarsely cut sweet potatoes were fried for eight minutes in edible oil of 150°C. Separately, sixty parts by weight of sucrose, 30 parts by weight, on a dry solid basis, of MVIA in a syrupy form, obtained by the method of Example 1, and 10 parts by weight, on a dry solid basis, of "TREHA®", hydrous crystalline α,α-trehalose commercialized by Hayashibara Shoji Inc., Okayama, Japan, were admixed with water, dissolved by heating, and boiled down to make into candy. The candy was coated on the above fried sweet potatoes to make into Chinese sugar coated sweet potatoes. Since the product comprises MVIA, the sticky property is quickly disappeared with keeping the adequate surface humidity. The product has a crispy taste and satisfactory gloss. Since the moisture variation inhibiting property of saccharide-derivatives of α,α-trehalose is increased by α,α-trehalose, the product shows no moisture-absorbing property and no crystallization of sucrose and α,α-trehalose of candy coated on the surface of sweet potatoes and no leak of sugar from the surface and keeps a satisfactory gloss and good flavor even in the case of preserving at -20°C for one year, thawing, and leaving in an ambient temperature for 20 hours.

### Example 75

### Roasted almond

One hundred parts by weight of almond was roasted at 160°C for 15 minutes. Separately, MVIA in a syrupy form, obtained by the method of Example 1, was admixed with water and its concentration was adjusted to 30% by heating to 125° C. To 20 parts by weight of the resulting solution, the above roasted almond was added and stirred gently, and then removed solution and cooled to make into roasted almond. Since the product is coated with MVIA, it hardly absorbs moisture and inhibits the oxidation and decomposition of lipids. Therefore, the product keeps preferable flavors of roasted almond just after preparation.

### Example 76

### Sugar coated gum

To 85 parts by weight, on a dry solid basis, of "TREHA®" , hydrous crystalline α,α-trehalose commercialized by Hayashibara Shoji Inc., Okayama, Japan, suitable amount of water was added and stirred with heating at 65°C to make into a syrup with a concentration of 65% for sugar coating. The resulting syrup was admixed with pre-prepared gum in a rotary pot and gum was sugar coated by repeating the conventional steps of crystallizing the saccharide on the surface of gum under the blow at 40°C and drying. After aging the coated gum, sugar coated gum was prepared. Since the moisture variation of sugar coating is inhibited, the sugar coated gum hardly absorbs moisture even in a high humidity condition and has a transparency and a crispy texture.

### Example 77

### Barley tea

MVIA in a syrupy form, obtained by the method in Example 1 was dissolved in water to make into a solution with a concentration of 10%, on a dry solid basis. One hundred parts by weight of barley was roasted by the conventional method and sprayed equally the above solution with keeping high temperature. After mixing and drying under circulation, the roasted barley was packed in a pouch and packed to make into barley tea. Since the moisture variation of the product is inhibited well, the barley tea hardly absorbs moisture and has a good flavor. Also, since the oxidation and decomposition of lipids are inhibited, the product has a satisfactory preserving stability.

### Example 78

### Sauce for "mitarashi-dango" (kind of Japanese rice cake)

Three hundred eighty parts by weight of sucrose, 550 parts by weight of MVIA in a syrupy form, obtained by the method of Example 1, one parts by weight of seasoning were admixed with 450 parts by weight of water and dissolved. To the resulting solution, 205 parts by weight of soy sauce, 30 parts by weight of *"mirin"* (sweet cooking rice wine), and a mixture of 200 parts by weight of water and 120 parts by weight of starch were added, heated to gelatinize starch, and cooled to make into sauce for *"mitarashi-dango".* The moisture variation of the product is inhibited. The product shows no drying and absorbing moisture when applied on *"dango"* (kind of Japanese rice cake), and keeps preferable gloss for a long period.

### Example 79

### Strawberry jam

To five parts by weight of pectin, 140 parts by weight of MVIA (hydrogenated) in a syrupy form, obtained by the method of example 9 was mixed well. Further, 260 parts by weight of sucrose, 130 parts by weight of "SUNMALT®", a maltose product commercialized by Hayashibara Shoji Inc., Okayama, Japan, and 180 parts by weight of water were admixed with the above mixture and completely dissolved these by heating. To the resulting solution, 350 parts by weight of strawberry which was frozen and thawed was added and the mixture was boiled down to give a concentration of 60% to make into jam. After cooling, pH of the resulting jam was adjusted to 3.2 by adding citric acid and then jam was put in a sealed container and sterilized at 85°C for 30 minutes to make into bottled strawberry jam. The product has a fresh and healthy color, keeps flavor just after preparation and shows no syneresis and color deterioration even after preserving at an ambient temperature for six months.

### Example 80

### Dried and mixed fruits and vegetables

To a mixture of 40 parts by weight of parsley, 40 parts by weight of spinach, 40 parts by weight of lettuce, 40 parts by weight of celery, 40 parts by weight of cabbage, and 100 parts by weight of carrot puree, "SUNMALT®", a maltose product commercialized by Hayashibara Shoji Inc., Okayama, Japan, was added to give a content of 10% and the resulting mixture was blanched at 80°C for one minutes. Further, 200 parts by weight of apple, lemon juice obtained from one lemon, 50 parts by weight of MVIA in a syrupy form, obtained by the method in Example 1, and 50 parts by weight of palatinose were admixed with the above mixture, and the resulting mixture was crushed with a mixer and heated to give a concentration of about 60%. The resulting product was cooled, spread on a wrap and dried at 60°C for overnight. The product is a dried and mixed fruits and vegetables, with no stickiness and good flavor.

The above dried and mixed fruits and vegetables was put on a dough for cookie which is prepared by the conventional method and spread on a wrap to give a thickness of about three mm, and then rolled. The resulting roll was cut to give a width of one cm and baked in an oven to make into cookie. Since the transition of moisture in dried and mixed fruits and vegetables to cookie part is inhibited, the cookie keeps a dried state and dried and mixed fruits and vegetables keeps moderate moisture. Therefore, the cookie keeps flavor of just after baking for a long period.

### Example 81

### Dried and mixed fruits and vegetables

To a mixture of 40 parts by weight of parsley, 40 parts by weight of spinach, 40 parts by weight of lettuce, 40 parts by weight of cabbage, 40 parts by weight of celery, and 100 parts by weight of carrot puree, "TREHA®", α,α-trehalose commercialized by Hayashibara Shoji Inc., Okayama, Japan, was added to give a content of 10% and the resulting mixture was blanched at 80°C for one minutes. Further, 200 parts by weight of apple, lemon juice obtained from one lemon, 80 parts by weight of MVIA in a powdery form, obtained by the method in Example 12, and 20 parts by weight of α,α-trehalose were admixed with the above mixture, and the resulting mixture was crushed with a mixer and heated to give a concentration of about 60%. The resulting product was cooled, spread on a wrap and dried at 60°C for overnight. The product is a dried and mixed fruits and vegetables, with no stickiness and good flavor.

### Example 82

### Merengue

Seventy parts by weight of palatinose, 30 parts by weight of concentrated (5-fold) coffee, nine parts by weight of MVIA in a powdery form, obtained by the method in Example 2 were mixed and heated to give a concentration of 75%. Separately, a solution was prepared by mixing 1.5 parts by weight of "SK- 5" , a vegetable protein product commercialized by Chiba Seifun Co. Ltd. , Chiba, Japan, 10 parts by weight of palatinose, and 15 parts by weight of water. The solution was added to the above mixture, and the resulting mixture was whipped. After adjusting the specific gravity to 0.4, the whipped product was shaped and dried in a thermostatic chamber at 50°C for three hours to make into merengue. Since the moisture variation of the merengue is inhibited, the product hardly absorbs moisture and keeps the shape for a long period. Also, the merengue has a satisfactory bulk and good texture.

### Example 83

### Merengue

Seventy parts by weight of "TREHA®", α,α-trehalose commercialized by Hayashibara Shoji Inc., Okayama, Japan, 30 parts by weight of concentrated (5-fold) coffee, nine parts by weight of MVIA in a powdery form, obtained by the method in Example 4 were mixed and heated to give a concentration of 75%. Separately, a solution was prepared by mixing 1.5 parts by weight of "SK-5", a vegetable protein product commercialized by Chiba Seifun Co. Ltd. , Chiba, Japan, 10 parts by weight of α,α-trehalose , and 15 parts by weight of water. The solution was added to the above mixture, and the resulting mixture was whipped. After adjusting the specific gravity to 0.4, the whipped product was shaped and dried in a thermostatic chamber at 50°C for three hours to make into merengue. Since the moisture variation of the merengue is inhibited, the product hardly absorbs moisture and keeps the shape for a long period. Also, the merengue has a satisfactory bulk and good texture.

### Example 84

### Chocolate cookie

Chocolate cookie was prepared by the conventional method using 140 parts by weight of soft flour, 90 parts by weight of butter, 115 parts by weight of chocolate, 360 parts by weight of sucrose, 200 parts by weight of whole egg, 200 parts by weight of almond, and 50 parts by weight of MVIA in a powdery form, obtained by the method in Example 3. After preserving at an ambient temperature for three months, the chocolate cookie was tasted. The moisture variation of the cookie was inhibited and the cookie showed no moisture absorption and not dried. The cookie also showed no oxidation and decomposition of fats and kept flavor of just after preparation.

### Example 85

### Pie

Pie was prepared by the conventional method using 100 parts by weight of flour, two parts by weight of sucrose, six parts by weight of MVIA in a syrup form, obtained by the method in Example 1, three parts by weight of fat, three parts by weight of whole egg, two parts by weight of skim milk, 0.3 part by weight of baking soda, 50 parts by weigh of fresh milk, and 100 parts by weight of water. After preserving in a refrigerator for one week, the pie was tasted. The moisture variation of the cookie was inhibited and the cookie showed no moisture absorption and not dried. The cookie also showed no oxidation and decomposition of fats, no retrogradation of gelatinized starch, and kept flavor of just after preparation.

### Example 86

### Japanese rice cracker

MVIA in a powdery from, obtained by the method in Example 2, was dissolved in a sauce for *"senbei"* (Japanese rice cracker) to give a concentration of 35%. Glutinous rice was steamed by the conventional method and pounded with a machine to make into *"mochi"* (Japanese rice cake). After preserving at low temperature for two days, the *"mochi"* was cut to give a cube (3 cm x 3 cm x 1 cm) and dried to give a moisture content of about 20%. The resulting cubes were baked in an oven at 300°C and immediately soaked into the above sauce for *"senbei",* containing MVIA, for 10 seconds. After removing the sauce adhered to baked cubes, the cubes were dried at 115°C for about 30 minutes and leaved at an ambient temperature to make into Japanese rice cracker. The Japanese rice cracker contains 10% of saccharide-derivatives of α,α-trehalose which are effective ingredients of MVIA. The Japanese rice cracker has a crispy texture because the moisture content of the surface is 1-3%. However, since the internal moisture content is 6-10%, it also shows texture of *"mochi".* Further, the moisture variation is inhibited by saccharide-derivatives of α,α-trehalose, the Japanese rice cracker has a feature of keeping texture of just after baking.

### Example 87

### Japanese rice cracker

MVIA in a powdery from, obtained by the method in Example 2, and α,α-trehalose were dissolved in a sauce for *"senbei"* (Japanese rice cracker) to give concentrations of 20% and 10%, on a dry solid basis. Glutinous rice was steamed by the conventional method and pounded with a machine to make into *"mochi"* (Japanese rice cake). After preserving at low temperature for two days, the *"mochi"* was cut to give a cube (3 cm x 3 cm x 1 cm) and dried to give a moisture content of about 20%. The resulting cubes were baked in an oven at 300°C and immediately soaked into the above sauce for *"senbei",* containing MVIA, for 10 seconds. After removing the sauce adhered to baked cubes, the cubes were dried at 115°C for about 30 minutes and leaved at an ambient temperature to make into Japanese rice cracker. The Japanese rice cracker contains 10% of saccharide-derivatives of α,α-trehalose which are effective ingredients of MVIA. The Japanese rice cracker has a crispy texture because the moisture content of the surface is 1-3%. However, since the internal moisture content is 6-10%, it also shows texture of *"mochi" .* Further, the moisture variation inhibiting activity of saccharide-derivatives of α,α-trehalose is increased by α,α-trehalose, the Japanese rice cracker has a feature of keeping texture of just after baking.

### Example 88

### Pudding

Two hundred parts by weight of fresh milk, 60 parts by weight of MVIA in a powdery from, obtained by the method in Example 2, 150 parts by weight of sucrose, 450 parts by weight of fresh cream, and 150 parts by weight of water were admixed with 150 parts by weight of gently stirred whole egg and the resulting mixture was pureed. Then, the mixture was poured into a vessel for pudding containing suitable amount of caramel and heat-sealed. The vessel was steamed in a steamer for 20 minutes and cooled to make into pudding. The hardness of the pudding measured by using a rheometer was 26 g/cm2 and the pudding had a soft and pleasant taste. Since the moisture variation of the pudding is inhibited, it can be preserved for a long period with either method of freezing, chilling, or refrigerating. The pudding shows no deterioration in hardness and texture, no syneresis, and a satisfactory stability and keeps flavor of just after preparation. Since the generation of bad smell, originated from hydrogen sulfide which is generated in the case of heating egg, is inhibited, the pudding has a good flavor and fine texture.

### Example 89

### Lact ice

Eight parts by weight of MVIA in a powdery form, obtained by the method in Example 2, 8.5 parts by weight of sucrose, three parts by weight of palm oil, four parts by weight of skim milk, 0.3 part by weight of stabilizer, 0.3 part by weight of emulsifier, and 72.9 parts by weight of water were mixed at 70°C and homogenized at 12,000 rpm for 10 minutes. The homogenate was sterilized at 70° C for 30 minutes and cooled, and then stand for one day. Further, 0.3 part by weight of vanilla essence and 0.03 part by weight of sucralose was admixed with the above homogenate and frozen. Then, the frozen homogenate was treated to give a overrun of 45% and divided into a cup to make into lact ice. After keeping the resulting lact ice at -45°C for 24 hour, it was further preserved at -18° C for six months. The lact ice is not watery and has uniform internal and surface moisture content. The lact ice has a satisfactory relish such as taste, flavor, dissolving property and smoothness.

### Example 90

### Ice cream

Ice cream was prepared by the conventional method using 61 parts by weight of water, 12 parts by weight of sucrose, 12 parts by weight of MVIA in a powdery form, obtained by the method in Example 2, 12 parts by weight of solid non fat milk, four parts by weight of corn syrup, 0.2 part by weight of vanilla extract, and 0.1 part by weight of stabilizer. Since the moisture variation of the ice cream is inhibited, it has a smooth texture almost equal with ice cream prepared by using about 30% of cream (fat content of 40%), though the ice cream is free from fat. In addition the ice cream exhibits low calorie (about 60%) compared with that prepared using fat.

### Example 91

### Freeze-dried green onion

One hundred grams of green onion cut in round slice with a length of 1cm was soaked into a solution, which is prepared by dissolving MVIA in a powdery form, obtained by the method in Example 10, to give a concentration of 8%, for three hours and then freeze-dried by the conventional method to make into dried green onion.

After preserving the freeze-dried onion at 15°C for six hours, the dried green onion was reconstituted in a hot water at 85°C for two minutes and tasted. The relish of the reconstituted green onion such as taste, flavor, color, shape, and texture was equivalent with fresh green onion. Since the dried green onion is easily reconstituted in water, it is preferable as a filling for instant foods.

### Example 92

### "Tofu" (bean curd)

To 250 parts by weight of soy milk, one part by weight of glucono-delta-lactone, one part by weight of bittern, and 25 parts by weight of MVIA in a powdery form, obtained by the method in Example 2, were added and dissolved. The resulting solution was poured into a container, sealed, and heated at 90°C for 40minutes to make into a packed *"tofu".*

The moisture variation of the *"tofu"* is inhibited. It has a fine texture and keeps the shape. Also, the product shows no syneresis and keeps flavor of just after preparation even in the case of preserving by frozen, chilled, or refrigerated condition.

The *"tofu"* was pulled out from the container and cut into cubes with a size of about one cm. After freezing the cubes at -80°C, they were freeze-dried for one day by the conventional method using a freeze-drier. The dried *"tofu"* is reconstituted by soaking into hot water at 85°C for one minute even after preserving at an ambient temperature for three months and has a relish equivalent with that just after preparation. Therefore, the dried *"tofu"* is preferable as a filling for instant foods.

### Example 93

### Bacon

Twenty-two parts by weight of sodium chloride, 2.5 parts by weight of MVIA in a powdery form, obtained by the method in Example 3, two parts by weight of sucrose, two parts by weight of sodium lactate, two parts by weight of sodium polyphosphate, 0.5 part by weight of ascorbic acid, and 0.2 part by weight of sodium nitrite were admixed with 68.8 parts by weight of water and dissolved to make into a pickle-solution. To nine parts by weight of pork lib, one part by weight of the above pickle-solution was injected to penetrate the solution into the meat evenly. The resulting meat was smoked by the conventional method to make into bacon. After smoking, the bacon was leaved at an ambient temperature for overnight and then sliced, vacuum-sealed, and preserved at 10°C. The moisture variation of the product is inhibited and the product keeps flavor of jut after preparation even after preserving for one week. Since the bacon contains saccharide-derivatives of α,α-trehalose, effective ingredients of MVIA of the present invention, and sodium lactate, the growth of contaminants is also inhibited. Since the denaturation of the proteins is also inhibited, the bacon shows no syneresis and good flavor after preserving with frozen condition and thawing.

### Example 94

### Processed liquid whole egg

Seven parts by weight of MVIA in a powdery form, obtained by the method in Example 2, was admixed with 93 parts by weight of egg and dissolved by stirring. The resulting liquid whole egg was heated to 65°C and kept for six minutes to make into processed liquid whole egg. Since the moisture variation of the product is inhibited and the denaturation of proteins by heating was not occurred, the product has a satisfactory whipping property. Since the product was processed, the contamination of microorganisms such as salmonella, which is a problem of egg, is not observed. Also, since the product has no α-amylase activity, the product can be used as a material of various foods and beverages such as Japanese or Western confectionaries and daily dishes as in the case of fresh egg.

To 200 parts by weight of the product which is preserved either at 4°C for 14 days, -1°C for 28 days, or -20°C for three months, 100 parts by weight of sucrose, 30 parts by weight of MVIA in a powdery form, obtained by the method in Example 2, 115 parts by weight of soft flour, and 40 parts by weight of salt-free butter were added and prepared a sponge cake by the conventional method. Since either of liquid whole egg shows a satisfactory whipping property and the denaturation of proteins during freezing or refrigerating is inhibited, sponge cakes, having equivalent relish such as taste, flavor, color, and texture and bulk with that prepared by using fresh egg, can be prepared. After preserving the sponge cakes at 4°C for four days, they were tasted. The products showed no retrogradation of gelatinized starch and kept relish such as taste of sponge cake, flavor, color, and texture of just after preparation.

### Example 95

### Retort-packed curry

Five parts by weight of flour and 4.5 parts by weight of lard were mixed and heated on an open fire, and then 1.5 parts by weight of curry powder was further admixed with the mixture and heated. Successively, 82 parts by weight of water, one part by weight of sodium chloride, 2.5 parts by weight of instant bouillon, and 3.5 parts by weight of MVIA in a powdery form, obtained by the method in Example 2 were admixed with the above mixture and boiled thoroughly for 10 minutes to make into curry roux. To 150 grams of the roux, 35 grams of beef, 15 grams of potato, and 20 grams of carrot, which are boiled thoroughly in an aqueous solution containing 5% of MIVA obtained by the method of Example 2 and 0.5% of calcium chloride, were added and the resulting mixture was stirred gently, packed in a retort pouch, and autoclaved at 121°C or 25 minutes.

After preserving the retort pouch at an ambient temperature for six months, it was broken seal and curry was tasted. The both roux and fillings kept relish such as taste, flavor, color, and texture of just after preparation. Also, the disintegration of fillings, the puckering of meats, and the deterioration of texture, which are problems on the production of retort foods, were inhibited. Beef, potato, and carrot, boiled in a solution containing MVIA and calcium chloride, shows no disintegration, no deterioration during freezing, and no dripping during thawing. Therefore, they can be preferably used as fillings for not only curry but also other retort foods and frozen foods.

### Example 96

### Pot-steamed hotchpotch

To 600 parts by weight of soup stock, 34 parts by weight of *"mirin"* (sweet cooking rice wine), 40 parts by weight of MVIA in a powdery form, obtained by the method in Example 2, six parts by weight of sodium chloride, and four parts by weight of light soy sauce were mixed and the resulting mixture was heated to 60°C. Then, nine parts by weight of powdery gelatin and two parts by weight of locust bean gum were dissolved in the mixture. After cooling the resulting mixture, 200 parts by weight of stirred whole egg was gently admixed with the mixture and filtrated. The filtrate was pored into a vessel containing chicken meat, ginkgo nuts, *"mitsuba"* (Japanese hornwort) gently and steamed in a steamer to make into pot-steamed hotchpotch. The product has a fine texture and good flavor of soup and fillings. The product is a delicious pot-steamed hotchpotch whose generation of bad smell originated from hydrogen sulfide.

The product was heated and tasted after freezing at -40°C and preserving at -20°C for one month. The spongy texture and syneresis, which are observed in the case of thawing the conventional frozen pot-steamed hotchpotch, are inhibited. The product keeps flavor of just after preparation.

### Example 97

### Salad dressing

Salad dressing was prepared by mixing 40.8 parts by weight of water, 20 parts by weight of distilled white vinegar, 15 parts by weight of plant oil, five parts by weight of sucrose, two parts by weight of sodium chloride, one part by weight of garlic powder, 0.7 part by weight of onion powder, 0.1 part by weight of ground white pepper, 0.3 part by weight of xanthan gum, 0.1 part by weight of potassium sorbate, and 15 parts by weight of MVIA in a powdery form, obtained by the method in Example 2. Since saccharide-derivatives of α,α-trehalose inhibit radical reaction, the deterioration of oils and flavors of garlic powder, onion powder, and ground white pepper. Therefore, the product stably keeps it quality for a long period. Further, since saccharide-derivatives of α,α-trehalose provide cream-like rich texture of fat as a substitute, the salad dressing is a remarkably low calorie dressing in comparison with a conventional product containing fat and keeps delicious taste though it contains only a half amount of plant oil compared with a conventional dressing. Furthermore, since the product contains saccharide- derivatives of α,α-trehalose in its waster phase, it is a high quality salad dressing with a good separating ability of oil phase and water phase in comparison with that containing only sucrose.

### Example 98

### Mayonnaise-type product

Ten parts by weight of vinegar, 10 parts by weight of MVIA in a syrupy form, obtained by the method in Example 1, eight parts by weight of sterilized whole egg, 3.5 parts by weight of water, two parts by weight of sodium chloride, 0.5 part by weight of sucrose, 0.5 parts by weight of mustard powder, and 0.5 parts by weight of sodium glutamate were mixed by stirring. Further, 45 parts by weight of salad oil was admixed with the mixture and emulsified by the conventional method to make into mayonnaise-type product. The product shows almost the same texture with mayonnaise. In the case of using the product to prepare salad with pasta, the moisture variation from the product to pasta or from the salad to the product was inhibited and the mayonnaise-like flavor was not deteriorated. Since saccharide-derivatives of α,α-trehalose inhibit radical reaction, the deterioration of oil and flavor of mustard and isolation of fat were inhibited. Therefore, the product stablykeeps its quality even after preserving in ambient, chilled, refrigerated, or frozen conditions. Further, since saccharide-derivatives of α,α-trehalose provide cream-like rich texture of fat as a substitute, the product has a relatively low calorie food showing the flavor of mayonnaise even though using a low amount plant oil.

### Example 99

### Cream

Thirty-five parts by weight of a mixed fat, prepared by mixing 80 parts of fractionated shea butter (melting point: 38°C) and 20 parts of rapeseed oil (melting point: 35°C), 0.3 parts by weight of soybean lecithin (HLB 3), 0.03 parts by weight of monoglycerin fatty acid ester (HLB 3), 0.15 parts by weight of hexa-glycerin penta-ester, 60 parts by weight of water, four parts by weight of skim milk, and phosphoric acid-alkaline metal salt were mixed and pre-emulsified by the conventional method. After homogenizing under a pressurized condition at 70 kg/cm², the homogenate was autoclaved at 145°C for few seconds. Then, the homogenate was re-homogenized under a pressurized condition at 70 kg/cm². After cooling the homogenate, it was aged for about 24 hours to make into an emulsion with a foaming property. Eight parts by weight of MVIA in a powdery form, obtained by the method in Example 2, was admixed with 100 parts by weight of the emulsion with a foaming property and whipped for two minutes and 45 seconds to make into a cream with an overrun 75%. Since saccharide-derivatives of α,α-trehalose inhibit the oxidation of lipids, the deterioration of flavor of the product is inhibited. In addition, saccharide-derivatives of α,α-trehalose in the product masks unpleasant smell originated from emulsifier such as fatty acid esters. The both products preserved at 5-20°C for seven days or thawed after freezing at -20°C for 14 days, keep the shape, flavor, texture, creamy property and show no change of appearance such as crack. Since the product comprises saccharide-derivatives of α,α-trehalose, the product has features such as satisfactory whipping property and bulk-keeping ability and gives fine foam.

### Example 100

### Custard cream

One hundred parts by weight of corn starch, 100 parts by weight of MVIA in a syrupy form, obtained by the method in Example 1, 70 parts by weight of maltose, 30 parts by weight of sucrose, and one part by weight of sodium chloride were mixed well, and then 280 parts by weight of whole egg was further admixed with the mixture. Successively, 1,000 parts by weight of boiled milk was gradually admixed with the resulting mixture and the resulting solution was continuously stirred on an open flame.
The heating was stopped at the point that corn starch was completely gelatinized to give a transparency. After cooling the mixture, a suitable amount of vanilla essence was admixed with the mixture to make into custard cream. Since the moisture variation of the product is inhibited, the denaturation of protein and the retrogradation of starch were inhibited, and the product shows no syneresis and keeps flavor of just after preparation for a long period. The product is a delicious custard cream in which unpleasant smell originated from hydrogen sulfide generated in the case of heating egg.

### Example 101

### Syrup containing Green-tea or green powdered tea

To 210 parts by weight of water, 210 parts by weight of "TREHA®", hydrous crystalline α,α-trehalose commercialized by Hayashibara Shoji Inc., Okayama, Japan, was added and completely dissolved by stirring and heating to about 70° C. After cooling the resulting solution to 25° C, 120 parts by weight of either of powdery green tea, prepared in Example 38, or a commercial green powdered tea was admixed the solution and stirred about 30 minutes. To each solution, 440 parts by weight of MVIA in a syrupy form, obtained by the method of Example1 and 20 parts by weight of chlorella were added and the resulting mixture was stirred and sieved to make into syrup containing green tea or green powdered tea. Since the moisture variation of these product was inhibited even in the case of preserving under frozen or refrigerating conditions, the deterioration of relish such as the color of tea-chlorophyll and flavor of tea was inhibited by repeating the freezing and thawing. These syrups have mild sweetness compared with those powderized using sucrose, dextrin, cyclodextrin, etc. In the case of diluting the products with water or hot water, the deterioration of relish such as color and flavor is inhibited for a ling period in comparison with the case of diluting tea extract in a same manner with adding α,α-trehalose or a syrup containing saccharide-derivatives of α,α-trehalose. Therefore, those products can be used as a health supplement intact or in a form of beverage prepared by diluting the products with water, hot water, milk, and others. Further, those products can be used as a material of various foods and beverages, containing tea. Those syrups can be arbitrarily used by filling a portion type vessel.

### Example 102

### Feed mixture

Thirty parts by weight of powderized wheat gluten, 35 parts by weight of skim milk, 10 parts by weight of rice bran, obtained as a by-product by the method of Example 43 , 10 parts by weight of lactosucrose high content powder, 10 parts by weight of a multi-vitamin agent, five parts by weight of fish powder, five parts by weight of calcium monohydrogen-phosphate, three parts by weight of a liquid fat, three parts by weight of calcium carbonate, two parts by weight of sodium chloride, two parts by weight of MVIA in a syrupy form, obtained by the method of Example 8, and two parts by weight of a mineral agent were mixed to make into a mixed feed. Since the moisture variation of the product and the oxidation and decomposition of lipids were inhibited well, the product can be preferably used as a feed with a satisfactory preservability for domestic animals, poultry, and pets, and is particularly preferable as a feed for pigs. Since the product comprises lactosucrose, intestinal bifidobacteria of those can be grown and the condition of intestine can be improved to promote the adsorption of minerals. Therefore, the product can be advantageously used for preventing infection and diarrhea, for promoting growth, and for inhibiting the smell of faces. The product can be optionally used as a mixed feed by incorporating with other feed materials including concentrated feeds such as grains, flour, starch, oil meal, and waste, and coarse feeds such as straw, hey, bagasse, corn cob.

### Example 103

### Soap

A neat soap was obtained by applying a mixture of four parts by weight of beef tallow and one part by weight of coconuts oil to the conventional saponification-salting out method. To 95.5 parts by weight of the neat soap, 1.5 parts by weight of MVIA in a powdery form, obtained by the method in Example 3, 0.5 part by weight of "AA2G™", ascorbic acid 2-glucoside commercialized by Hayashibara Biochemical Laboratories Inc., Okayama, Japan, 0.5 part by weight of sucrose, 0.5 part by weight to "αG-RUTIN™", a glycosyl-rutin product commercialized by Hayashibara Biochemical Laboratories Inc., Okayama, Japan, one part by weight of maltitol, 0.0001 part by weight of *"kankoso* No. 201", and suitable amount of flavor were added and mixed to homogeneity. The mixture was poured into a mold, cooled and solidified to make into soap. Since the moisture variation of the soap is inhibited, the soap has a good shape-keeping property. Also, since the oxidation and decomposition of lipids originated from sweat, grime, sebum, etc., the soap can be advantageously used for preventing the generation of body odor and itch.

### Example 104

### Cosmetic cream

Two parts by weight of polyoxiethylenglycol mono-stearate, five parts by weight of self-emulsified glycerin mono-stearate, five parts by weight of potassium DL-lactate, one part by weight of behenylalcohol, two parts by weight of eicosatetraenoic acid, one part by weight of liquidparaffin, 10 parts byweight of glycerin trioctanoate, and suitable amount of preservative were mixed and dissolved by the conventional heating method. Then, seven parts by weight of MVIA in a powdery form, obtained by the method in Example 4, three parts by weight of 1, 3-butylene glycol, and 66 parts by weight of purified water were admixed with the above mixture and emulsified using a homogenizer. Further, suitable amount of flavor was admixed with the emulsion and stirred to make into cream. Since the moisture variation of the product is inhibited, the oxidation and decomposition of lipids in the product is inhibited. The product is a skin-whitening product whose quality is stabilized, showing no deterioration of color and no generation of bad smell. The product can be advantageously used for preventing cutaneous stimulation and itch and for curing or preventing pigmentation such as fleck, freckle, sunburn, etc. The cream has a great availability and shows no stickiness when it is applied on the skin.

### Example 105

### Cosmetic cream

Two parts by weight of polyoxiethylenglycol mono-stearate, five parts by weight of self-emulsified glycerin mono-stearate, five parts by weight of potassium DL-lactate, one part by weight of behenylalcohol, two parts by weight of eicosatetraenoic acid, one part by weight of liquid paraffin, 10 parts by weight of glycerin trioctanoate, two parts by weight of "AA2G™", ascorbic acid 2-glucoside commercialized by Hayashibara Biochemical Laboratories Inc., Okayama, Japan, and suitable amount of preservative were mixed and dissolved by the conventional heating method. Then, five parts by weight of MVIA in a powdery form, obtained by the method in Example 7, 0.1 part by weight of sodium hyaluronate, 0.1 parts by weight of dipotassium glycyrrhizinate, 0.1 part by weight of aloe barbadensis, 0.05 parts by weight of mellssa extract, 0.05 part by weight of chamomile extract, "αG-HESPERIDIN™", a glycosyl-hesperidin product commercialized by Hayashibara Biochemical Laboratories Inc. , Okayama, Japan, one part by weight of indigo extract, three parts by weight of 1,3-butylene glycol, and 66 parts by weight of purified water were admixed with the above mixture and emulsified using a homogenizer. Further, suitable amount of flavor was admixed with the emulsion and stirred to make into cream. Since the moisture variation of the product is inhibited, the oxidation and decomposition of lipids in the product is inhibited. The product is a skin-whitening product whose quality is stabilized. Also, since the oxidation and decomposition of lipids originated from sweat, grime, scurf, sebum, etc., the product can be advantageously used for preventing the generation of body odor, cutaneous stimulation and itch, and for curing or preventing pigmentation such as fleck, freckle, sunburn, etc., and aging of skins. The product has a satisfactory moisture-keeping property and can be used with no fear of hypersensitivity because of its low cutaneous-stimulating property. The cream has a great availability and shows no stickiness and good feeling when it is applied on the skin.

### Example 106

### Cosmetic emulsion

Two-point five parts by weight of stearic acid, 1.5 parts by weight of cetanol, five parts by weight of vaseline, 10 parts by weight of liquid paraffin, two parts by weight of polyoxyethylene oleate, 0.5 part by weight of tocopherol acetate, 0.2 part by weight of dipotassium gylcyrrhizinate, three parts by weight of polyethyleneglycol (1500), three parts by weight of "AA2G™", ascorbic acid 2-glucoside commercialized by Hayashibara Biochemical Laboratories Inc. , Okayama, Japan, three parts by weight of a water-extract of indigo, one parts by weight of "αG-RUTIN™", glycosyl rutin commercialized by Hayashibara Biochemical Laboratories Inc., Okayama, Japan, one part by weight of triethanolamine, four parts by weight of MVIA in a powdery form, obtained by the method in Example 7, 66 parts by weight of purified water, and 0.1 part by weight of propyl paraben were mixed and the pH of the resulting mixture was adjusted to 6.7 with potassium hydroxide. Further, a suitable amount of flavor was admixed with the mixture and made into an emulsion by the conventional method. Since the moisture variation of the product is inhibited well, the product is an emulsion for skin whitening with a satisfactory availability and no stickiness after applying. Also, since the product inhibits the formation of volatile aldehydes and/or the oxidation and decomposition of lipids, it can be used for preventing the body odor, cutaneous stimulation and itch, and for curing or preventing pigmentation such as fleck, freckle, sunburn, etc., and aging of skins. The product has a satisfactory moisture-keeping property and can be used with no fear of hypersensitivity because of its low cutaneous-stimulating property.

### Example 107

### Hair conditioner

Two-point five parts by weight of liquid paraffin, 0.5 parts by weight of myristic acid, 1.5 parts by weight of cetanol, three parts by weight of glycerin mono-stearate, one part by weight of lauroyl glutamate polyoxyethyleneoctyldodecylether diester, 0.5 parts by weight of polyoxyethyleneglyceryl pyroglutamate isostearate, and 0.1 parts by weight of photosensitizing dye No.301 were mixed and heated. Separately, three parts by weight of MVIA in a powdery form, obtained by the method in Example 7, 2.5 parts by weight of lauroyl L-lysine, 0.5 part by weight of fatty acid L-arginine ethylpyrrolidonecaboxylic acid salt, 0.5 part by weight of stearyl trimethylammonium chloride, 0.1 part by weight of "αG-RUTIN™", glycosyl rutin commercialized by Hayashibara Biochemical Laboratories Inc., Okayama, Japan, one part by weight of sodium pyrrolidonecaboxylic acid and 75 parts by weight of purified water were mixed and heated. The resulting mixture and the above mixture were mixed and emulsified by the conventional method to make into a hair conditioner. Since the moisture variation of the product is inhibited, it can be used as a hair conditioner with a satisfactory availability. Also, since the product inhibits the formation of volatile aldehydes and/or the oxidation and decomposition of lipids, it can be advantageously used for preventing the bad smell originated from scarp sebum, itch and scurf, for increasing hair growth, and for curing and preventing the aging of scarp. Since the product comprises saccharide-derivatives of α,α-trehalose which are effective ingredients of MVIA of the present invention, the product has a satisfactory moisture-keeping property though it not comprise glycerin. The product can be used with no fear of hypersensitivity because of its low cutaneous-stimulating property.

### Example 108

### Shampoo

Thirty-five parts by weight of 2-alkyl-N-carboxymethyl-N-hydroxymethyl-imidazolium betain (30% aqueous solution), 35 parts by weight of coconut oil-fatty acid triethanolamin glutamate solution (30% aqueous solution), 10 parts by weight of MVIA in a powdery form, obtained by the method in Example 7, 10 parts by weight of potassium cocoyl glycinate (30% aqueous solution), 2.3 parts by weight of coconut oil-fatty acid diethanolamide, three parts by weight of "αG-HESPERIDIN™", glycosyl hesperidin commercialized by Hayashibara Biochemical Laboratories Inc., Okayama, Japan, three parts by weight of "AA2G™", ascorbic acid 2-glucoside commercialized by Hayashibara Biochemical Laboratories Inc., Okayama, Japan, 0.1 parts by weight of photosensitizing dye No.201, 0.1 parts by weight of photosensitizing dye No.301, and 10 parts by weight of purified water were mixed and dissolved by heating to 70°C and stirring. Further, a suitable amount of flavor was admixed with the mixture and made into a shampoo by the conventional method. Since the moisture variation of the product is inhibited, it can be used as a shampoo with a satisfactory foaming property and availability. Also, since the product inhibits the formation of volatile aldehydes and/or the oxidation and decomposition of lipids, it can be advantageously used for preventing the bad smell originated from scarp sebum, itch and scurf, for increasing hair growth, and for curing and preventing the aging of scarp. Since the product comprises saccharide-derivatives of α,α-trehalose which are effective ingredients of MVIA of the present invention, the product has a satisfactory moisture-keeping property though it not comprise glycerin. The product can be used with no fear of hypersensitivity because of its low cutaneous-stimulating property.

### Example 109

### Hair treatment

Five parts by weight of stearyl alcohol, five parts by weight of glycerin mono-stearate, 3.5 parts by weight of liquid paraffin, two parts by weight of lauroylglutamate polyoxyethyleneoctyldodecylether diester, and one part by weight of polyoxyethyleneglyceril pyroglutamate isostearate were mixed with heating. Separately, five parts by weight of MVIA in a powdery form, obtained by the method in Example 7, three parts by weight of 1,3-butyleneglycol, one part by weight of stearyl trimethylammonium chloride, one part by weight of sodium pyrrolidonecarboxylic acid, 0.1 part by weight of "αG-RUTIN™", glycosyl rutin commercialized by Hayashibara Biochemical Laboratories Inc., Okayama, Japan, and 65 parts by weight of deionized water were mixed with heating. The resulting mixture and the above mixture were mixed and emulsified by the conventional method to make into a hair treatment. Since the moisture variation of the product is inhibited, it can be used as a hair treatment with a satisfactory availability. Also, since the product inhibits the formation of volatile aldehydes and/or the oxidation and decomposition of lipids, it can be advantageously used for preventing the bad smell originated from scarp sebum, itch and scurf, for increasing hair growth, and for curing and preventing the aging of scarp. Since the product comprises saccharide-derivatives of α,α-trehalose which are effective ingredients of MVIA of the present invention, the product has a satisfactory moisture-keeping property though it not comprise glycerin. The product can be used with no fear of hypersensitivity because of its low cutaneous-stimulating property.

### Example 110

### Body soap

Fifteen parts by weight of potassium laurate, five part by weight of potassium myristate, four parts by weight of MVIA in a powdery form, obtained by the method in Example 7, two parts by weight of propylene glycol, 0.5 part by weight of polyethylene powder, 0.5 part by weight of hydroxylpropylchitosan solution, 0.25 part by weight of glycine, 0.25 part by weight of glutamine, 0.1 part by weight of photosensitizing dye No.201, a suitable amount of phenol, pH conditioner, and a suitable amount of lavender water were mixed and further admixed with purified water to give a final weight of 100 parts by weight. The resulting solution was emulsified by the conventional method to make into body soap. Since the moisture variation of the product is inhibited, it can be used as a body soap with a satisfactory foaming property and availability. Also, since the product inhibits the formation of volatile aldehydes and/or the oxidation and decomposition of lipids, it can be advantageously used for preventing body odor and itch, and for curing and preventing the aging of skin. Since the product comprises saccharide-derivatives of α,α-trehalose which are effective ingredients of MVIA of the present invention, the product has a satisfactory moisture-keeping property. The product can be used with no fear of hypersensitivity because of its low cutaneous-stimulating property.

### Example 111

### Tooth paste

Forty parts by weight of calcium monohydrogen phosphate, 25 part by weight of glycerin, fifteen parts by weight of MVIA in a syrupy form, obtained by the method in Example 1, 1.5 parts by weight of sodium lauryl sulfate, one part by weight of "αG-HESPERIDIN™", glycosyl hesperidin commercialized by Hayashibara Biochemical Laboratories Inc., Okayama, Japan, one part by weight of sodium carboxymethyl-cellulose, 0.7 part by weight of sodium monofluorophospahte, 0.5 part by weight of polyoxyethylene sorbitanlaurate, 0.05 part by weight of preservative, 0.02 part by weight of saccharin, and 13 parts by weight of water were mixed to make into tooth paste. The product can be preferably used as a tooth paste having a good availability, gloss, and washing property.

### Example 112

### Ointment for curing wound

Four hundred fifty parts by weight of macrogol, three parts by weight of carboxyvinyl polymer, one part by weight of pullulan, 400 parts by weight of isopropanol, and one part by weight of chlorhexidine gluconate solution were mixed by stirring in *vacuo.* To the resulting mixture, 70 parts by weight of MVIA in a powdery form, obtained by the method in Example 3, three parts by weight of sodium hydroxide, and 77 parts by weight of purified water were mixed to make into a ointment for curing wound with a suitable extending property and adhesive property. The moisture variation of the product is inhibited well, it shows no stickiness and good availability. The product can be used for curing wounds such as incisure, abration, burn, foot ringworm, frost damage, etc. by applying it to affected area intact or by applying on gauze and using it.

### Example 113

### Multi-vitamin tablet

Five parts by weight of retinol palmitate, five parts by weight of ergocalciferol, 10 parts by weight of fursultiamine, five parts by weight of riboflavin, 10 parts by weight of pyridoxine hydrochloride, 60 parts by weight of ascorbic acid, 10 parts by weight of tocopherol acetate, 30 parts by weight of nicotinic acid amide, 0.01 part by weight of cyanocobalamin, 40 parts by weight of calcium pantothenate, and 15 parts by weight of "AA2G", ascorbic acid 2-glucoside commercialized by Hyayashibara Biochemical Laboratories Inc., Okayama, Japan, were mixed by stirring. To one part by weight of the mixture, 24 parts by weight of MVIA in a powdery form, obtained by the method in Example 3 was mixed by stirring and made into a multi-vitamin tablet using a tablet machine. Since the moisture variation of the product is inhibited even after preserving for a long period, it shows no moisture-adsorption, and the oxidation and decomposition of vitamins are inhibited.

### Example 114

### Eye-drops

Five parts by weight of MVIA in a powdery form, whose pyrogen was removed and obtained by the method in Example 5, 0.4 part by weight of sodium chloride, 0.15 part by weight of potassium chloride, 0.2 part by weight of sodium dihydrogen phosphate, 0.15 part by weight of borax, and 0.1 part by weight of dipotassium glycirrhizinate, were mixed and then sterilized purified water was added to the mixture to give a final weight of 100 parts by weight. The resulting mixture was stirred to make into sterilized eye-drops with no pyrogen. Since MVIA inhibits the moisture variation on the surface of mucosa of eye, the product protects mucosa of eye and cells on the surface of eye from disorder by drying and activates these cells. Therefore, the product can be advantageously used for a preventing agent or curing agent for patients of dry-eye and Sjogren syndrome. Further, since the product comprises saccharide- derivatives of α,α-trehalose, the product has a satisfactory anti-inflammatory activity, and the stimulation on the applying are lowered.

### Example 115

### Growth-promoting agent for plants

Twenty parts by weight of MVIA in a powdery form, obtained by the method in Example 2, was admixed with 77.5 parts by weight of water and dissolved. Then, one gram of gibberelline was added to the above solution and the resulting solution was powderized by the conventional spray-drying method. The resulting powder was granulated to make into granules comprising gibberelline. Since the moisture variation of the product is inhibited, the product shows no moisture-adsorption. The product is a growth-promoting agent for plants being stable for a long period and showing a satisfactory handleability. Since saccharide-derivatives of α,α-trehalose, which are effective ingredients of MVIA of the present invention, exhibits the activity of promoting the growth of plants as in the case of gibberelline, the growth of plants and fruits, and the increase of sugar content in fruits are promoted by dissolving two parts by weight of the product in 98 parts by weight of water and applying the resulting solution on leaves. Also, saccharide-derivatives of α,α-trehalose, which are effective ingredients of MVIA of the present invention can be used as a nutrition for plants and/or for preventing and improving a disorder by dryness, salts, frost, etc.

### Example 116

### Pullulan film

Two hundred parts by weight of "PULLULAN PI-20" , a commercially available pullulan product commercialized by Hayashibara Shoji Inc., Okayama, Japan, 0.5 part by weight of a surfactant (sucrose monolaurate), 10 parts by weight of MVIA in a powdery form, obtained by the method in Example 7, and 10 parts by weight of glycerol were admixed with 780 parts by weight of deionized water to make into a solution for producing a film. After removing bubbles under a reduced pressure, the solution was continuously poured and extended on a synthetic plastic film, then dried by passing through hot-air at 70°C to make into a pullulan film with a thickness of 30 µm. Since the moisture variation of the product is inhibited, the film has a satisfactory stability to the change of temperature. The product can be heat-sealed and has a transparency and gloss and a satisfactory water-solubility. Further, the unpleasant taste of the product, which is originated from a surfactant, is reduced. An edible and water-soluble film prepared from the product can be advantageously used as a material for the secondary processing for the purpose of pinching, stacking, filling various substances such as foods and beverages, cosmetics, pharmaceuticals, chemicals, etc.

### Example 117

### Pullulan capsule

One hundred and fifty parts by weight of "PULLULAN PI-20", a commercially available pullulan product commercialized by Hayashibara Shoji Inc., Okayama, Japan, one part by weight of "GENUVISCO CSW-2", a carrageenan product commercialized by Sansho Co., Ltd., Osaka, Japan, two parts by weight of ammonium chloride, and 42 parts by weight of MVIA in a powdery form, obtained by the method in Example 5 (before removing pyrogen), were admixed with 800 parts by weight of deionized water to make into a material solution. After removing bubbles under a reduced pressure, the resulting material solution was heated to 50° C. A pin for shaping capsule was inserted to the material solution and pulled out, and then dried to make into a capsule. Since the moisture variation of the product is inhibited, the film has a satisfactory stability to the change of temperature. The product has a transparency and gloss and a satisfactory water-solubility. The product can be advantageously used as an edible and water-soluble capsule for filling up various ingredients such as foods and beverages, cosmetics, pharmaceuticals, chemicals, etc.

### INDUSTRIAL APPLICABILITY

As described avove, the present invention inhibits the moisture variation in compositions, further, the denaturation of proteins, the retrogradation of gelatinized starches, and the oxidation and decomposition of lipids, accompanying with the moisture variation, by incorporating saccharide-derivatives of α,α-trehalose into various compositions. Futhermore, since saccharide-derivatives of α,α-trehalose have safety and satisfactory stability, the moisture variation inhibiting agent, comprising the saccharide-derivatives, can be used in various fields such as foods and beverages, cosmetics, medicated cosmetics, pharmaceuticals, daily goods, feeds, pet foods, baits, groceries, and chemical industrial products. The present invention, having these outstanding functions and effects, is a significantly important invention that greatly contributes to this art.

## Claims

1. A method for inhibiting the moisture variation in a composition, **characterized in that** it comprises a step of incorporating a saccharide-derivative of α,α-trehalose into said composition.

2. The method of claim 1, **characterized in that** wherein said saccharide-derivative of α,α-trehalose is composed of any of mono-glucose, di-glucose, tri-glucose, and tetra-glucose bound to at least one of glucose residues of α,α-trehalose molecule.

3. The method of claim 1 or 2, **characterized in that** wherein said saccharide-derivative of α,α-trehalose is a saccharide having a trehalose structure as an end unit.

4. The method of any one of claims 1 to 3, **characterized in that** wherein said saccharide-derivative of α,α-trehalose is in an amorphous form.

5. The method of any one of claims 1 to 4, **characterized in that** wherein said saccharide-derivative of α,α-trehalose is incorporated into said composition in combination with another saccharide.

6. The method of claim 5, wherein said another saccharide is one or more members selected from the group consisting of reducing saccharides, non-reducing saccharides, sugar alcohols, and water-soluble polysaccharides.

7. The method of any one of claims 1 to 6, **characterized in that** wherein said saccharide-derivative of α,α-trehalose is incorporated in an amount of at least one percent to the total weight of said composition, on a dry solid basis.

8. A composition obtainable by the method of any one of claims 1 to 7.

9. The composition of claim 8, **characterized in that** said composition is in the form of a member selected from the group consisting of food products, cosmetics, medicated cosmetics, pharmaceuticals, daily goods, feeds, pet foods, baits, groceries, and chemical industrial products.

10. The composition of claim 9, **characterized in that** wherein said food products are members selected from the group consisting of seasonings, creams, jams, pastes, Japanese confectionery, Western confectionery, bread/buns, processed products of meat including fish meat, fishery processed foods, agricultural processed food products, noodles, daily dishes, cooked rice products, frozen-food products, retort pouched food products, chilled food products, dried food products, and freeze-dried food products.

11. The composition of claim 9, **characterized in that** wherein said food products are members selected from the group consisting of table sugars, dried bonito seasoning extracts, processed products of sea urchin, dried swellfish, dried sardines, dried small sardines, dried young sardines, shucked short-necked clams, boiled octopuses, herrings soaked in vinegar, yellowtails boiled hard with soy, kneaded fish meat paste products, seasoned lavers, powdery seasonings, powdered milk, powdered eggs, powdered vegetable juice, powdered green tea, powdered oil and fat, powdered vitamins, powdered minerals, powdered DHA, powdered peppermint oils, powdered flavors, powdered pigments, powdered ginseng extract, tablets with vegetable juice, vitamin preparations, polished rice, *"musenmai"* (a pre-washed or pre-treated rice with no need for washing with water before cooking), cooked rice, Chinese dish of fried rice, noodles, instant noodles, rice pastes, *"ohagi"* (a kind of rice paste), *"mizu-manju"* (a kind of bun with a bean-jam), "an", (abean-jam), *"monaka"* (a beam-jam-filled wafer), *pao de Castella,* freezed-dough for bread, bread made from rice flour, *"takoyaki"* (a Japanese-style pancake containing octopus, i.e., octopus dumpling)", fruit gummy, caramel, jelly, fondant, hard candy with a high moisture content, cotton confectionery, Chinese sweet potato, roasted almond, sugar-coated gum, barley water, *"dango-no-tare"* (a seasoning for boiled starch paste), strawberry jam, dried fruit and mixed vegetable, meringue confectionery, chocolate cookie, pie, rice confectionery, *"nure-senbei"* (a baked rice cake coated with sugar-containing paste), pudding, lacto ice, freeze-dried Welsh onion, bean curd, bacon, solution for treating processed meat, processed whole egg liquid, retort pouched curry, *"chawan-mushi"* (a pot-steamed hotchpotch), salad dressing, mayonnaise-like food, cream, custard cream, green-tea syrup, *"matcha"* (a ground green tea) syrup, edible film, and capsule.

12. A moisture variation inhibiting agent, **characterized in that** it comprises a saccharide-derivative of α,α-trehalose.

13. The moisture variation inhibiting agent of claim 12, **characterized in that** wherein said saccharide-derivative of α,α-trehalose is a member selected from the group consisting of mono-glucose, di-glucose, tri-glucose and tetra-glucose, each of which is bound to at least one of glucose molecules of α,α-trehalose molecule.

14. The moisture variation inhibiting agent of claim 12 or 13, **characterized in that** wherein said saccharide-derivative of α,α-trehalose is a saccharide having a trehalose structure as an end unit.

15. The moisture variation inhibiting agent of any one of claims 12 to 14, **characterized in that** wherein said saccharide-derivative of α,α-trehalose is in a form of amorphous.

16. The moisture variation inhibiting agent of any one of claims 12 to 15, **characterized in that** wherein said saccharide-derivative of α,α-trehalose is incorporated in combination with another saccharide.

17. The moisture variation inhibiting agent of claim 16, **characterized in that** wherein said another saccharide is one or more members selected from the group consisting of reducing saccharides, non-reducing saccharides, sugar alcohols, and water-soluble polysaccharides.

18. The moisture variation inhibiting agent of any one of claims 12 to 17, **characterized in that** wherein said saccharide-derivative of α,α-trehalose is incorporated in an amount of at least 10% to the total weight of the agent, on a dry solid basis.

19. A composition obtainable by incorporating the moisture variation inhibiting agent of any one of claims 12 to 18 to a material for said composition.

20. The composition of claim 19, **characterized in that** said composition is in the form of a member selected from the group consisting of food products, cosmetics, medicated cosmetics, pharmaceuticals, daily goods, feeds, pet foods, baits, groceries, and chemical industrial products.

21. The composition of claim 20, **characterized in that** wherein said food product is a member selected from the group consisting of seasonings, creams, jams, pastes, Japanese confectioneries, Western confectioneries, bread, processed fish meat or red meat, fishery processed products, agricultural processed food products, noodles, cooked rice products, daily dishes, *"tare"* (a kind of seasoning for foods) or the like, freezed-food products, retort pouched foods, chilled food products, and dried food products.

22. The composition of claim 20 or 21, **characterized in that** wherein said food product is a member selected from the group consisting of table sugars, dried bonito seasoning extracts, processed products of sea urchin, dried swellfish, dried sardines, dried small sardines, dried young sardines, shucked short-necked clams, boiled octopuses, herrings soaked in vinegar, yellowtails boiled hard with soy, kneaded fish meat paste products, seasoned lavers, powdery seasonings, powdered milk, powdered eggs, powdered vegetable juice, powdered green tea, powdered oil and fat, powdered vitamins, powdered minerals, powdered DHA, powdered peppermint oils, powdered flavors, powdered pigments, powdered ginseng extract, tablets with vegetable juice, vitamin preparations, polished rice, pre-washed or pre-treated rice with no need of washing with water, cooked rice, Chinese dish of fried rice, noodles, instant noodles, *"ohagi"* (a kind of rice paste), rice pastes, *"mizu-manju"* (a kind of bun with a bean-jam), "*an*", (a bean-jam), *"monaka"* (a beam-jam-filled wafer), *pao de Castella,* freezed-dough for bread, bread made from rice flour, *"takoyaki"* (a Japanese-style pancake containing octopus, i.e., octopus dumpling)", fruit gummy, caramel, jelly, fondant, hard candy with a high moisture content, cotton confectionery, Chinese sweet potato, roasted almond, sugar-coated gum, barley water, *"dango-no-tare"* (a seasoning for boiled starch paste), strawberry jam, dried fruit and mixed vegetable, meringue confectionery, chocolate cookie, pie, rice confectionery, *"nure-senbei"* (a baked rice cake coated with sugar-containing paste), pudding, lacto ice, freeze-dried Welsh onion, bean curd, bacon, solution for treating processed meat, processed whole egg liquid, retort pouched curry, *"chawan-mushi"* (a pot-steamed hotchpotch), salad dressing, mayonnaise-like food, cream, custard cream, green-tea syrup, *"matcha"* (a ground green tea) syrup, edible film, and capsule.

23. The moisture variation inhibiting agent of any one of claims 12 to 18, which is used as a substrate for pulverization.

24. The moisture variation inhibiting agent of any one of claims 12 to 18, which is used as a preventive for binding.

25. The moisture variation inhibiting agent of any one of claims 12 to 18, which is used as a luster-imparting agent.

26. The moisture variation inhibiting agent of any one of claims 12 to 18, which is used as a gloss-imparting agent.

27. The moisture variation inhibiting agent of any one of claims 12 to 18, which is used as a shape-retaining agent.

28. The moisture variation inhibiting agent of any one of claims 12 to 18, which is used as an agent for inhibiting oxidation and/or decomposition of lipids.

29. The moisture variation inhibiting agent of any one of claims 12 to 18, which is used as a denaturation-preventing agent.

30. The moisture variation inhibiting agent of any one of claims 12 to 18 , which is used as a deterioration-preventing agent for flavors.

31. The moisture variation inhibiting agent of any one of claims 12 to 18, which is used as an agent for preventing color change and/or color fading of pigments.

32. The moisture variation inhibiting agent of claim 31, **characterized in that** wherein said pigment is chlorophyll.

33. The moisture variation inhibiting agent of claim 32, **characterized in that** wherein said chlorophyll is one contained in green tea or *"matcha"* (a ground green tea).

34. Themoisturevariation inhibiting agent of anyone of claims 12 to 18, which is used as a freshness-retaining agent.

35. The moisture variation inhibiting agent of anyone of claims 12 to 18, which is used as a flavor- and taste-retaining agent for food products.

36. The moisture variation inhibiting agent of any one of claims 12 to 18, which is used as an agent for inhibiting metmyoglobin formation and/or blackening.

37. The moisture variation inhibiting agent of any one of claims 12 to 18, which is used as a vitrifying agent.

38. The moisture variation inhibiting agent of any one of claims 12 to 18, which is used as a substrate for sugar coating.

39. The moisture variation inhibiting agent of any one of claims 12 to 18, which is used as a growth-promoting agent for plants.
